(19) Europäisches Patentamt / European Patent Office / Office européen des brevets

(11) **EP 2 710 377 B1**

(12) **EUROPEAN PATENT SPECIFICATION**

(45) Date of publication and mention
of the grant of the patent:
**09.09.2015 Bulletin 2015/37**

(21) Application number: **12725948.9**

(22) Date of filing: **18.05.2012**

(51) Int Cl.:
***G01N 33/564*** (2006.01)

(86) International application number:
**PCT/US2012/038612**

(87) International publication number:
**WO 2012/159045 (22.11.2012 Gazette 2012/47)**

(54) **DETECTION OF CIRCULATING ADAMTS13-ANTIBODY COMPLEXES**

NACHWEIS VON ZIRKULIERENDEN ADAMTS13-ANTIKÖRPERKOMPLEXEN

DÉTECTION DE CIRCULATION DE COMPLEXES ADAMTS13-ANTICORPS

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **19.05.2011 US 201161488105 P**

(43) Date of publication of application:
**26.03.2014 Bulletin 2014/13**

(73) Proprietors:
• **Baxter International Inc
Deerfield, IL 60015 (US)**
• **Baxter Healthcare SA
8152 Glattpark (Opfikon) (CH)**

(72) Inventors:
• **FERRARI, Silvia
A-1220 Vienna (AT)**
• **GRUBER, Bernadette
A-1220 Vienna (AT)**
• **PLAIMAUER, Barbara
A-1020 Vienna (AT)**
• **ROTTENSTEINER, Hanspeter
A-1020 Vienna (AT)**
• **SCHEIFLINGER, Friedrich
A-1090 Vienna (AT)**

(74) Representative: **Hoffmann Eitle
Patent- und Rechtsanwälte PartmbB
Arabellastraße 30
81925 München (DE)**

(56) References cited:
**EP-A1- 1 962 091    US-A1- 2004 214 346**

• **RIEGER MANFRED ET AL: "Relation between
ADAMTS13 activity and ADAMTS13 antigen
levels in healthy donors and patients with
thrombotic microangiopathies (TMA)",
THROMBOSIS AND HAEMOSTASIS,
SCHATTAUER GMBH, DE; US, vol. 95, no. 2, 1
February 2006 (2006-02-01), pages 212-220,
XP008153285, ISSN: 0340-6245, DOI:
10.1160/TH05-D8-0550 [retrieved on 2006-01-19]
cited in the application**
• **S. FERRARI ET AL: "Inverse correlation of free
and immune complex-sequestered anti-
ADAMTS13 antibodies in a patient with acquired
thrombotic thrombocytopenic purpura",
JOURNAL OF THROMBOSIS AND
HAEMOSTASIS, vol. 10, no. 1, 1 January 2012
(2012-01-01), pages 156-158, XP55032151, ISSN:
1538-7933, DOI: 10.1111/j.
1538-7836.2011.04548.x**

**Description**

FIELD OF THE INVENTION

The invention relates to methods for the determination of anti-von Willebrand Factor-cleaving protease (anti-ADAMTS13) antibodies which are bound in circulating immune complexes (CIC) with ADAMTS13, and the clinical uses thereof.

BACKGROUND OF THE INVENTION

Von Willebrand Factor-cleaving protease (a disintegrin and metalloproteinase with a thrombospondin type 1 motif, member 13, "ADAMTS13") has been isolated, purified and characterised previously, such as in WO 1997/041206 and WO 02/42441. ADAMTS13 degrades large VWF multimers in the blood stream, decreasing their platelet aggregation activity.

ADAMTS13 dysfunction can lead to the accumulation of large amounts of von Willebrand Factor high molecular weight multimers in blood, which in turn can lead to microthrombotic events and damage blood vessels. Deficiency of ADAMTS13 is linked with Upshaw-Schülman syndrome and the hereditary form of thrombotic thrombocytopenic purpura (TTP). Further, ADAMTS13 dysfunction can be caused by aberrant immune responses, targeting ADAMTS13. One example of a diseases with immunological causes characterised by the formation of immune-inhibitors of ADAMTS13 is acquired TTP. In acquired TTP IgM and IgG antibodies to von Willebrand Factor-cleaving protease can be non-neutralising (Scheiflinger 2003; Levy 2005). Persistent immune complexes also may accumulate in the plasma and cause adverse complement activating reactions damaging blood vessels. ADAMTS13-neutralizing autoantibodies are the major cause of acquired TTP. IgG subtypes of anti-ADAMTS13 antibodies with different effector functions have been investigated (Ferrari, 2009).

Acquired TTP (aTTP) is a remitting disease (Sadler, 2008) characterized by low platelets, elevated LDH, low red blood cell count (caused by premature breakdown of the cells), fragmented red blood cells (schistocytes) and neurological abnormalities. The sharp drop in the number of red blood cells and platelets in the blood is associated with severe problems affecting the kidneys and brain, along with fever and bleeding. Purpura refers to the characteristic bleeding that occurs beneath the skin, or in mucus membranes, which produces bruises, or a red rash-like appearance. The neurological symptoms associated with this disease include headaches, confusion, speech changes, and alterations in consciousness, which vary from lethargy to coma; other symptoms include development of kidney abnormalities. If untreated, acute TTP can lead to death in a few days (about 90% of patients presenting died before current treatment regimens) so rapid detection of the cause and type of the disease is of the essence. Current treatment consists of infusion of fresh frozen plasma with or without plasma exchange or plasmapheresis. In addition, treatment with recombinant ADAMTS13 is being explored. ADAMTS13 immune complex burden may fluctuate during progression of the disease. In order to tailor an effective therapy for aTTP there is a need to identify, qualify, and quantify immune reactions against ADAMTS13 and CIC. However, very few laboratories can perform ADAMTS13 assays rapidly enough, and the clinician must make a diagnosis and initiate therapy without this information (Sadler, 2008).

A cell-based assay was developed to detect immune complexes in serum samples (Theofilopoulos, 1976). However, such assays are time-intensive, cumbersome and suffer from low reproducibility.

Simpler assays exist to determine the ADAMTS13 activity or the presence of total ADAMTS13 concentration in plasma (Rieger, 2006). Other assays are aimed at detecting anti-ADAMTS13 antibodies in plasma samples (Rieger, 2005; WO 2004/095027). However, these assays suffer from limited use in the diagnosis of specific diseases associated with ADAMTS13 dysfunction like acquired TTP (Rieger, 2006).

A goal of the present invention is to improve sensitivity and accuracy of assays for the detection and determination of CIC of antibodies to ADAMTS13 in biological samples, as well as the use of these improved assays in patient care.

SUMMARY OF THE INVENTION

The present invention provides a method for detecting or determining amounts of anti-ADAMTS13 antibodies in complex with ADAMTS13 as defined in the claims. This method can be combined with a determination of free anti-ADAMTS13 antibodies and/or the determination of free ADAMTS13 or of ADAMTS13 activity. Target anti-ADAMTS13 antibodies are bound by ADAMTS13, or if already complexed to ADAMTS13, by an ADAMTS13 binding unit, which is an anti-ADAMTS13 antibody. This step allows the specific selection of the anti-bodies with ADAMTS13 specificity from a sample background. The antibodies are further bound by a moiety that recognizes the antibodies and /or their specific subclasses, termed the antibody binding unit, namely an anti-antibody antibody. ADAMTS13 immune complexes comprise ADAMTS13 and anti-ADAMTS13 antibodies. The inventive method is based on a two component binding mechanism, the ADAMTS13 part of the complex is bound by said ADAMTS13 bind-ing unit and the anti-ADAMTS13 antibody part of the complex is bound by said antibody binding unit. Both binding reactions ensure the specific detection of ADAMTS13 immune complexes.

In one particular aspect, the present invention provides a method for detecting anti-ADAMTS13 antibody-ADAMTS13 immune complexes in a sample comprising contacting said sample with an ADAMTS13 binding unit, which is an anti-ADAMTS13 antibody, contacting said sample with an antibody binding unit, namely an anti-antibody antibody and detecting anti-ADAMTS13 an-tibody-ADAMTS13 immune complexes bound by said ADAMTS13 binding unit and said antibody binding unit.

In a further aspect, the present invention relates to a combination method for detecting a) anti-ADAMTS13 antibody-ADAMTS13 immune complexes in a sample comprising contacting said sample with an ADAMTS13 binding unit, which is an anti-ADAMTS13 antibody, contacting said sample with an antibody binding unit, namely an anti-antibody antibody, and detecting anti-ADAMTS13 antibody-ADAMTS13 immune complexes bound by said ADAMTS13 binding unit and said antibody binding unit; and b) anti-ADAMTS13 antibodies in a sample comprising binding said anti-ADAMTS13 antibodies to ADAMTS13 or a fragment thereof and detecting said anti-ADAMTS13 antibodies bound to ADAMTS13 or said fragment with antibody binding units specific for said anti-ADAMTS13 antibod-ies; wherein the relative amounts and types of uncomplexed and complexed anti-ADAMTS13 antibodies may be determined. Said anti body binding units in steps a) and/or b) can be antibody class or antibody subtype specific.

In another aspect, the present invention provides a combination of the method of detecting a) anti-ADAMTS13 anti-body-ADAMTS13 immune complexes in a sample and detecting b) free ADAMTS13 or determining ADAMTS13 activity in the sample, wherein the relative amounts of uncomplexed and complexed ADAMTS13 or the relation of ADAMTS13 activity to ADAMTS13 immune complexes may be determined.

In another aspect the specification relates to a kit comprising an ADAMTS13 binding unit and at least one antibody binding unit.

Since the presence of complexed and/or free anti-ADAMTS13 antibodies in a biological sample can indicate a pathological condition, the present invention also relates to a method of diagnosing a disease associated with ADAMTS13 dysfunction (e.g, TTP) in a patient comprising obtaining a sample from a patient and detecting ADAMTS13 immune complexes and free anti-ADAMTS13 antibodies or ADAMTS13 immune complexes only in said sample. A further aspect of this invention is the monitoring of ADAMTS13 immune complexes and free anti-ADAMTS13 antibodies, free ADAMTS13 and/or ADAMTS13 immune complexes in a patient in order to adjust treatment of the patient with an ADAMTS13 containing medicament (such as fresh frozen plasma or a recombinant ADAMTS13 composition).

BRIEF DESCRIPTION OF THE DRAWINGS

Fig. 1 shows a schematic representation of the inventive method for detecting immune complexes. Antibodies (1) targeting one part of the sample immunoglobulin-antigen complex (2), here the antigen, are coated onto a solid surface. Secondary antibodies (3) target the other part of the immune complex, here the immunoglobulin. A label on the secondary antibodies results in a detectable signal (4), such as the horse radish peroxidase (HRP) system.

Fig. 2 shows the determination of immune complexes in diluted positive samples (TTP) and negative samples (NHP) in a setup using polyclonal anti-ADAMTS13 antibodies (fig. 2a: commercial antibody abcam, fig. 2b rabbit polyclonal antibody K1-4) as coating antibodies and IgG subtype specific secondary antibodies.

Fig. 3 shows results of an anti-IgG4 immunoassay using different immobilized anti-ADAMTS13 antibodies: a: polyclonal antibody abcam; b: polyclonal antibody K1-4; c: polyclonal antibody K6; d: monoclonal antibody 5.1; e: monoclonal antibody 12.1;

Fig. 4 shows results of an anti-IgG immunoassay to determine the cross-reactivity of different secondary antibodies; fig. 4a shows the IgGl-4 subtype and IgA and IgM reactivity of an anti-IgG1 antibody; fig. 4b the same of an anti-IgG2 antibody; fig. 4c the same of an anti-IgG3 antibody; fig. 4d the same of an anti-IgG4 antibody; fig. 4e the same of an anti-IgA antibody; fig. 4f the same of an anti-IgM antibody;

Fig. 5 shows results of an ADAMTS13 immune complex immunoassay with different monoclonal anti-ADAMTS13 antibodies as anti-ADAMTS13 antibodies and different Ig class and subtype specific secondary antibodies (fig. 5a: anti-IgG1, fig. 5b: anti-IgG2, fig. 5c: anti-IgG3, fig. 5d: anti-IgG4, fig. 5e: anti-IgA, fig. 5f: anti-IgM secondary anti-bodies).

Fig. 6 shows results of an ADAMTS13 immune complex immunoassay comparison of an polyclonal and a monoclonal anti-ADAMTS13 antibody as immobilized antibodies and different Ig class and subtype specific secondary antibodies (fig. 6a: polyclonal Ab K1-4, 6b: monoclonal Ab 5C11) and in fig. 6c results of an anti-IgG4 immunoassay using polyclonal anti-ADAMTS13 antibody K1-4.

Fig. 7 shows results of an ADAMTS13 immune complex immunoassay with non-subtype specific IgG class specific anti-IgG antibodies in different samples (fig. 7a: TTP sample spiked with rADAMTS13 vs. NHP plasma; fig. 7b: TTP plasma vs. NHP plasma).

Fig. 8 shows results of an ADAMTS13 immune complex immunoassay with affinity purified polyclonal antibodies as anti-ADAMTS13 antibodies and different Ig class and subtype specific antibodies as secondary antibodies (fig. 8a: anti-IgG1, fig. 8b: anti-IgG2, fig. 8c: anti-IgG3, fig. 8d: anti-IgG4, fig. 8e: anti-IgA, fig. 8f: anti-IgM secondary antibodies).

Fig. 9 shows results of an ADAMTS13 immune complex immunoassay with affinity purified polyclonal and a mixture of monoclonal antibodies as anti-ADAMTS13 antibodies and different Ig class and subtype specific antibodies as secondary antibodies (fig. 9a: anti-IgG1, fig. 9b: anti-IgG2, fig. 9c: anti-IgG3, fig. 9d: anti-IgG4, fig. 9e: anti-IgA, fig. 9f: anti-IgM secondary antibodies).

Fig. 10 shows results of an ADAMTS13 immune complex immunoassay with different affinity purified polyclonal antibodies as anti-ADAMTS13 antibodies (fig. 10a: antibody K1-4, fig. 10b: antibody K1-10, affinity purified, fig. 10c: antibody K1-10, non-affinity purified).

Fig. 11 shows results of an anti-IgG4 immunoassay using different non-affinity purified (a) and affinity purified (b) anti-ADAMTS13 antibodies.

Fig. 12 shows results of an anti-IgA antibody immunoassay against IgG1-4 subtype, IgA and IgM immunglobulins.

Fig. 13 shows immunoassay results illustrating immunglobulin subtype specificity of non-subtype specific anti-IgG specific secondary antibodies.

Fig. 14 shows results of an ADAMTS13 immune complex immunoassay with non-subtype specific anti-human IgG secondary antibodies using different samples (fig. 14a: TTP plasma spiked with recombinant ADAMTS13; fig. 14b: TTP plasma).

Fig. 15 shows results of immunoassays illustrating immunglobulin subtype specificity of mouse non-subtype specific anti-human IgG specific secondary antibodies.

Fig. 16 shows results of an ADAMTS13 immune complex immunoassay with non-subtype specific anti-human IgG secondary antibodies using different samples (fig. 16a: TTP plasma spiked with rADAMTS13; fig. 16b: TTP plasma).

Fig. 17 shows immunoassay results of the determination of ADAMTS13 immune complexes in TTP samples and a pooled healthy control sample (NHP) at different salt concentrations for selected class and subtype specific antibodies (a: IgG1, b: IgG2, c: IgG3, d: IgG4, e: IgA, f: IgM).

Fig. 18 shows results of ADAMTS13 immune complex immunoassays of individual plasma samples of TTP patients using Ig class and subtype specific antibodies (a: IgG1, b: IgG2, c: IgG3, d: IgG4, e: IgA, f: IgM).

Fig. 19 shows immunoassay results of the determination of ADAMTS13 immune complexes in a TTP sample and a pooled healthy control sample at different salt concentrations using an anti-human IgG1 secondary antibody (Fitzgerald). (a: 1:32000 dilution, b: 1:64000 dilution)

Fig. 20 shows immunoassay results of the determination of ADAMTS13 immune complexes in a TTP sample and a pooled healthy control sample at different salt concentrations for two anti-human IgG4 secondary antibodies. (a: 1:32000 dilution, Invitrogen antibody; b: 1:2000 dilution AbD Serotech antibody).

Fig. 21 shows results of cross-reactivity assays for an anti-human IgG1 secondary antibody (Fitzgerald). (a: 1:16000 dilution; b: 1:32000 dilution; c: 1:64000 dilution)

Fig. 22 shows results of cross-reactivity assays for two anti-human IgG4 secondary antibodies (a: Plate 1, b: Plate 2)

## DETAILED DESCRIPTION OF THE INVENTION

The present invention provides a method of detecting antibodies with a specificity to ADAMTS13, a von Willebrand Factor (vWF) cleaving protease, in immune complexes with ADAMTS13. Furthermore the detection of free anti-ADAMTS13 antibodies in a sample without complexed ADAMTS13 is contemplated. The detection of free anti-ADAMTS13 antibodies can be combined with the detection of ADAMTS13 immune complexes, in particular to determine the ratio of free and complexed anti-ADAMTS13 antibodies in a sample, which is of diagnostic importance. In a further embodiment, the detection of free ADAMTS13 or the determination of ADAMTS13 activity in a sample is contemplated. The detection of free ADAMTS13 and/or determination of ADAMTS13 activity can be combined with the detection of ADAMTS13 immune complexes, in particular to determine the ratio of free and complexed ADAMTS13 or the relation between ADAMTS13 activity and complexed ADAMTS13 in a sample.

VWF cleaving proteases (VWF-cp, EC 3.4.24.87) are disclosed in WO 1997/041206, WO 2002/042441 and WO 2004/095027. Special reference is made to the sequences of the VWF cleaving protease ADAMTS13 or fragments thereof as disclosed in WO 02/42441. For the inventive purposes, plasma derived or recombinant ADAMTS13, or fragments thereof, can be purchased commercially or obtained as disclosed in these references. ADAMTS13 sequences are further disclosed in UniProt database entry Q76LX8 and NCBI GenBank database entry AAL11095.1. "ADAMTS13" as used herein refers to the VWF-cp and polypeptides of the above mentioned sequences as well as isoforms, polymorphisms, fragments, variants and other mutant forms thereof that are immunologically indistinguishable from wild type ADAMTS13. In particular, ADAMTS13 isoforms, polymorphisms, fragments, variants and other mutant forms thereof, comprise an epitope of ADAMTS13, recognized by the anti-ADAMTS13 antibody or the ADAMTS13 binding unit.

The term "epitope" defines any region of a molecule that can be recognised by specific antibody or that provoke the formation of those specific antibodies. Epitopes may be conformational epitopes (made up of non-sequential portions of a polymeric or complex molecule,) or may be linear epitopes.

ADAMTS13 binding units or antibody binding units are antibodies that comprise a binding domain for a given target (ADAMTS13 or an antibody).

The term "binding domain" characterizes in connection with the present invention a domain of a binding unit which specifically binds to/interacts with a given target structure/epitope. Thus, the binding domain is a "target-interaction-site". The term "target-interaction-site" defines, in accordance with the present invention, a motif, which is able to specifically interact with a specific target or a specific group of targets. Said binding/interaction is also understood to define a "specific recognition". The term "specifically recognizing" means in accordance with this invention that the binding unit is capable of specifically interacting with and/or binding to at least 2, 3, 4, 5, 6 or more amino acids of a target. Such binding may be exemplified by the specificity of a "lock-and-key-principle". Thus, specific motifs in the amino acid sequence of the binding domain and the target bind to each other as a result of their primary, secondary or tertiary structure as well as the result of secondary modifications of said structure. The specific interaction of the target-interaction-site with its specific target may result as well in a simple binding of said site to the target.

The binding unit in line with the present invention is an antibody, including an anti-immunoglobulin antibody or an anti-ADAMTS13 antibody.
The binding unit may be a monoclonal or polyclonal antibody or derived from a monoclonal or polyclonal antibody. The term "antibody" comprises derivatives or functional fragments thereof which still retain the binding specificity. The definition of the term "antibody" also includes embodiments such as chimeric, single chain and humanized antibodies, as well as antibody fragments, such as, inter alia, Fab fragments. Antibody fragments or derivatives further comprise F(ab')2, Fv, scFv fragments or single domain antibodies, single variable domain antibodies or immunoglobulin single variable domain comprising merely one variable domain, which might be VH or VL, that specifically bind to a target or epitope independently of other V regions or domains. Such immunoglobulin single variable domain encompasses not only an isolated antibody single variable domain polypeptide, but also larger polypeptides that comprise one or more monomers of an antibody single variable domain polypeptide sequence.

In preferred embodiments the binding unit has a high binding affinity to the target (in particular ADAMTS13 or the antibody). A high binding affinity distinguishes specific binding from any forms of unspecific binding. In particular, high binding affinity is characterized by high equilibrium dissociation constant (KD), especially at 22°C, of below 10e-3 M, below 10e-4 M, below 10e-5 M, below 10e-6 M, below 10e-7 M, below 10e-8 M, or even below 10e-9 M.

The present invention provides for a method of detecting or determining anti-ADAMTS13 antibody-ADAMTS13 immune complexes (also referred to as ADAMTS13 immune complexes herein) in a sample comprising contacting said sample with an ADAMTS13 binding unit, which is an anti-ADAMTS13 antibody, contacting said sample with an antibody binding unit, namely an anti-antibody antibody, and detecting anti-ADAMTS13 antibody-ADAMTS13 immune complexes bound by said ADAMTS13 binding unit and said antibody binding unit. "Determining" may include quantifying.

The present invention also describes a method of detecting or determining anti-ADAMTS13 antibodies in a sample, preferably comprising binding said anti-ADAMTS13 antibodies to ADAMTS13 or a fragment thereof and detecting said anti-ADAMTS13 antibodies bound to ADAMTS13 or said fragment with antibody binding units specific for said anti-ADAMTS13 antibodies. This method is combined with the method of detecting or determining ADAMTS13 immune complexes, in particular to determine the ratio of free and complexed anti-ADAMTS13 antibodies in the sample. Thus, the present invention further provides a method for detecting or determining, in conjunction with anti-ADAMTS13 antibody-ADAMTS13 immune complexes, anti-ADAMTS13 antibodies to ADAMTS13 or a fragment thereof and detecting said anti-ADAMTS13 antibodies bound to ADAMTS13 or said fragment with antibody binding units specific for said anti-ADAMTS13 antibodies.

Also contemplated are methods of detecting or determining ADAMTS13 in a sample. Such method may comprise the steps of binding said ADAMTS13 of the sample to an ADAMTS13 binding unit, which is an anti-ADAMTS13 antibody, and detecting said bound ADAMTS13. For the detecting step, it is possible to use a second ADAMTS13 binding unit which is labelled. This method can be combined with the inventive method of detecting or determining ADAMTS13 immune complexes to determine the ratio of ADAMTS13 immune complexes to free ADAMTS13.

Also contemplated are methods of detecting ADAMTS13 activity in a sample. Such methods may comprise the steps of contacting ADAMTS13 in said sample with a substrate of ADAMTS13 and detecting cleavage products of VWF or a smaller peptide substrate, e.g., by fluorescent label detection upon cleavage from a quencher on the substrate. Contacting can be performed under conditions where ADAMTS13 is active to cleave the substrate. Such conditions can be selected by a skilled man in the art, such as physiologic conditions. ADAMTS13 cleaves the peptidic bond between Tyr1605 and Met1606 of VWF (Plaimauer et al, 2002) or between Tyr842 and Met843 in the A2 domain of mature vWF (WO 97/041206). A suitable substrate of ADAMTS13 is VWF or polypeptide comprising a fragment of VWF comprising an ADAMTS13-cleavable peptidic bond, such as a polypeptide comprising Asp1596-Arg1668 of VWF (Wu, 2005). The substrate may be modified, e.g. to facilitate immobilization and/or labelling of the substrate or the cleavage products as known in the art. This method can be combined with the inventive method of detecting or determining ADAMTS13 immune complexes to determine the ratio of ADAMTS13 immune complexes to ADAMTS13 activity.

The inventive methods are for detecting or determining ADAMTS13 complexes, free anti-ADAMTS13 antibodies and/or free ADAMTS13 (or ADAMTS13 activity) in a sample potentially comprising these analytes. "Free" anti-ADAMTS13 antibodies shall refer herein to anti-ADAMTS13 antibodies that are not bound or complexed to its antigen ligand, ADAMTS13. Likewise, "free" ADAMTS13 shall be understood to refer to ADAMTS13 not complexed by an anti-ADAMTS13 antibody.

In favourable embodiments of the inventive methods, the antibody binding units are antibody subtype or antibody class specific. "Antibody subtype" means that the antibody binding units have a distinctive preference for binding antibodies of a particular subtype with little or no binding to antibodies of other subtypes (subtype cross-reactivity). Likewise, "antibody class specific" means that the antibody binding units have a distinctive preference for binding antibodies of a particular class with little or no binding to antibodies of other classes (class cross-reactivity). In particular preferred embodiments antibody binding units are used with less that 25%, less than 20%, less than 15%, less than 13%, less than 10%, less than 9%, less than 8%, less than 7%, less than 6%, less than 5%, less than 4%, less than 3%, less than 2%, less than 1%, less than 0.1%, less than 0.01% or less than 0.001% subtype or class cross-reactivity, respectively, when comparing the binding affinity of the selective class with non-selective classes.

Example antibody class are selected from IgG, IgM, IgA, IgD, IgE. Antibody classes may be further divided into subtypes, like IgG1, IgG2, IgG3, IgG4 (human subtypes). In particular preferred embodiments, the subtype specificity of the antibodies used according to the present invention is for either IgG1, IgG3, or IgG4, in special embodiments also for IgG2; each with the low cross-reactivities mentioned above to the respective other subtypes. Subtype specific antibodies are usually also class specific antibodies. On the other hand, class specific antibodies may or may not be subtype specific. In preferred embodiments, the class specific antibodies are selected from antibodies specific for either IgG or IgM, in further embodiments also for either IgA, IgD, IgE; each with low cross-reactivity mentioned above to the respective other classes.

The antibody binding unit may be specific for certain domains of the target antibody, such as the Fc part of the target antibody. Usually this domain is distinctive for the antibody class or subtype, like the gamma, or mu chains of the antibodies.

The ADAMTS13 binding units may be a polyclonal or monoclonal antibody. In both cases, but especially for polyclonal antibodies, it is favourable to use immune or affinity purified antibodies. Immune or affinity purification can be achieved by contacting the ADAMTS13 binding unit, or generally any binding unit, with its target, such as ADAMTS13, and selecting ADAMTS13 binding units that bind said target. Immune or affinity purification is preferably performed by selective immobilisation of the binding units, such as on a solid phase like a bead on a column, and isolating the bound binding units, such as by elution from the column after washing. The binding units can be selectively immobilised by first immobilising the target and then binding the binding unit onto said immobilised target. Immune or affinity purification may result in binding units with less specificity to any other targets, lowering cross-reactivities. As demonstrated in the examples, the use of non-affinity purified polyclonal antibodies results in assays with less specificity and sensitivity than affinity purified materials when used in anti-ADAMTS13 antibody-ADAMTS13 complex detection. Polyclonal antibodies are usually available as mixtures of antibodies. Such mixtures of polyclonal antibodies may contain only up to 10% or slightly more antibodies specific to a given target, e.g. ADAMTS13. In preferred embodiments the ADAMTS13 binding units, especially in case of antibodies, are provided in purified form wherein at least 20% of the, at least 30%, at least 40%, at least 50%, at least 60%, at least 70%, at least 75%, at least 80%, at least 85%, at least 90%, at least 95%, at least 98%, at least 99%, or even 100% of the binding units or antibodies (especially polyclonal antibodies) bind ADAMTS13 or are ADAMTS13 specific.

The inventive methods are based on a two component binding mechanism, the anti-ADAMTS13 antibodies of the target are bound by 1) either ADAMTS13 or, if already bound to ADAMTS13, to an ADAMTS13 binding unit, which is an anti-ADAMTS13 antibody, and 2) to an antibody binding unit, namely an anti-antibody antibody. Steps 1) and 2) can be performed in any order, first 1) then 2) or first 2) then 1) or simultaneously. In preferred embodiments the agents of step 1), i.e. the ADAMTS13 or the ADAMTS13 binding unit may be immobilized onto a solid phase or the agent of step 2), the antibody binding unit may be immobilized onto a solid phase. Contacting the sample with the immobilized agents leads to immobilization of the anti-ADAMTS13 antibodies or immune complexes of the sample. Immobilized anti-ADAMTS13 antibodies or immune complexes can be easily handled and purified in washing steps.

The term "solid phase" does not imply any specific limitations, and relates, for example, to an insoluble polymer material, which can be an organic polymer, such as polyamide or a vinyl polymer (e.g., poly(meth)acrylate, polystyrene and polyvinyl alcohol, or derivates thereof), a natural polymer such as cellulose, dextrane, agarose, chitin and polyamino acids, or an inorganic polymer, such as glass or metallohydroxide. The solid phase can be in the form of a microcarrier, particles, membranes, strips, paper, film, pearls or plates, such as microtiter plates. The immobilized agents can be immobilized on the solid phase directly by covalent coupling or via a carrier such as a linker molecule or an antibody immobilized on the solid phase.

In further embodiments the agents of step 1), i.e. the ADAMTS13 or the ADAMTS13 binding unit may be labelled or the agent of step 2), the antibody binding unit may be labelled. Contacting the sample with the labelled agents allows the generation of a signal in dependence of the anti-ADAMTS13 antibodies originally present in the sample. The combination of steps 1) and 2), with one agent being immobilized and the other being labelled, means that the signal created by immobilized substances is dependent solely on the presence of anti-ADAMTS13 antibodies. In particular preferred embodiments, the antibody binding unit is labelled and the ADAMTS13 binding unit or ADAMTS13 is immobilized. The label can be a fluorogenic, chromogenic, radioactive or enzymatic label.

The complex of ADAMTS13/ADAMTS13 binding units with anti-ADAMTS13 antibodies/immune complexes and antibody binding units can be detected by methods well known in the art, e.g. by detection of a label. The detection method can be selected from the group consisting of an enzyme assay, a chromogenic assay, a lumino assay, a fluorogenic assay, and a radioimmune assay. The reaction condition to perform detection of the antibody/antigen-/complex formation depends upon the detection method selected. It is within the knowledge of the person skilled in the art to choose the optimal parameters, such as buffer system, temperature and pH for the respective detection system to be used.

A two component binding mechanism is also possible for methods of detecting free ADAMTS13. ADAMTS13 may be bound to 1) a first ADAMTS13 binding unit, which can be immobilized onto a solid support, and 2) to a second ADAMTS13 binding unit, which may be labelled.

In preferred embodiments, the sample is obtained from a mammal, especially from a human. The antibody binding unit should recognize antibodies from the same organism, such as human antibodies. ADAMTS13 immune complex detection or determination, optionally also of free anti-ADAMTS13 antibodies and/or of free ADAMTS13 or of ADAMTS13 activity, can be performed in dilution, including a gradual dilution of the sample with a diluent of e.g. 1:1, 1:2, 2:3, 1:4, 1:5, :10, 1:15, 1:20, 1:25, 1:30, 1:40, 1:50, 1:75, 1:100, 1:150, 1:200, 1:300, 1:400, 1:500, 1:600, 1:700, 1:800, 1:1000 or more, including any ranges within these dilutions. Samples can be diluted with any inert diluent, including water, buffers or analyte- (ADAMTS13 immune complex, free anti-ADAMTS13 antibodies, free ADAMTS13) -negative serum samples. Gradual dilution can be performed with 2, 3, 4, 5, 6 or more different dilutions.

The term "sample" as used herein is meant to include a biological fluid such as blood, plasma or tissue of a patient. The patient may be a human patient. The sample may be in particular obtained from patients suspected of having a disorder associated with occurrence of anti-ADAMTS13 antibodies. In particular, the sample may be obtained from a patient having or suspected of having a disease associated with ADAMTS13 dysfunction or deficiency, such as TTP. In further embodiments, the patient may be receiving an ADAMTS13 substitution therapy, such as by administration of plasma derived or recombinant ADAMTS13 or a plasma infusion or plasmapheresis therapy.

A sample known not to comprise any anti-ADAMTS13-antibody, e.g., normal human plasma can be used as negative control. A negative control can be used as comparison to a sample of interest. Such a comparison can be included to quantify anti-ADAMTS13-antibodies in a sample of interest to adjust or correlate signal values of a method of detection to determine blank values. In certain embodiments, the signal ratio of a sample of interest to a negative control is used.

In preferred embodiments, the binding reaction of the anti-ADAMTS13 antibody immune complexes of the sample to ADAMTS13 binding units and/or the step of binding the anti-ADAMTS13 antibodies or of the immune complexes of the sample to the antibody binding units and/or in the detecting step is under conditions of ionic strength of at least 0.1 M, at least 0.2 M, at least 0.3 M, at least 0.4 M, at least 0.5 M, at least 0.6 M, at least 0.7 M, at least 0.8 M, at least 0.9 M, at least 1.0 M or greater. The ionic strength, I, of a solution is a function of the concentration of all ions present in that solution: $I = \frac{1}{2}\sum_{i=1}^{n} c_i z_i^2$ where ci is the molar concentration of ion i, zi is the charge number of that ion, and the sum is taken over all ions in the solution. In preferred embodiments I represents the ionic strength of only the salt ions in said solution, especially ions selected from $Li^+$, $Na^+$, $K^+$, $Mg^{++}$, $Ca^{++}$, $NH_4^+$ and $Cl^-$, $I^-$, $PO_4^{-3}$, $SO_4^{-2}$, $CO_3^{-2}$, in particular of mono- or bivalent salt ions. In especially preferred embodiments the salt concentration of NaCl is at least 0.1 M, at least 0.2 M, at least 0.3 M, at least 0.4 M, at least 0.5 M, at least 0.6 M, at least 0.7 M, at least 0.8 M, at least 0.9 M, at least 1.0 M or greater. Such high ionic strength of high salt conditions are especially preferred for using IgG4, IgG3, IgA or IgM subtype or class specific antibodies as antibody binding moieties. High ionic strength or salt concentrations are also acceptable for IgG1 specific antibodies as antibody binding moieties. Alternatively or in addition thereto, the ionic strength or (NaCl) salt concentration in all the above reactions may be of equal or less than 0.1 M, equal or less than 0.2 M, equal or less than 0.3 M, equal or less than 0.4 M, equal or less than 0.5 M, equal or less than 0.6 M, equal or less than 0.7 M, equal or less than 0.8 M, equal or less than 0.9 M, equal or less than 1.0 M. It was found that in low ionic strength conditions higher signals can be observed, however also the blank value of anti-ADAMTS13 antibody negative samples was higher which could reduce the effectiveness. Nevertheless, for situations, e.g. when using IgG1 specific antibodies as antibody binding moieties or in low signal samples, low ionic strength or salt conditions can be beneficial. Such low ionic strength or salt concentrations may also be acceptable for IgG4, IgG3, IgA or IgM subtype or class specific antibodies as antibody binding moieties.

In a further aspect, the present specification provides a kit comprising an ADAMTS13 binding unit and/or an antibody binding unit, and/or buffers of a higher and/or lower ionic strength as described above. Furthermore a kit is provided comprising ADAMTS13 or a fragment thereof and at least one antibody binding unit. A kit may further comprise one or more of the group of ADAMTS13 or a fragment thereof and a substrate of ADAMTS13. The antibody binding unit may be antibody subtype specific as described above. The kits can be used in the above described methods. Either component of the kits can be immobilized on a solid phase, such as microtiter plate wells. The ADAMTS13 binding unit/ADAMTS13 or the antibody binding unit can be immobilized on the solid phase separately on different spots, e.g. in different wells of a microtiter plate, wherein typically one defined agent such as ADAMTS13/ADAMTS13 binding units is contained in one spot.

The antibody binding unit and/or ADAMTS13/ADAMTS13 binding units as specified above can be labelled. The label can be a fluorogenic, chromogenic, radioactive or enzymatic label. Kits may further comprise additives, diluents, detecting reagents, wash solutions and/or buffers (PBS, PBS-T, Tris, acetate or phosphate buffers) having various pH values and ionic strengths, solubilizer such as Tween or Polysorbate, carriers such as human serum albumin or gelatin, preservatives such as thimerosal or benzyl alcohol, and antioxidants such as ascorbic acid or sodium metabisulfite, and/or negative control serum samples. Selection of a particular kit composition will depend upon a number of factors, including the sample being used.

In another aspect, the present invention provides a method of diagnosing a disease or predisposition of a disease associated with ADAMTS13 dysfunction or deficiency in a patient comprising obtaining a sample from a patient and detecting anti-ADAMTS13 antibodies or immune complexes in said sample according to a method of the invention described herein. The disease may be TTP, especially acquired TTP. "ADAMTS13 dysfunction" or "ADAMTS13 deficiency" in particular relate to a loss of ADAMTS13 function due to the presence of antibodies targeting ADAMTS13 and formation of ADAMTS13 immune complexes in the patient. Such antibodies targeting ADAMTS13 can be inhibitory antibodies or non-neutralizing antibodies. As a consequence, ADAMTS13 activity and/or concentration can be reduced in said patient. The direct result of ADAMTS13 dysfunction can be the cause of a certain class of diseases associated with ADAMTS13, such as ADAMTS13 deficiency associated TTP, and can be the diagnostic parameter to diagnose or predict such diseases. "Predicting a disease" or "diagnosing a predisposition" as used herein shall not be understood in an absolute sense, in that in all cases a disease will develop, but as a relative increased risk of the patient to develop a disease. The inventive methods can also be used to detect the formation of ADAMTS13 immune complexes during a therapy of a patient suffering from a ADAMTS13 dysfunction or deficiency, especially during an enzyme substitution therapy with ADAMTS13.

The inventive method of detecting ADAMTS13 immune complexes in a sample of a patient can be combined with methods of detecting free anti-ADAMTS13 antibodies or methods of detecting free ADAMTS13 or determining ADAMTS13 activity. The determination of a ratio or of a correlation between the amounts of free anti-ADAMTS13 antibodies and ADAMTS13 immune complexes, or a ratio or correlation between amounts of free ADAMTS13/ADAMTS13 activity and ADAMTS13 immune complexes can be of particular diagnostic value in the diagnosis or prediction of certain diseases or in monitoring a therapy.

Example ADAMTS13 associated diseases include TTP (thrombotic thrombocytopenic purpura), in particular acquired TTP and Upshaw-Schülman syndrome (hereditary TTP). In acquired TTP, patients have a low ADAMTS13 activity due to the development of autoimmune antibodies directed at ADAMTS13. Immune-complexed ADAMTS13 is inactivated, neutralized and/or cleared from the blood stream and patient plasma. Reduced ADAMTS13 activity leads to the accumulation of large uncleaved VWF multimers which can spontaneously adhere to platelets and leading to platelet-VWF-rich thrombi in the microcirculation. The presence of anti-ADAMTS13 antibodies is the main diagnostic marker to diagnose acquired TTP. This marker can be used to distinguish acquired TTP from hereditary TTP, which is a congenital lack of functional ADAMTS13 protein. The ratio of immune complexes to free anti-ADAMTS13 antibodies provides further information of the severity of the disease and to a possible treatment outcome. An inverse relationship between free antibodies and immune complexes can be observed. In a mild form of the disease or even in healthy patients with a predisposition towards the disease some immune complexes can be detected but no or little amounts of free anti-ADAMTS13 antibodies will be observed. Free ADAMTS13 or ADAMTS13 activity can still be observed in plasma samples from such patients. As the disease progresses, an amount of free anti-ADAMTS13 antibodies will become detectable and may further increase in relation to the amount of immune complexes. Such an excess of free anti-ADAMTS13 antibodies may indicate severe forms of the disease. Knowledge of free anti-ADAMTS13 antibodies may further be of importance to tailor a substitution therapy as an excess of free antibodies may reduce or diminish the effect of administered ADAMTS13. Therefore, larger doses may be required.

Patients with Upshaw-Schulman syndrome (hereditary TTP) suffer from a mutation in the ADAMTS13 gene and do not express active ADAMTS13. Formation of anti-ADAMTS13 alloantibodies and ADAMTS13 immune complexes may result as a consequence of administration of ADAMTS13, a foreign substance to these patients with no tolerance to ADAMTS13. The inventive method is of particular importance to monitor a therapy in these patients - similar as described above for TTP.

The information on the amount of immune complexes, especially in relation to free anti-ADAMTS13 antibodies or free ADAMTS13/ADAMTS13 activity, may provide further information on secondary symptoms in a patient. TTP patients may suffer from kidney failure. Kidney failure in turn may reduce clearance of circulatory ADAMTS13 immune complexes resulting in an increased complex accumulation in blood or plasma samples. Increased immune complex amounts, especially in relation to free anti-ADAMTS13 antibodies or free ADAMTS13/ADAMTS13 activity, indicate increased inflammatory reaction in the patient, which may even result in inflammatory shock. For such patients, antiinflammatory therapy may be required.

Diseases associated with ADAMTS13 dysfunction or deficiency, like acquired TTP can be remitting diseases with fluctuating ADAMTS13 immune complex burden during progression of the disease. Therefore, the inventive method may be used to routinely monitor a patient for the presence of immune complexes and free anti-ADAMTS13 antibodies or immune complexes alone. Repeated determinations can be daily or in, 2 days, 1 week, 2 week, 1 month, 2 month, 3 month or longer or shorter intervals. The inventive method may also be used to monitor the presence of anti-ADAMTS13 antibodies or immune complexes during a treatment to recognize progress or adverse reactions. A treatment may include an ADAMTS13 substitution therapy, especially the administering ADAMTS13 to said patient. ADAMTS13 may be administered in form of a plasma infusion (e.g. of fresh or frozen plasma), plasmapheresis, or treatment with a recombinant or plasma derived ADAMTS13 containing medicament. In such diagnostic or monitoring methods, the ratio of free anti-ADAMTS13 to anti-ADAMTS13 in CIC may be determined.

Examples

The present invention is further illustrated by the following examples, however without being limited to these embodiments. It is understood that the examples and embodiments described herein are for illustrative purposes only.

Example 1: Recombinant ADAMTS13 (rADAMTS13)

Recombinant human ADAMTS13 with and without His-tag was expressed in human embryonic kidney cells (HEK293) or in Chinese hamster ovary cells (CHO), e.g. clone 938, as described (Plaimauer, 2002; WO2004/095027; WO02/42442). C-terminally His-tagged ADAMTS13 was constructed by the in-frame fusion of 6 histidine and 3 glycine residues as a linker with the C-terminal threonine residue (Thr-1427) of full-length ADAMTS13.

Example 2: Anti-ADAMTS13 antibodies

Polylconal anti-ADAMTS13 antibodies were either purchased (Abcam, ab28273) or produced by immunisation of rabbits: 1 to 10 male New Zealand White rabbits were immunized with rADAMTS13 according to example 1. 200 $\mu$l injection solution consisted of 100 $\mu$l buffer solution (860 $\mu$g rADAMTS13 in 20mM Histidine, 150 mM NaCl, 0.05 % Tween 80, 2% Saccharose, 2mM CaCl, pH 7.0) and 100 $\mu$l Adjuvant (Freund's Complete Adjuvant, CFA,[2] for prime immunisation; Incomplete Freund's adjuvant, IFA, for booster immunisations). In 3 week intervals one prime and two booster immunisations were administered subcutaneous in 2-4 spots on thighs. Blood was withdrawn 3 weeks after the first booster and 2 and 3 weeks after the second booster.

Three different lots were produced, lot "K1-4" originating from pooled sera of 4 rabbits immunized with His-tagged rADAMTS13, lot "K6" originating from the serum of one rabbit immunized with His-tagged rADAMTS13 and lot "K1-10" originating from pooled sera of 10 rabbits immunized with rADAMTS13.

Monoclonal anti-ADAMTS13 antibodies were purchased from American Diagnostica (antibody clones 5.1 and 12.1) or from Hycultbiotechnology (antibody 5C11).

Antibodies were further purified on a Protein G-sepharose column: Immune sera were centrifuged for 10 min at 13000 RPM and each 10ml of the supernatant was diluted with 20ml PBS. Serum can be stored in this condition at 4°C. All anti-ADAMTS13 antibody sera were purified by elution on a protein G column: All purification steps were done on an AktaExplorer 100 chromatographic system. Protein G Sepharose was filled into a column and equilibrated in PBS, pH 7.1. Sera were loaded onto the column and washed with PBS buffer. Elution was effected with Elution Buffer (100mM glycine, pH 2.8). Eluted antibody fractions were collected and stored at -20°C for later use.

Example 3: Affinity purification of anti-ADAMTS13 antibodies

rADAMTS13 according to example 1 was coupled to NHS activated sepharose (Sepharose 4FF from GE Healthcare). To this end, 25 ml of NHS-activated Sepharose was washed and activated with 1mM HCl, pH 3.3. After centrifugation, 25ml rADAMTS13 was added in Coupling Buffer (100mM $NaHCO_3$, 500mM NaCl, pH 8.3) and incubated over night at 4°C. Sepharose[3] was centrifuged, washed and incubated for 2h with Blocking Buffer (100mM Tris-HCl, pH 8.5) at room temperature. The sepharose gel is subsequently moved into a column that was then filled and washed with Coupling Buffer. Further washing steps of the column included 3 cycles of washing with Blocking Buffer and Acetate Buffer (acetate, pH 4.5).

After purification on a Protein G column, anti-ADAMTS13 antibodies according to example 2 were loaded onto the rADAMTS13-NHS-activated sepharose column and washed with Equilibration buffer in the presence of $Zn^{2+}$ and $Ca^{2+}$ (TBS, pH 8.4). Purified antibodies were eluted with Elution Buffer 1 (100mM glycine, pH 2.8) and Elution Buffer 2 (2M NaCl, 100mM glycine, 50% ethyleneglycole, pH 5) and collected. 11 mg affinity purified antibody was recovered from 40 mg Sepharose purified antibody after affinity purification.

Example 4: Anti-Ig antibodies

Anti-immunoglobulin (Ig) antibodies with Ig class or subtype specificity were purchased.

Mouse anti-human IgG1 antibodies were purchased from Zymed,; mouse anti-human IgG2 antibodies were purchased from Southern Biotech,; mouse anti-human IgG3 antibodies were purchased from Zymed,; mouse anti-human IgG4 antibodies were purchased from Zymed(Invitrogen) anti-human IgG antibodies were purchased from AbD Serotec,; goat anti-human IgM antibodies were purchased from SIGMA,; mouse anti-human IgA antibodies were purchased from SIGMA.

Mouse anti-human IgG4-HRP was further purchased from Alpha Diagnostics, ab 10124.

For use as secondary antibodies anti-Ig antibodies were purchased with an alkaline phosphatase label or HorseRadish-Peroxidase (HRP) label.

Example 5: Plasma samples

Sample plasma was obtained from healthy persons as negative controls (NHP, platelet-poor pooled normal human plasma of 35 different healthy donors, George King Bio-Medical, Inc.) and from patients diagnosed with acquired TTP. TTP plasma was provided from the Vienna General Hospital. TTP patients were in part subject to plasmapheresis therapy. Plasma may be obtained from blood samples or from plasmapheresis bags to monitor ADAMTS13 immune complex reduction during therapy. As positive control samples TTP patient blood samples were used.

Example 6: Anti-ADAMTS13 antibody detection

Recombinant ADAMTS13 according to example 1 was coated onto ELISA plates (Maxisorp; Nunc, Rochester, NY) in a concentration of 2 $\mu$g/ml by applying 100 $\mu$l per well in Coating Buffer (0.05M carbonate-bicarbonate buffer, pH 9.6, Sigma). Plates were incubated at 4°C overnight or for 6h at room temperature followed by washing 4 times with Washing Buffer (PBS) in volumes of 300$\mu$l per well in an auto strip plate washer. Free binding sites were blocked with Blocking Buffer (Protein-free T20 (PBS), Pierce) 250 $\mu$l per well and incubation for 2h at room temperature. Blocking is followed by washing 4 times with Washing Buffer (PBS) in volumes of 300$\mu$l per well in an auto strip plate washer. Plasma samples as well as controls were serially diluted 1:25 to 1:6400 in sample buffer (Protein-free T20, Pierce, +0.5 M NaCl), applied in amounts of 100 $\mu$l/well and incubated overnight at 4°C or for 3 hours at room temperature. Sample incubation is followed by washing 4 times with Washing Buffer (PBS) in volumes of 300$\mu$l per well in an auto strip plate washer. HRP-labelled anti-immunoglobulin antibodies according to example 4 are applied in dilution (anti-IgG1 1:2000, anti-IgG2 1:500, anti-IgG3 1:3000, anti-IgG4 1:3000, anti-IgG 1:30000, anti-IgM 1:30000 anti-IgA 1:30000) in Sample Buffer, applied in amounts of 100 $\mu$l per well and incubated for 2 h at room temperature. Antibody incubation is followed by washing 4 times with Washing Buffer (PBS) in volumes of 300$\mu$l per well in an auto strip plate washer. For HRP reaction, equal volumes of each of TMB Solution and Peroxide solution (Immuno Pure TMB Substrate Kit, Pierce) were added in a total substrate volume of 100$\mu$l/well and incubated for 5-30 min at room temperature. Normal development times for different secondary antibodies are anti-IgG: 5-6min, anti-IgM: 5-10min, anti-IgA: 15-30min, anti-IgG1 10-15min, anti-IgG2 10-15min, anti-IgG3 10-15min, anti-IgG4 5-10min. The color changes are from clear to brilliant blue while the reaction should be stopped before any well displays a green color. Reaction was stopped by addition of 100$\mu$l 1.9 $H_2SO_4$. Color reaction was measured at 450 nm versus 620 nm on a Tecan ELISA-Reader. Results are calculated as ratio between Sample OD and Control OD.

Example 7: ADAMTS13 immune complex detection

Anti-ADAMTS13 antibodies according to example 2 or 3 were diluted to a concentration of 1-2$\mu$g/ml with Coating Buffer (0.05M Carbonate - Bicarbonate Buffer pH 9,6, 25°C) and coated onto the surface of ELISA plates (Maxisorp;

Nunc, Rochester, NY) in a volume of 100 μl/well over night at 4°C or for 5-6 hours at room temperature. After washing (4 times with Wash Buffer: PBS (PBS, Invitrogen, 300 μl/well), free binding sites were blocked for 2 hours at room temperature with 250 μl/well Blocking Buffer (PBS-T:PBS with 0.01% Tween 20, Biorad, with 0.5% Non Fat Dry Milk, pH 7.4-7.5, BioRad).

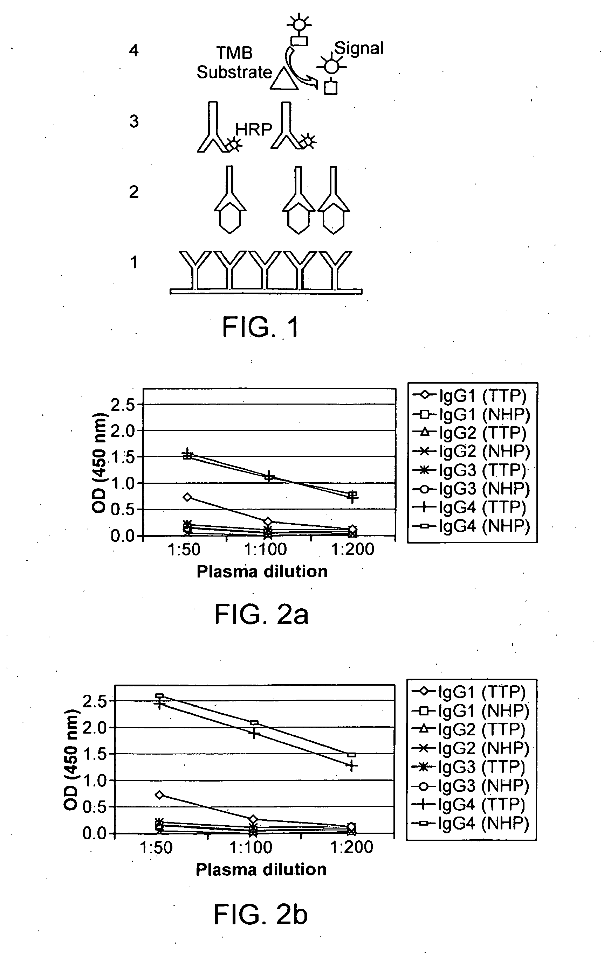

Samples were diluted in Dilution Buffer (PBS-T with 0.5 % Non Fat Dry Milk, optionally with 0.1 to 1 M NaCl) serial-ly from 1:25 to 1:400. Diluted samples were added (100μl/well) and incubated overnight at 4°C. Wells were washed 4 times with 300 μl/well Wash Buffer. Anti-Ig antibodies according to example 4 were added as secondary antibodies (diluted in Dilution Buffer; anti-IgG1 1:2000, anti-IgG2 1:2000, anti-IgG3 1:1000, anti-IgG4 1:4000, anti-IgM 1:80000 anti-IgA 1:25000), 100 μl/well and incubated for 3h at room temperature. The wells were washed 4 times with 300μl/well Washing buffer. Equal volumes of each of TMB Solution and Peroxide solution (Immuno Pure TMB Substrate, Pierce) were added in a total substrate volume of 100μl/well and incubated for 5-30 min. Normal development times for different secondary antibodies are anti-IgG1, -IgG2, -IgG3, -IgA and -IgM: 20min, anti-IgG4: 10-20min. The color changes are from clear to brilliant blue while the reaction should be stopped before any well displays a green color. Reaction was stopped by addition of 100μl 1.9 or 2M $H_2SO_4$. Color reaction was measured at 450 nm versus 620 nm on a Tecan ELISA-Reader. Results are calculated as ratio between Sample OD and Control OD. A schematic representation of this method is shown in fig. 1.

Example 8: ADAMTS13 immune complex ELISA with polyclonal antibodies

Polyclonal anti-ADAMTS13 antibodies "abeam" and "K1-4" were purchased or obtained as described in example 2 and coated onto Nunc Maxisorp ELISA Plates as described in example 7 with the following modifications to the procedure: Antibodies abcam and K1-4 were used as coating antibodies in concentrations of 1 mg/ml and 3,356 μg/ml, respectively, in amounts of 10 μl or 3 μl antibody solution, respectively, with 5 ml Coating Buffer. Incubation was over night at 4°C. Dilution Buffer was used without NaCl (thus being identical to Blocking Buffer). TTP plasma and NHP plasma was used as positive and negative ADAMTS13:ic samples. Samples were diluted 1:50, 1:100 and 1:200 with Blocking Buffer and incubated for 3h at room temperature. As secondary detection antibody, IgG1-HRP (Zymed), IgG2-HRP (SouthernBiotech), IgG3-HRP (Zymed) and IgG4-HRP (Zymed) were used in 1:2000 dilutions together with Blocking Buffer (1.5 μl antibody dilution with 3ml Blocking Buffer) and incubated for 2h at room temperature. After washing, coloring reaction was performed by incubation with TMB Solution and Peroxide Solution for 15-30 min at room temperature.

Results are given in table 1 below and shown in figure 2 (Fig. 2a: polyclonal antibody abcam; Fig. 2b polyclonal antibody K1-4). In particular in case of IgG4, very high background signals were shown for negative NHP samples. This effect might be due to unspecific binding of IgG4 to IgG (Ito, 2010).

Table 1:

| | | TTP | | | NHP | | | |
| --- | --- | --- | --- | --- | --- | --- | --- | --- |
| | | 1:50 | 1:100 | 1:200 | 1:50 | 1:100 | 1:200 | Average Blank |
| I :Rbt pAb to ADAMTS13 (abcam) raised against full length hADAMTS13 C-terminal end | IgG1 | 0,713 | 0,2584 | 0,105 | 0,1741 | 0,1122 | 0,0892 | 0,04735 |
| | IgG2 | 0,1994 | 0,0847 | 0,0244 | 0,0489 | 0,0292 | 0,0216 | 0,02 |
| | IgG3 | 0,198 | 0,0947 | 0,0437 | 0,1107 | 0,0565 | 0,0334 | 0,01815 |
| | IgG4 | 1,5718 | 1,1323 | 0,6832 | 1,4646 | 1,0863 | 0,7692 | 0,0921 |
| II: K1-4 | IgG1 | 0,7372 | 0,292 | 0,1158 | 0,1611 | 0,1101 | 0,0829 | 0,05925 |
| | IgG2 | 0,5936 | 0,0629 | 0,0266 | 0,038 | 0,0286 | 0,0248 | 0,0199 |
| | IgG3 | 0,0882 | 0,1206 | 0,0542 | 0,0786 | 0,05 | 0,0315 | 0,01975 |
| | IgG4 | 2,5972 | 1,9351 | 1,2918 | 2,6489 | 2,1579 | 1,5111 | 0,14675 |

Example 9: IgG4 ELISA with different antibodies

Polyclonal anti-ADAMTS13 antibodies "abcam", "K1-4" and "K-6" and monoclonal antibody clones 5.1 and 12.1 were purchased or obtained as described in example 2 and coated onto Nunc Maxisorp ELISA Plates as described in example 7 with the following modifications to the procedure: Antibodies were used as coating antibodies in the following concentrations: abcam: 1 mg/ml, K1-4: 3,356 μg/ml, K6: 13148 μg/ml, clone 5.1: 0.2 mg/ml, clone 12.1: 0.2 mg/ml. Antibody solutions were used in amounts of: abcam: 10 μl, K1-4: 3 μl, K6: 0.8 μl, clone 5.1: 50 μl, clone 12.1 50 μl, with 10 ml Coating Buffer. Incubation was over night at 4°C. Dilution Buffer was used with 0.6 M NaCl. Three sample compounds were investigated at different dilutions: 1) A pool of IgG4 antibodies (1:1; human IgG4 kappa, Sigma, 1 mg/ml; human IgG4 lambda, Sigma, 1 mg/ml), 2) human albumin (Sigma) and 3) ADAMTS3 produced in CHO cells according to example 1. Samples were diluted to concentrations of 20 μg/ml to 0.625 μg/ml with Dilution Buffer and incubated for 5h at room temperature. As secondary detection antibody, mouse anti-human IgG4-HRP (Alpha Diagnostics) was used in 1:1000 dilution together with Dilution Buffer (50 μl antibody solution with 50ml Blocking Buffer) and incubated for 2h at room temperature. After washing, coloring reaction was performed by incubation with TMB Solution and Peroxide Solution for 15 min at room temperature.

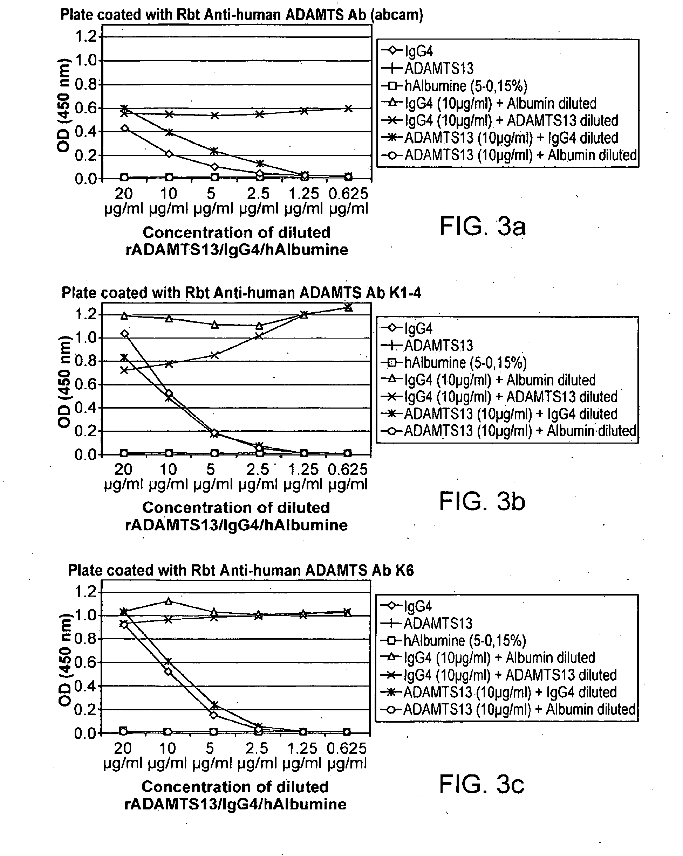

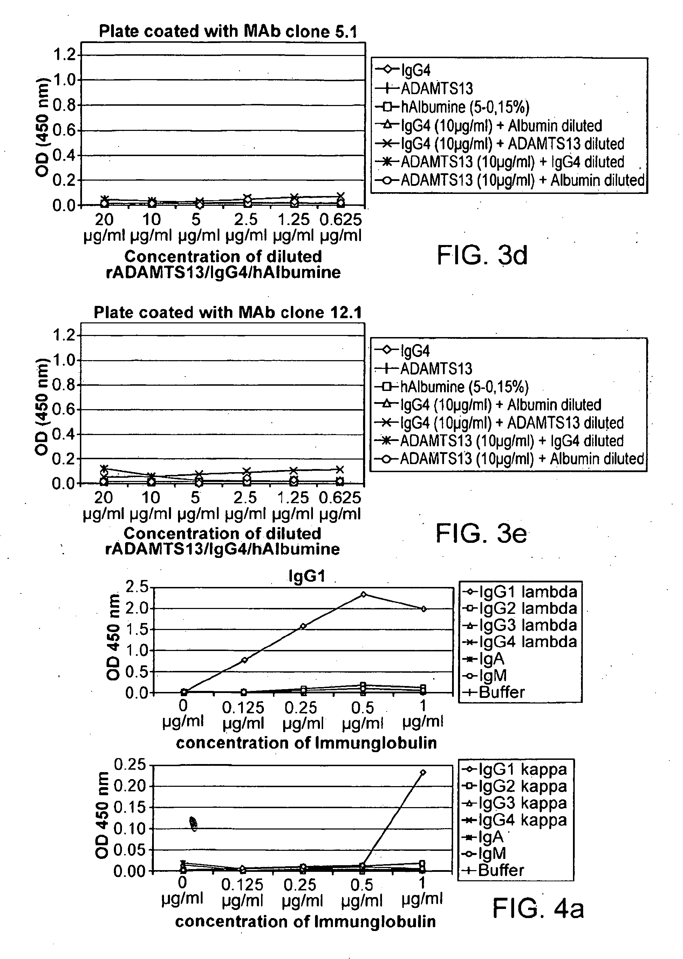

Results are shown in figure 3 (Fig. 3a: polyclonal antibody abcam; Fig. 3b polyclonal antibody K1-4; Fig. 3c polyclonal antibody K6; Fig. 3d monoclonal antibody 5.1; Fig. 3e monoclonal antibody 12.1;). The results illustrate that for all polyclonal antibodies high anti-IgG4 signals were detected, even in the absence of ADAMTS13 in the sample. The effects were lower for commercial antibody abcam. In case of coating with monoclonal antibodies, no adsorption of IgG4 was detected. It could be concluded that IgG4 background signals were due to the nature of the multi-epitope specificity of polyclonal antibodies.

Example 10: IgG ELISA, cross-reactivity of secondary antibodies

Cross-reactivities of subtype specific antibodies were tested by coating Ig of distinct classes and subtypes onto Nunc Maxisorp ELISA plates as described in example 7. No samples were used. Immediately after coating and incubation (over night at 4°C), incubation and signal generation with secondary HRP-labeled antibodies followed. The following coating antibodies were used: IgG1: human IgG1 kappa (Sigma, 1.2 mg/ml) and human IgG1 lambda (Sigma, 1.0 mg/ml); IgG2: human IgG2 kappa (Sigma, 1.1 mg/ml) and human IgG2 lambda (Sigma, 1.06 mg/ml); IgG3: human IgG3 kappa (Sigma, 1.1 mg/ml) and human IgG3 lambda (Sigma, 1.04 mg/ml); IgG4: human IgG4 kappa (Sigma, 1.0 mg/ml) and human IgG4 lambda (Sigma, 1.0 mg/ml); IgA: human IgA (Sigma, 1.0 mg/ml); and IgM: human IgM (Sigma, 1.01 mg/ml); Anti-immunoglobulin antibodies with Ig class or subtype specificity according to example 4 were used as secondary antibodies.

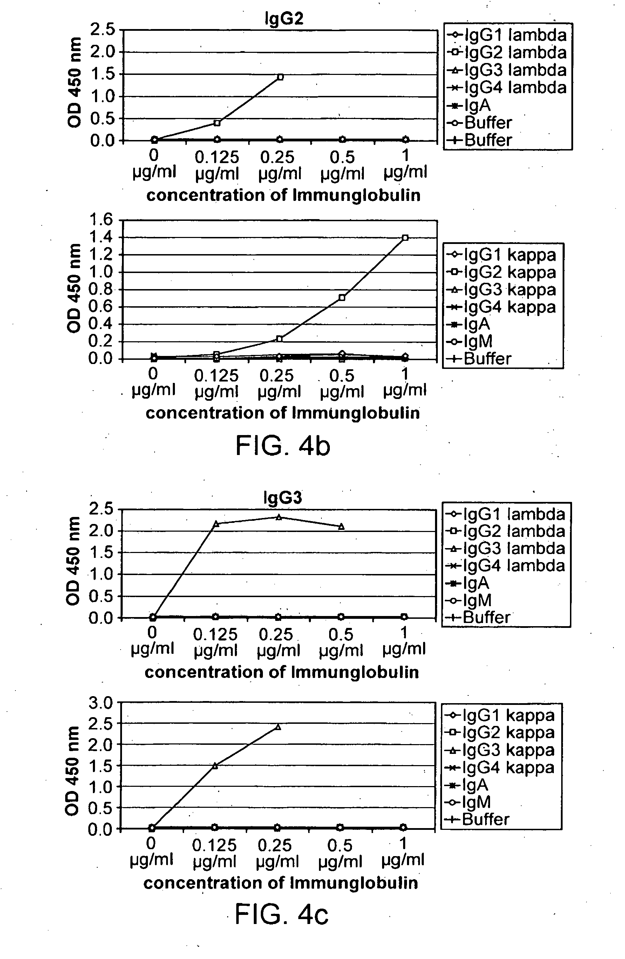

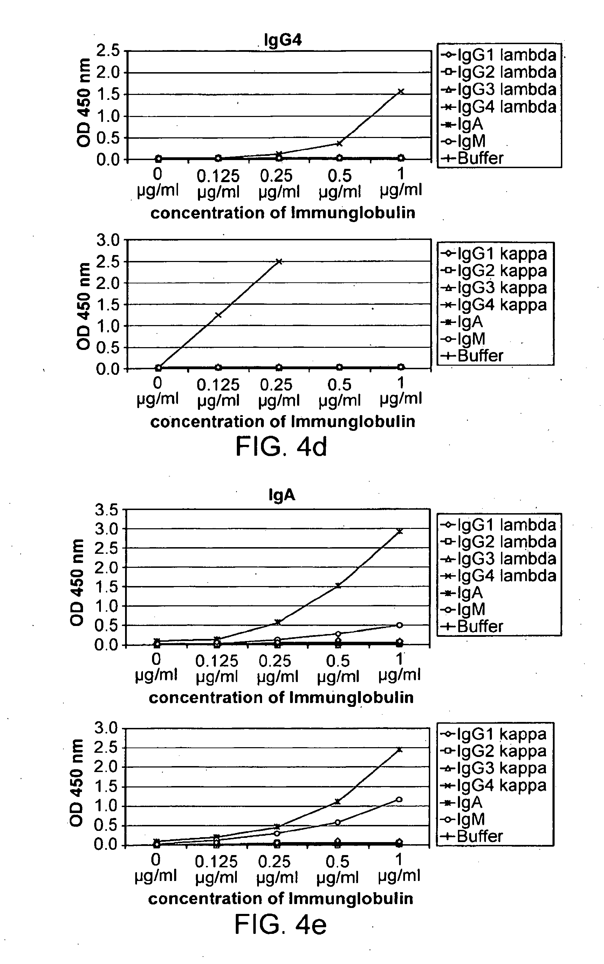

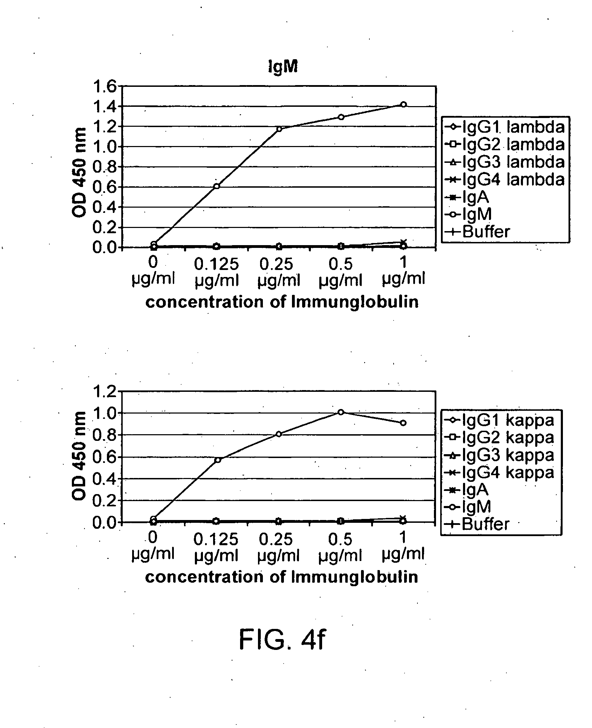

Results are shown in figure 4; Graphs show signals of indicated concentrations of coating antibody (Fig. 4a: anti-human IgG1-HRP, diluted 1:2000; Fig. 4b: anti-human IgG2-HRP, diluted 1:1000; Fig. 4c: anti-human IgG3-HRP, diluted 1:1000; Fig. 4d: anti-human IgG4-HRP, diluted 1:2000; Fig. 4e: anti-human IgA-HRP; diluted 1:1000; Fig. 4f: anti-human IgM-HRP, diluted 1:80000). Distinguished cross-reactivity was observed for anti-human IgA to coated IgM but not for subtype specific antibodies.

Example 11: ADAMTS13 immune complex ELISA with monoclonal antibodies as coating abs

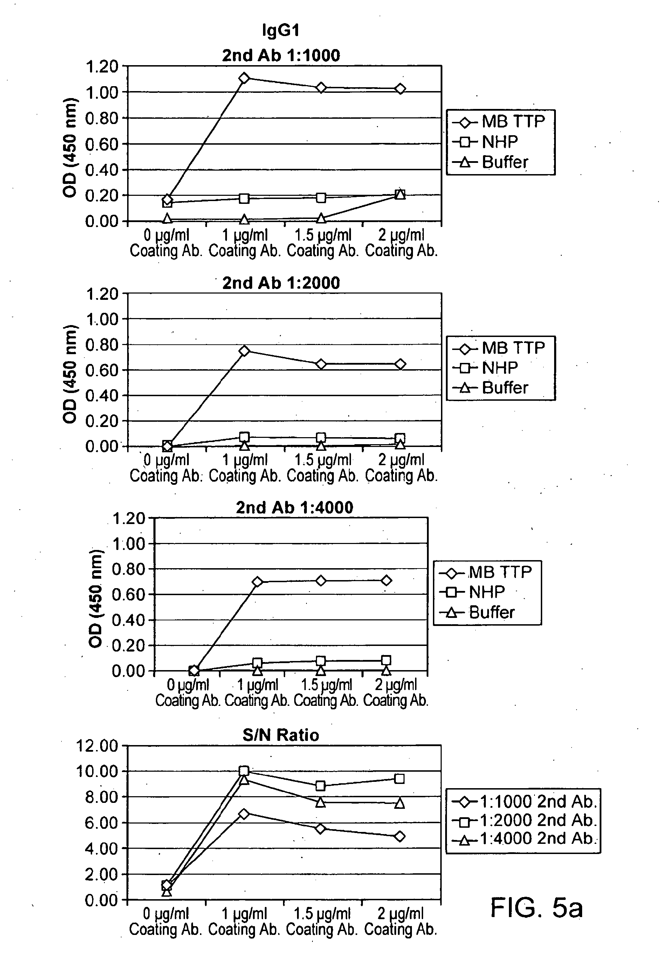

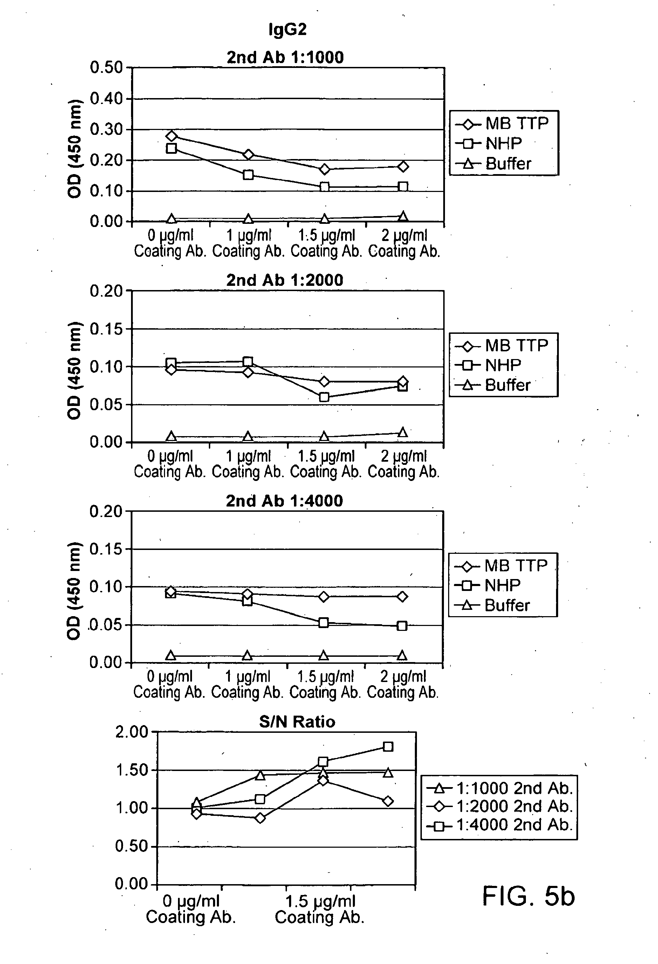

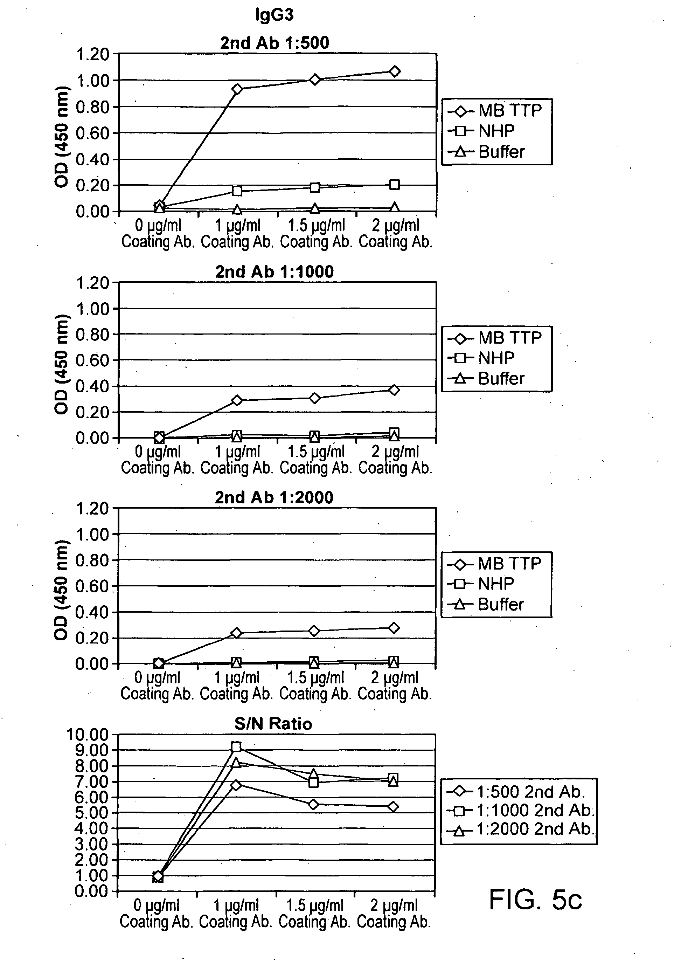

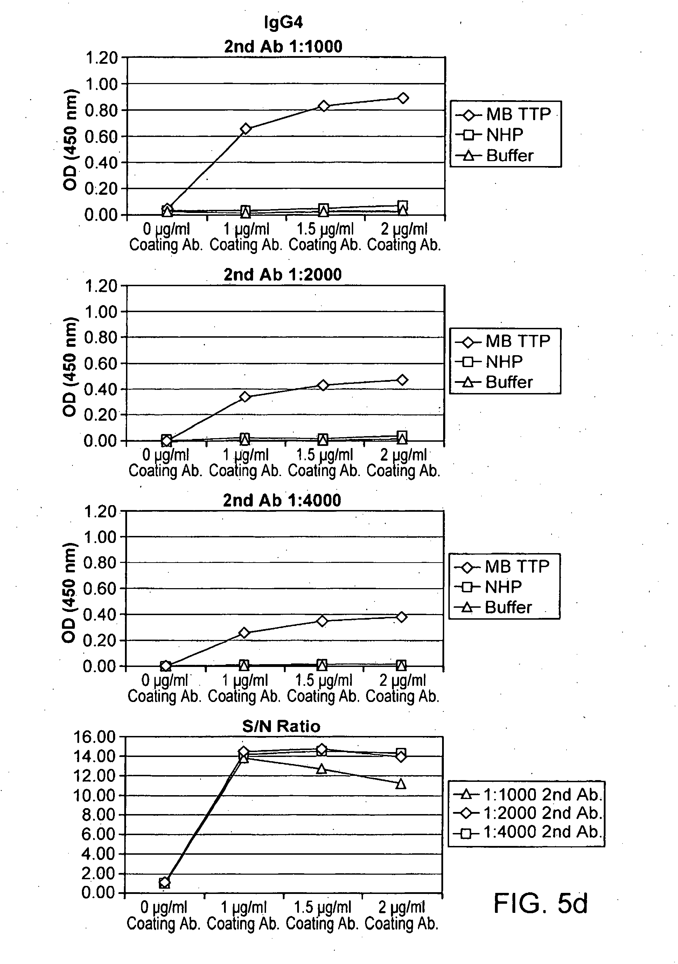

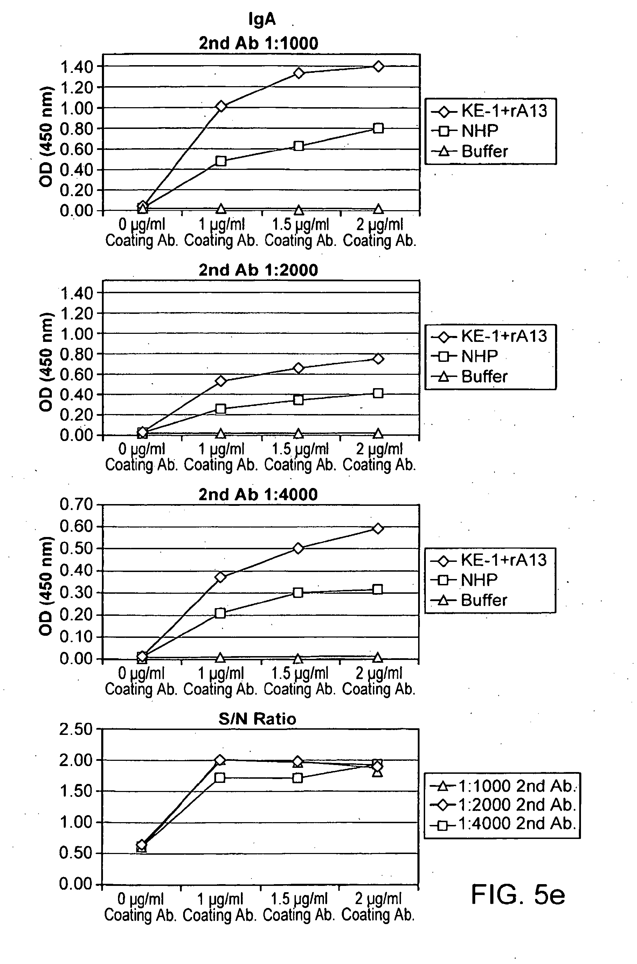

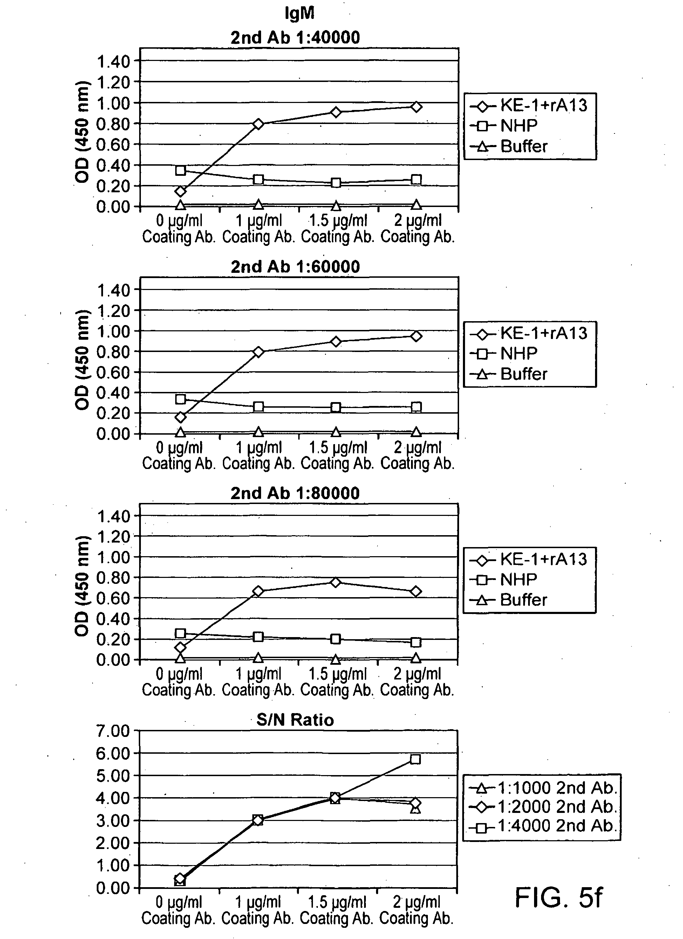

A mixture of monoclonal anti-ADAMTS13 antibodies 5C11, 5.1 and 12.1 of example 2 were coated onto Nunc Maxisorp ELISA Plates as described in example 7 with the following modifications to the procedure: Monoclonal antibodies solutions were used as coating antibodies in the following concentrations: clones 5.1 and 12.1 (1:1): 0.2 mg/ml, 5C11: 0.1 mg/ml. For coating, antibody solutions were diluted with Coating Buffer to concentrations of 1 $\mu$g/ml, 1.5 $\mu$g/ml and 2 $\mu$g/ml (each with 15 ml Coating Buffer). Dilution Buffer was used with 0.6 M NaCl. Three samples were investigated: 1) TTP plasma, 2) NHP plasma and 3) TTP plasma spiked with rADAMTS13 produced in CHO cells according to example 1 (273.5 $\mu$g/ml). Samples were diluted with Dilution Buffer as follows: TTP: 400$\mu$l plasma with 9600 $\mu$l Dilution Buffer; NHP 600$\mu$l plasma with 4800 $\mu$l Dilution Buffer and 37.1 $\mu$l rADAMTS13 with 200 $\mu$l TTP plasma and 4800 $\mu$l Dilution Buffer and incubated over night at 4°C. Anti-immunoglobulin antibodies with Ig class or subtype specificity according to example 4 were used as secondary antibodies. Secondary antibodies were diluted with Dilution Buffer 1:1000 to 1:8000 as shown in fig. 5 and incubated for 3h at room temperature. After washing, coloring reaction was performed by incubation with TMB Solution and Peroxide Solution for 30 min at room temperature.

Results are shown in figure 5 (fig. 5a: anti-IgG1, fig. 5b: anti-IgG2, fig. 5c: anti-IgG3, fig. 5d: anti-IgG4, fig. 5e: anti-IgA, fig. 5f: anti-IgM secondary antibodies). A signal to noise ratio was determined by comparison of signals obtained for TTP (used for IgG1-4 detection) or TTP plasma spiked with rADAMTS13 samples (used for IgA and IgM detection) as positive samples and NHP plasma as negative control. Exceptional s/n rations were observed for IgG1, IgG3 and IgG4. Next, IgM appeared superior over IgA and IgG2.

Example 12: ADAMTS13 immune complex ELISA with affinity purified polyclonal antibodies as coating abs

Monoclonal antibody 5C11 and polyclonal anti-ADAMTS13 antibody K1-4 were purchased or obtained as described in example 3 and coated onto Nunc Maxisorp ELISA Plates as described in example 7 with the following modifications to the procedure: For coating, polyclonal antibody K1-4 solutions were diluted 1:1000 and monoclonal antibody 5C11 solution 1:100 with Coating Buffer. Dilution Buffer was used with 0.6 M NaCl. As positive sample, TTP plasma spiked with 500ng/$\mu$l rADAMTS13 produced in CHO cells according to example 1 and as negative sample NHP plasma was used. Samples were diluted with Dilution Buffer 1:25, 1:50 and 1:100 with Dilution Buffer and incubated over night at 4°C. Anti-immunoglobulin antibodies with Ig class or subtype specificity according to example 4 were used as secondary antibodies. Secondary antibodies were diluted with Dilution Buffer to 1:2000 (IgG1, IgG3, IgG4 and IgA), 1:1000 (IgG2 and IgG3) or 1:80000 (IgM) and incubated for 3h at room temperature. After washing, coloring reaction was performed by incubation with TMB Solution and Peroxide Solution for 15 min at room temperature.

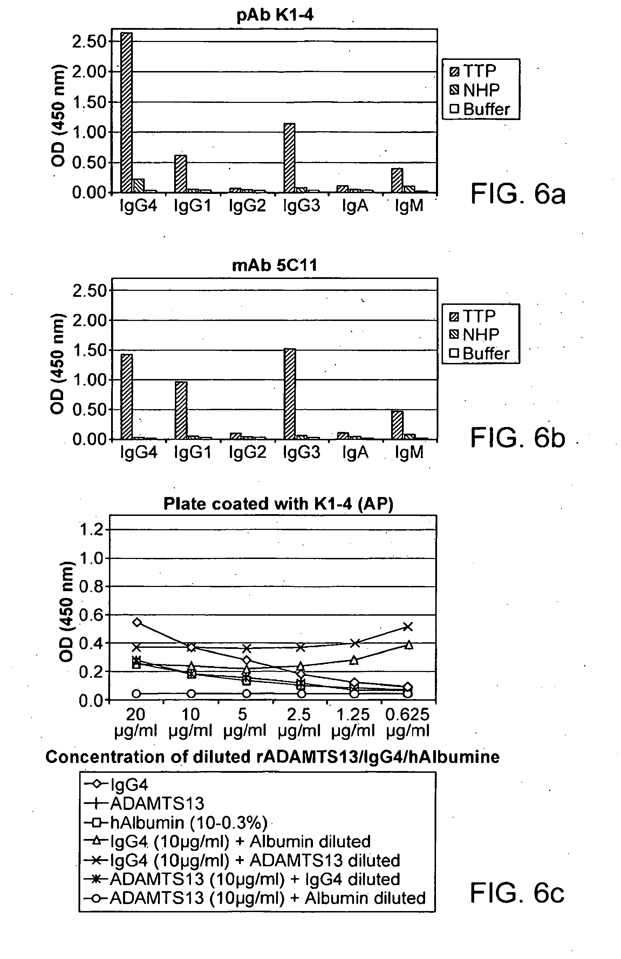

Results are shown in figure 6 (fig. 6a: polycolonal Ab K1-4, 6b: monoclonal Ab 5C11). Surprisingly, after affinity purification of pAb K1-4 best results were achieved with anti-IgG4 antibodies as secondary detection antibodies with low s/n ratio and highest signals. Very good results could be shown for antibodies IgG1, IgG3 and IgM, being superior over IgA and IgG2. For monoclonal antibody 5C11 similar good results are shown for IgG4, IgG1, IgG3 with acceptable results for IgM being superior over IgG2 and IgA (also cf. example 11).

IgG4 ELISA test according to example 9 was repeated for affinity purified antibody K1-4. Results are shown in fig. 6c (cf. fig. 3b). Unspecific reactivity could be significantly reduced through affinity purification.

Example 13: ADAMTS13 immune complex ELISA with anti-human IgG-HRP secondary antibodies (not-subtype specific)

Polyclonal anti-ADAMTS13 antibody K1-10 was obtained and purified as described in example 3 and coated onto Nunc Maxisorp ELISA Plates as described in example 7 with the following modifications to the procedure: the initial antibody solution with a concentration of 3.135 mg/ml was diluted with 10 ml Coating Buffer to concentrations of 1 or 2 $\mu$g/ml. Incubation was for 6 hours at room temperature. Dilution Buffer was used with 0.6 M NaCl. As positive samples, TTP plasma and TTP plasma spiked with 300ng/$\mu$l rADAMTS13 produced in CHO cells according to example 1 (initial concentration 273.5 $\mu$g/ml) were used and as negative sample, NHP plasma or Buffer were used. Samples were diluted 1:25 and 1:50 with Dilution Buffer and incubated over night at 4°C. As secondary detection antibody, mouse anti-human IgG-HRP (SouthernBiotech,) was used in 1:32000, 1:64000 and 1:128000 dilutions with 5 ml Dilution Buffer. Samples were incubated for 3h at room temperature. After washing, coloring reaction was performed by incubation with TMB Solution and Peroxide Solution for 20 min at room temperature.

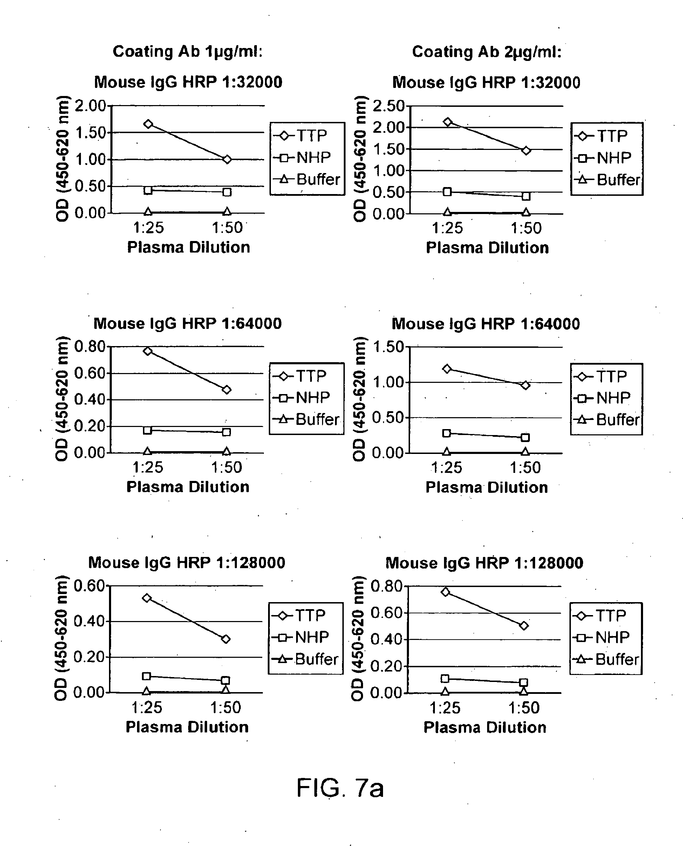

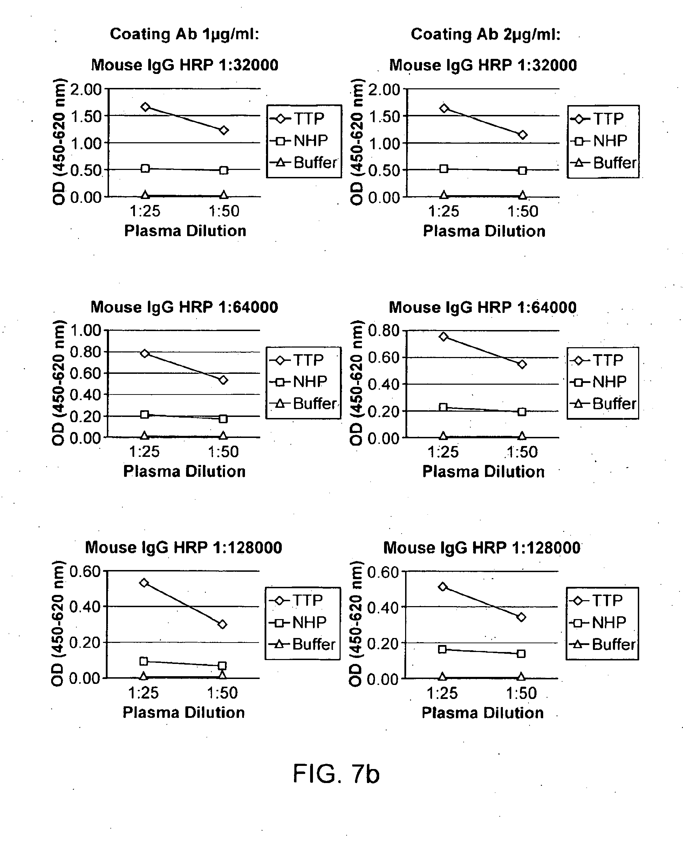

Results are shown in fig. 7 (fig. 7a: rADAMTS13 TTP sample vs. NHP plasma; fig. 7b: TTP plasma vs. NHP plasma). This example shows that IgG class specific antibodies suffer from high background signals for ADAMTS13-negative NHP samples.

Example 14: ADAMTS13 immune complex ELISA with affinity purified polyclonal antibodies as coating abs

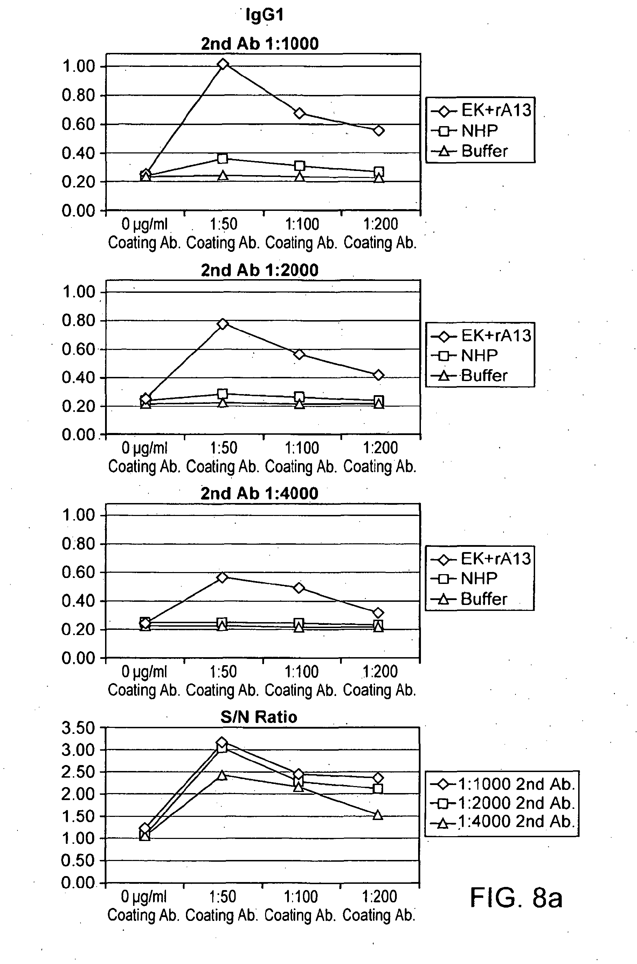

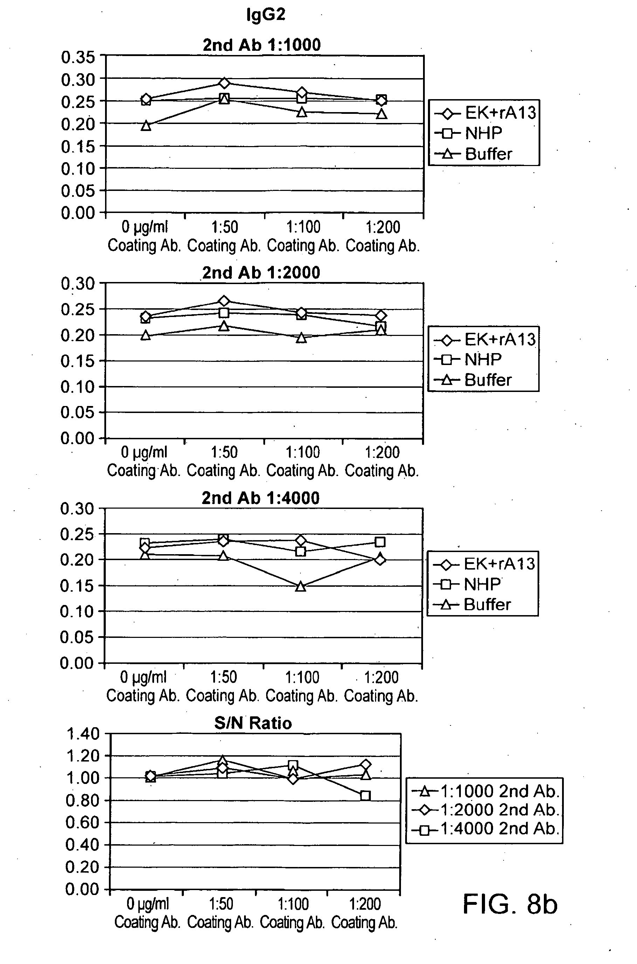

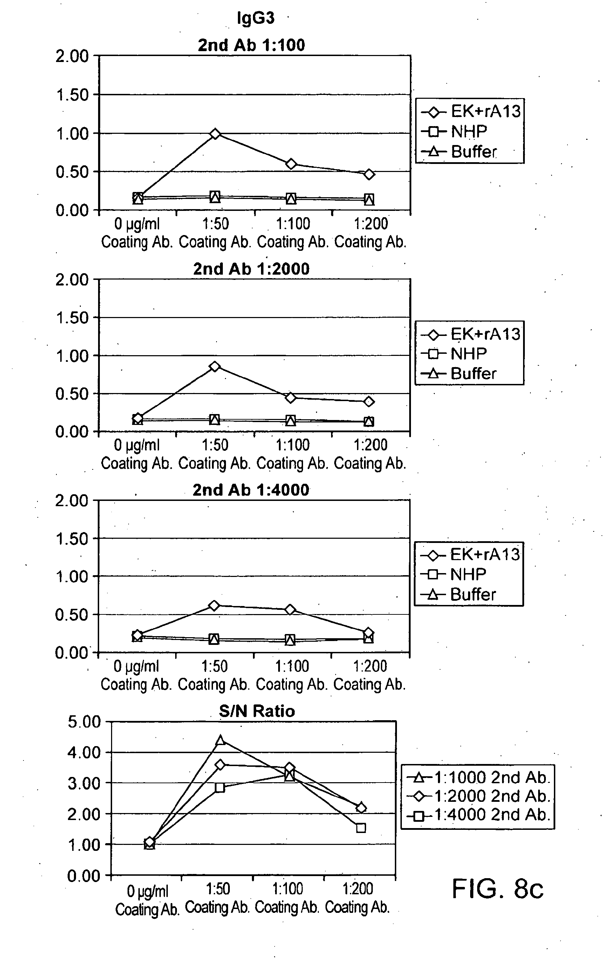

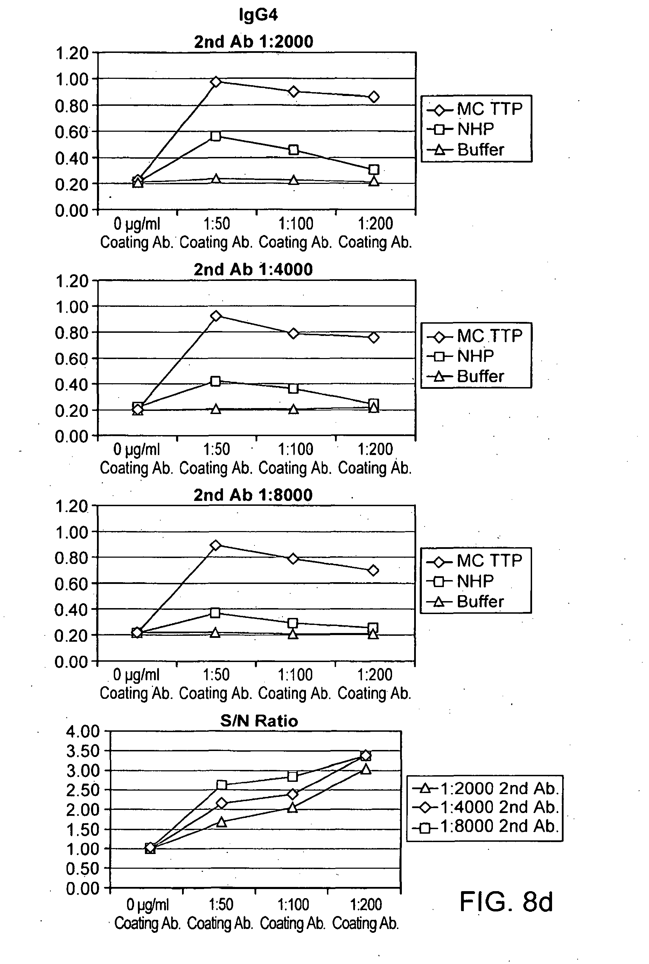

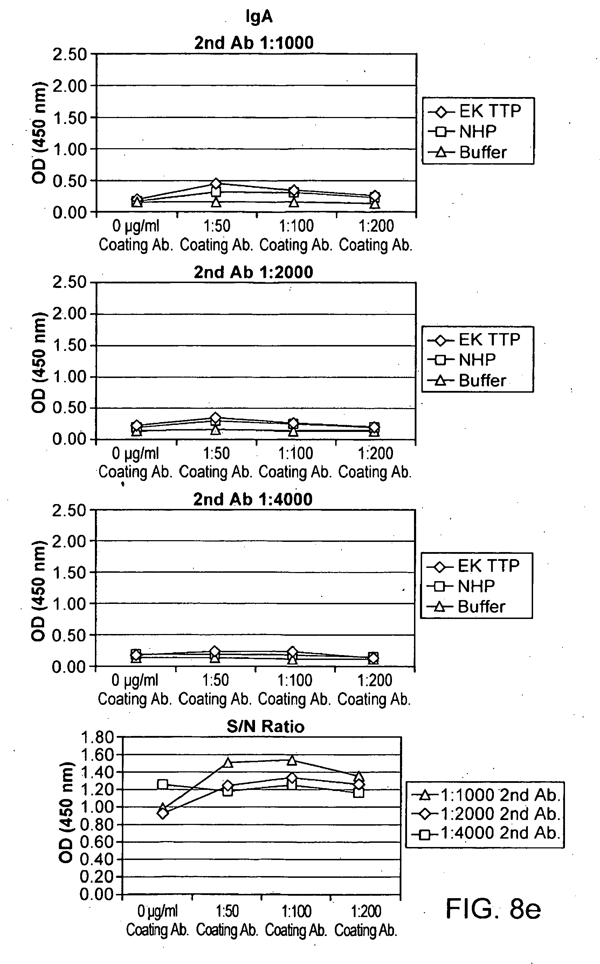

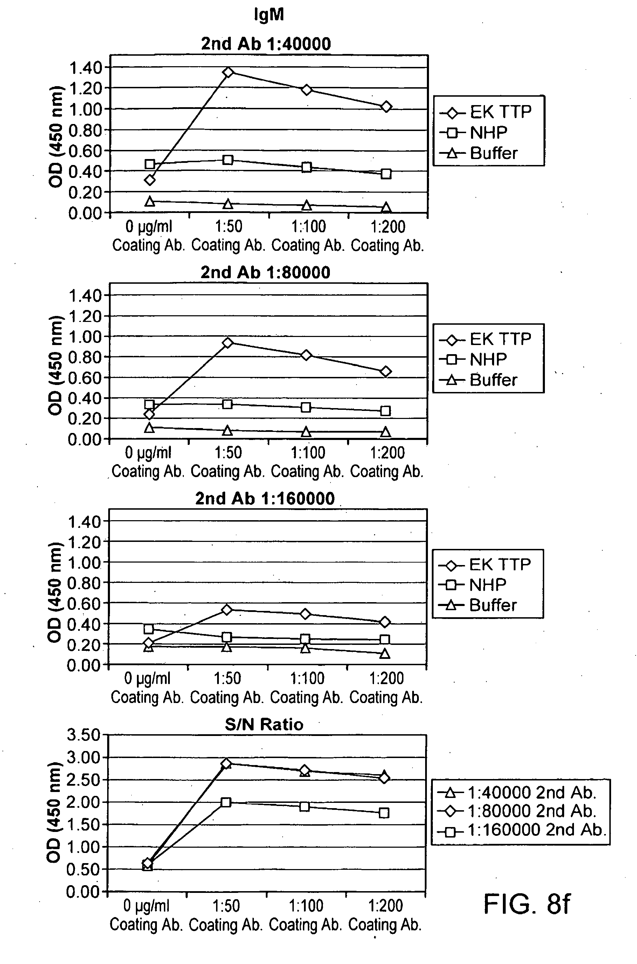

Affinity purified polyclonal anti-ADAMTS13 antibody K1-4 of example 3 was coated onto Nunc Maxisorp ELISA Plates as described in example 7 with the following modifications to the procedure: Polyclonal antibody solutions were used as coating antibodies in the following concentrations: For coating, initial antibody solutions (180 $\mu$g/ml) were diluted 1:50 and 1:100 with Coating Buffer. Dilution Buffer was used with 0.6 M NaCl. Three samples were examined: 1) TTP plasma, 2) NHP plasma and 3) TTP plasma spiked with rADAMTS3 produced in CHO cells according to example 1 (273.5 $\mu$g/ml). Samples were diluted with Dilution Buffer as follows: TTP: 100$\mu$l plasma with 2400 $\mu$l Dilution Buffer; NHP 600$\mu$l plasma with 11900 $\mu$l Dilution Buffer and 220 $\mu$l rADAMTS13 with 500 $\mu$l TTP plasma and 12 ml Dilution Buffer and incubated over night at 4°C. Anti-immunoglobulin antibodies with Ig class or subtype specificity according to example 4 were used as secondary antibodies. Secondary antibodies were diluted with Dilution Buffer 1:1000 to 1:8000 as shown in fig. 8 and incubated for 3h at room temperature. After washing, coloring reaction was performed by incubation with TMB Solution and Peroxide Solution for 30 min at room temperature.

Results are shown in figure 8 (fig. 8a: anti-IgG1, fig. 8b: anti-IgG2, fig. 8c: anti-IgG3, fig. 8d: anti-IgG4, fig. 8e: anti-IgA, fig. 8f: anti-IgM secondary antibodies). A signal to noise ratio was determined by comparison of signals obtained for TTP (used for IgG1-4 detection) or rADAMTS13 samples (used for IgA and IgM detection) as positive samples and NHP plasma as negative control.

Example 15: ADAMTS13 immune complex ELISA with affinity purified polyclonal and monoclonal antibodies as coating abs

Affinity purified polyclonal anti-ADAMTS13 antibody K1-4 of example 3 and a mixture of monoclonal antibodies 5C11, 5.1 and 12.1 were coated onto Nunc Maxisorp ELISA Plates as described in example 7 with the following modifications to the procedure: Polyclonal antibody solutions were used as coating antibodies in the following concentrations: For coating, initial antibody K1-4 solutions (180 $\mu$g/ml) and were diluted 1:50-100$\mu$l solution in 10 ml Coating Buffer - for IgGl-3, IgA and IgM testing and 10$\mu$l in 2ml Coating Buffer for IgG4 testing. 50 $\mu$l of monoclonal antibody 5C11 (0.1 mg/ml) and 25 $\mu$l of both of mab 5.1 and 12.1 (0.1 mg/ml each) were mixed in 10 ml Coating Buffer. Dilution Buffer was used with 0.6 M NaCl. Three samples were examined: 1) TTP plasma spiked with 100 ng/100$\mu$l rADAMTS13, 2) NHP plasma and 3) rADAMTS13 produced in CHO cells according to example 1 (273.5 $\mu$g/ml). Samples were diluted with Dilution Buffer 1:25 (20$\mu$l plasma and 480 $\mu$l Dilution Buffer) to 1:100 and incubated over night at 4°C. Anti-immunoglobulin antibodies with Ig class or subtype specificity according to example 4 were used as secondary antibodies. Secondary antibodies were diluted with Dilution Buffer (IgG1, IgG4 and IgA: 2.5$\mu$l antibody solution in 5ml Dilution Buffer (DB), IgG2 and IgG3: 5$\mu$l antibody solution in 5 ml DB, IgM: 6.3$\mu$l antibody solution in 5 ml DB) and incubated for 3h at room temperature. After washing, coloring reaction was performed by incubation with TMB Solution and Peroxide Solution for 15 min at room temperature.

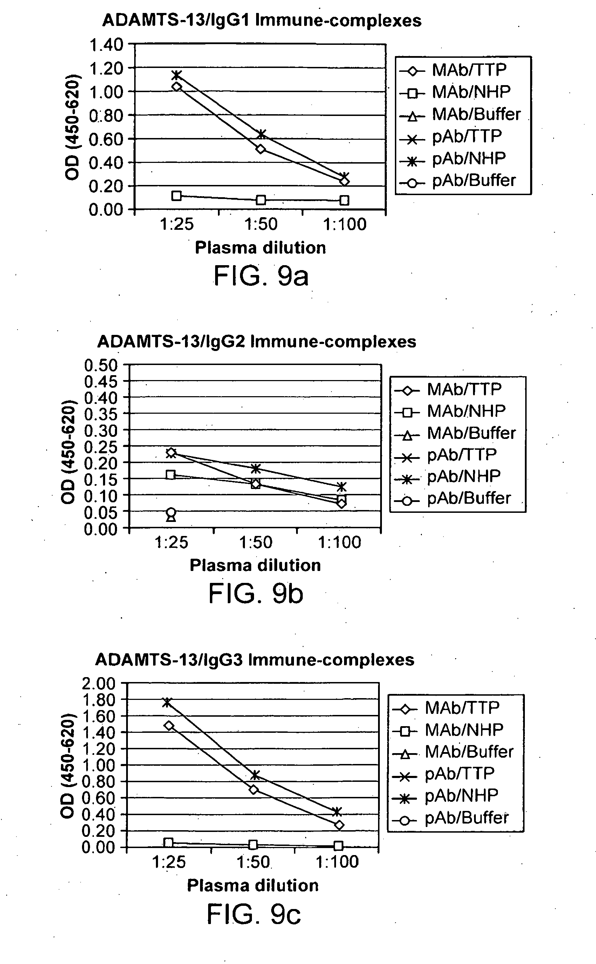

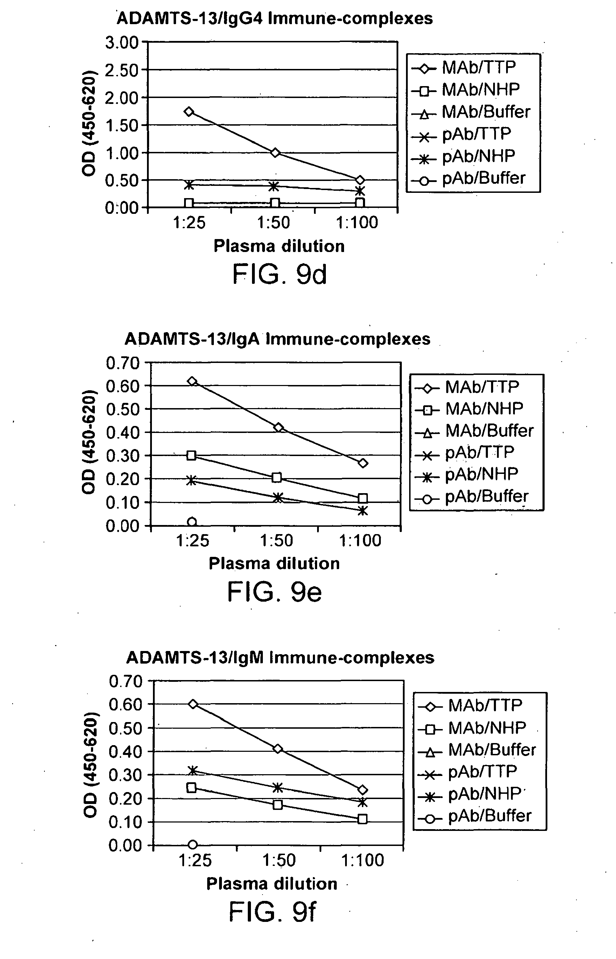

Results are shown in figure 9 (fig. 9a: anti-IgG1, fig. 9b: anti-IgG2, fig. 9c: anti-IgG3, fig. 9d: anti-IgG4, fig. 9e:

anti-IgA, fig. 9f: anti-IgM secondary antibodies). For most antibody subtypes, the use of (affinity purified) polyclonal antibodies as coating antibodies resulted in higher signals as compared to monoclonal antibodies.

Example 16: ADAMTS13 immune complex ELISA with different affinity purified polyclonal antibodies

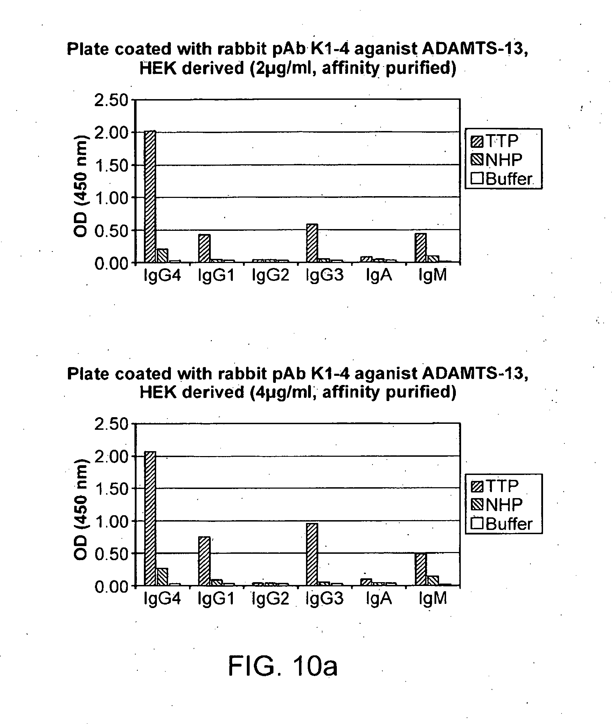

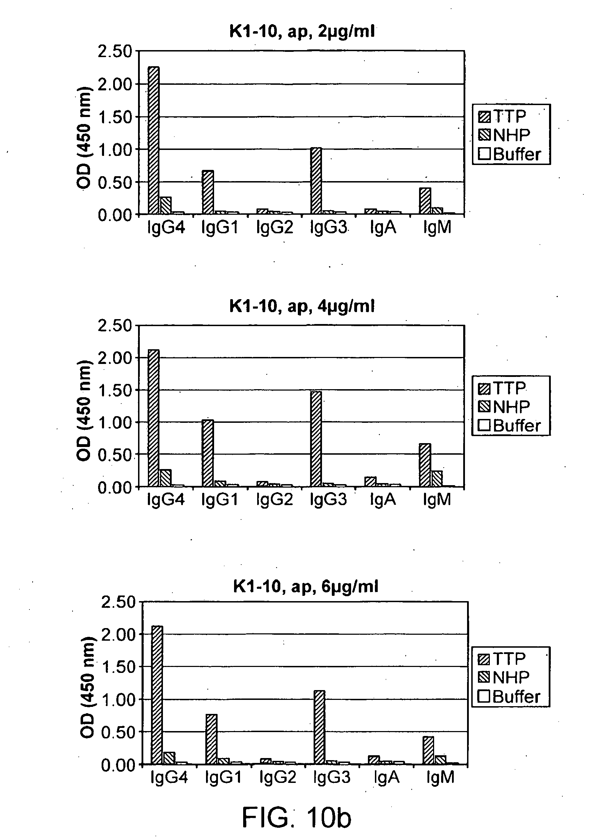

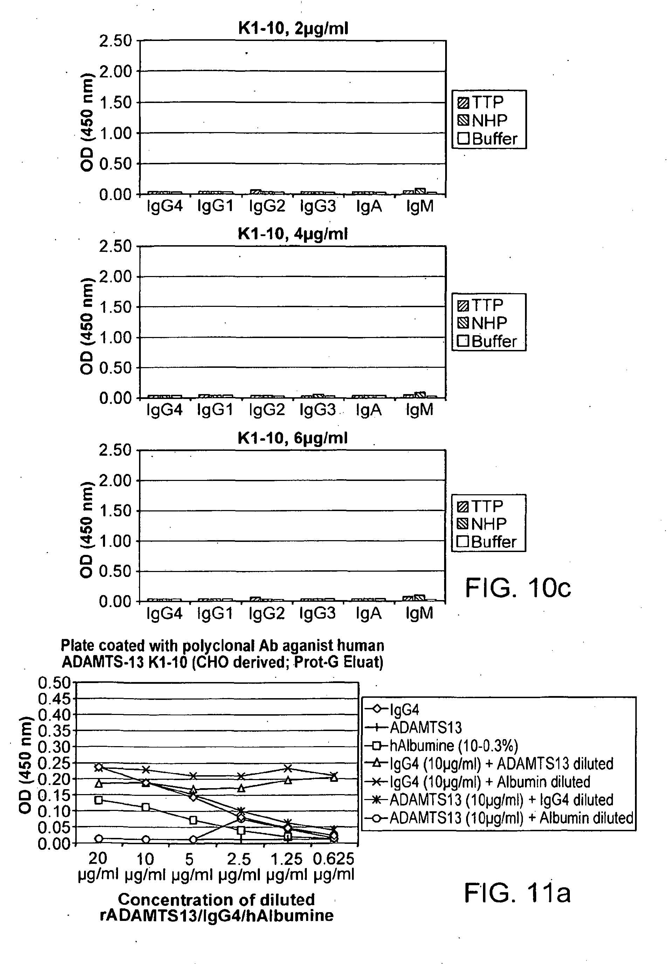

Affinity purified polyclonal anti-ADAMTS13 antibody K1-4 and non-affinity purified and affinity purified antibody K1-10 of examples 2 and 3 were coated onto Nunc Maxisorp ELISA Plates as described in example 7 with the following modifications to the procedure: Polyclonal antibody solutions were used as coating antibodies in concentrations of 2µg/ml, 4µg/ml and 6µg/ml in Coating Buffer dilutions. Dilution Buffer was used with 0.6 M NaCl. Two samples were examined: 1) TTP plasma spiked with 500 ng/100µl rADAMTS13 produced in CHO cells according to example 1 (273.5 µg/ml), 2) NHP plasma. Samples were diluted 1:25 with Dilution Buffer and incubated over night at 4°C. Anti-immunoglobulin antibodies with Ig class or subtype specificity according to example 4 were used as secondary antibodies. Secondary antibodies were diluted with Dilution Buffer (IgG4: 2 µl antibody solution in 8ml Dilution Buffer (DB), IgG1, IgG2 and IgA: 4µl antibody solution in 8ml DB, IgG3: 8µl antibody solution in 8 ml DB, IgM: 10µl of 1:100 antibody solution in 8 ml DB) and incubated for 3h at room temperature. After washing, coloring reaction was performed by incubation with TMB Solution and Peroxide Solution for 15 min at room temperature.
Results are shown in figure 10 (fig. 10a: antibody K1-4, fig. 10b: antibody K1-10, affinity purified, fig. 10c: antibody K1-10, non-affinity purified). No significant signals were measured for non-affinity purified antibody K1-10, illustrating the importance for additional purification.

Example 17: IgG4 ELISA with polyclonal anti-ADAMTS13-antibody

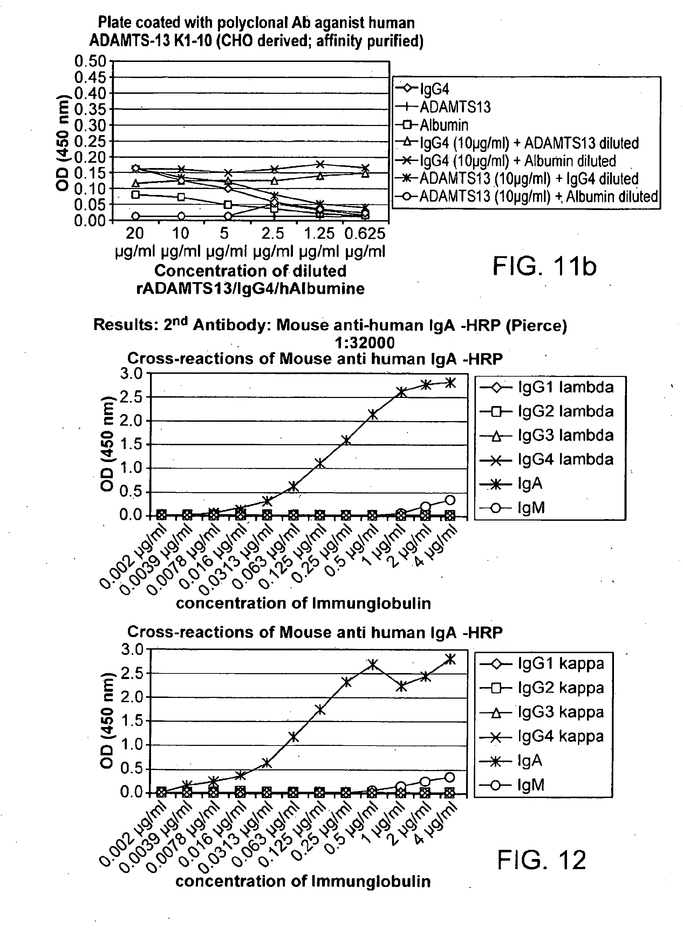

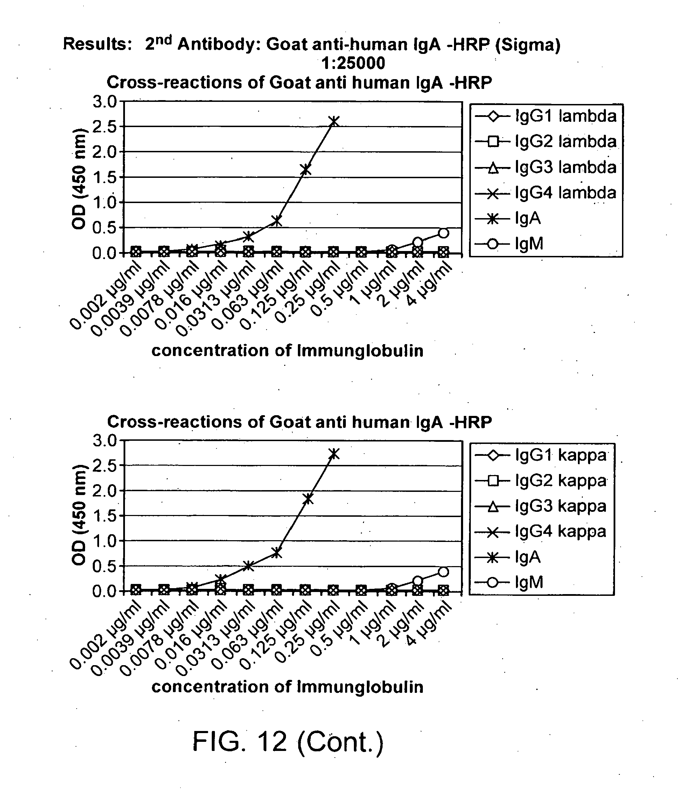

Non-affinity purified (Protein G column eluate) and affinity purified antibody K1-10 of examples 2 and 3 were coated onto Nunc Maxisorp ELISA Plates as described in example 7 with the following modifications to the procedure: Non-affinity purified polyclonal antibody solution of a concentration of 0.95 mg/ml was used as coating antibody in a dilution of 10.5 µl antibody solution with 10ml Coating Buffer. Affinity purified polyclonal antibody solution of a concentration of 0.32 mg/ml was used as coating antibody in a dilution of 33 µl antibody solution with 10ml Coating Buffer. Dilution Buffer was used with 0.6 M NaCl. Examined samples include: 1) IgG4 1:1 mixture of IgG4 kappa (Sigma, 1.2 mg/ml)) and IgG4 lambda (Sigma, 1.0 mg/ml); 2) human albumin (Sigma, 30%); ADAMTS13, produced in CHO cells according to example 1, 273,5 µg/ml (undiluted). Samples were incubated over night at 4°C. Anti-immunoglobulin antibodies with IgG4 subtype specificity (IgG4-HRP, Lymed Laboratories 1:4000) were used as secondary antibodies. 5 µl secondary antibody solution were diluted with 20 ml Dilution Buffer and incubated for 2h at room temperature. After washing, coloring reaction was performed by incubation with TMB Solution and Peroxide Solution for 15 min at room temperature.
Results are shown in figure 11 (fig. 11a: non-affinity purified antibody, fig 11b: affinity purified antibody): Affinity purification leads to a markedly reduction of non-specific binding.
Example 18: IgA ELISA with human immunoglobulin as coating antibodies
Cross-reactivities of subtype specific antibodies were tested by coating Ig of distinct classes and subtypes onto Nunc Maxisorp ELISA plates as described in example 7. No samples were used. Immediately after coating and incubation (over night at 4°C), incubation and signal generation with secondary HRP-labeled antibodies followed. The following coating antibodies were used: IgG1: human IgG1 kappa (Sigma, 1.2 mg/ml) and human IgG1 lambda (Sigma, 1.0 mg/ml); IgG2: human IgG2 kappa (Sigma, 1.1 mg/ml) and human IgG2 lambda (Sigma, 1.08 mg/ml); IgG3: human IgG3 kappa (Sigma, 1.1 mg/ml) and human IgG3 lambda (Sigma, 1.04 mg/ml); IgG4: human IgG4 kappa (Sigma, 1.0 mg/ml) and human IgG4 lambda (Sigma, 1.0 mg/ml); IgA: human IgA (Sigma, 1.0 mg/ml); and IgM: human IgM (Sigma, 1.01 mg/ml); with 1 µl Ig solution with 500µl Coating Buffer for coating during incubation over night at 4°C. Dilution Buffer contained 0.6M NaCl. Mouse anti-human IgA-HRP (Pierce 1:32000) and goat anti-human IgA (alpha chain specific)-peroxidase (Sigma, 1:25000) were used as secondary antibodies (Pierce ab: 0.63 µl in 20 ml Dilution Buffer; Sigma ab: 0.8 µl in 20 ml Dilution Buffer). Secondary antibodies were incubated for 3h at room temperature. After washing, coloring reaction was performed by incubation with TMB Solution and Peroxide Solution for 20 min at room temperature.
Results are shown in figure 12. For both secondary antibodies, cross-reactivities with IgM were observed only at higher coating antibody concentrations.

Example 19: Immunglobulin subtype specificity of IgG specific antibodies

Subtype reactivities of IgG class specific antibodies were tested by coating Ig of distinct IgG subtypes onto Nunc Maxisorp ELISA plates as described in example 7. No samples were used. Immediately after coating and incubation (over night at 4°C), incubation and signal generation with secondary HRP-labeled antibodies followed. The following coating antibodies were used: IgG1: human IgG1 kappa (Sigma, 1.2 mg/ml) and human IgG1 lambda (Sigma, 1.0 mg/ml);

IgG2: human IgG2 kappa (Sigma, 1.1 mg/ml) and human IgG2 lambda (Sigma, 1.08 mg/ml); IgG3: human IgG3 kappa (Sigma, 1.1 mg/ml) and human IgG3 lambda (Sigma, 1.04 mg/ml); IgG4: human IgG4 kappa (Sigma, 1.19 mg/ml) and human IgG4 lambda (Sigma, 1.15 mg/ml); diluted to a concentration of 4 $\mu$g/ml to 0.25 $\mu$g/ml with Coating Buffer. Bovine serum albumn (BSA) and human serum albumin (HAS) were used as negative controls. Coating antibodies were incubation over night at 4°C. Dilution Buffer contained 0.6M NaCl. Mouse anti-human IgG-HRP (SouthernBiotech) was used as secondary antibody. Secondary antibodies were diluted 1:10 and incubated for 3h at room temperature. After washing, coloring reaction was performed by incubation with TMB Solution and Peroxide Solution for 10 min at room temperature.

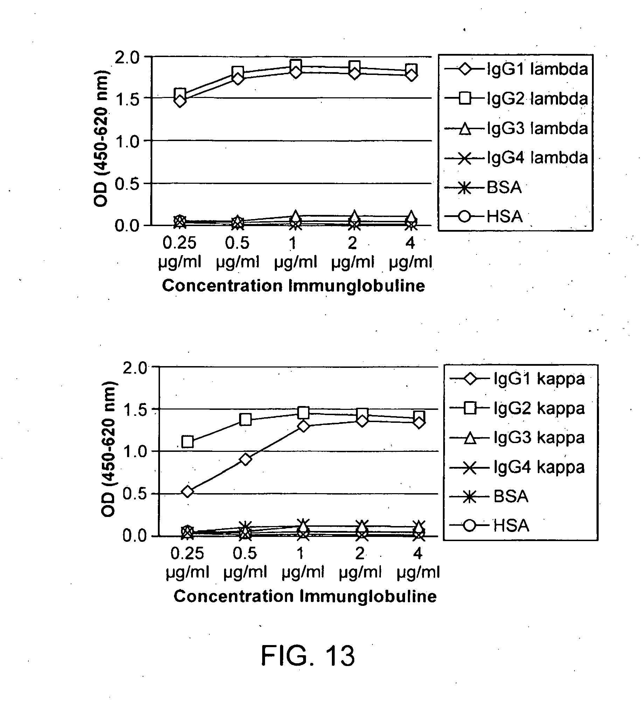

Results are shown in Fig. 13. For IgG class specific antibodies, highest reactivities were found against IgG1 and IgG2 subtypes with lower reactivity against IgG3 and IgG4 subclasses. At high concentration of coating antibodies (4 $\mu$g/ml), reactivities were similar for kappa and lambda chain specificity.

Example 20: ADAMTS13 immune complex ELISA with anti-human IgG-HRP secondary antibodies (not-subtype specific)

Polyclonal anti-ADAMTS13 antibody K1-10 was obtained and purified as described in example 3 and coated onto Nunc Maxisorp ELISA Plates as described in example 7 with the following modifications to the procedure: the initial antibody solution with a concentration of 3.135 mg/ml was diluted with 20 ml Coating Buffer to concentrations of 2 $\mu$g/ml. Incubation was for 5 hours at room temperature. Dilution Buffer was used with 0.6 M NaCl. As positive sample, TTP, plasma, TTP plasma spiked with 300ng/$\mu$l rADAMTS13 produced in CHO cells according to example 1 (initial concentration 273.5 $\mu$g/ml) were used and as negative sample, NHP plasma or Buffer were used. Samples were diluted 1:25 and 1:50 with Dilution Buffer and incubated over night at 4°C. As secondary detection antibody, three different mouse anti-human IgG-HRP (Zymed Laboratories, Jackson ImmunoResearch, , SouthernBiotech,) were tested dilutions with 3 ml Dilution Buffer: Zymed and Jackson I. antibodies were diluted 1:1000 (1$^{st}$ dilution) or 1:5000 (2$^{nd}$ dilution), SouthernB. antibody was diluted 1:1000 (1$^{st}$ dilution) and 1:2000 (2$^{nd}$ dilution). Samples were incubated for 3h at room temperature. After washing, coloring reaction was performed by incubation with TMB Solution and Peroxide Solution for 20 min at room temperature.

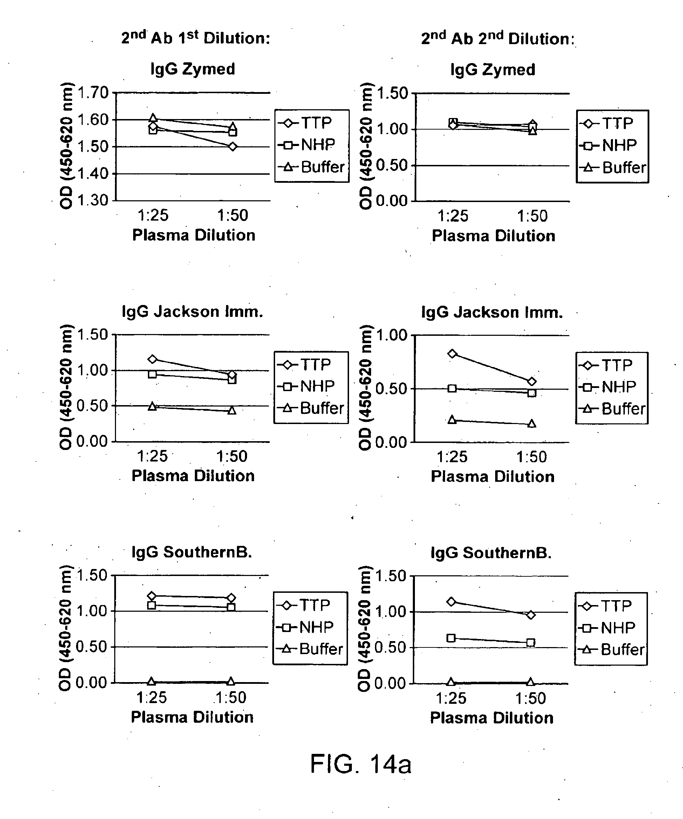

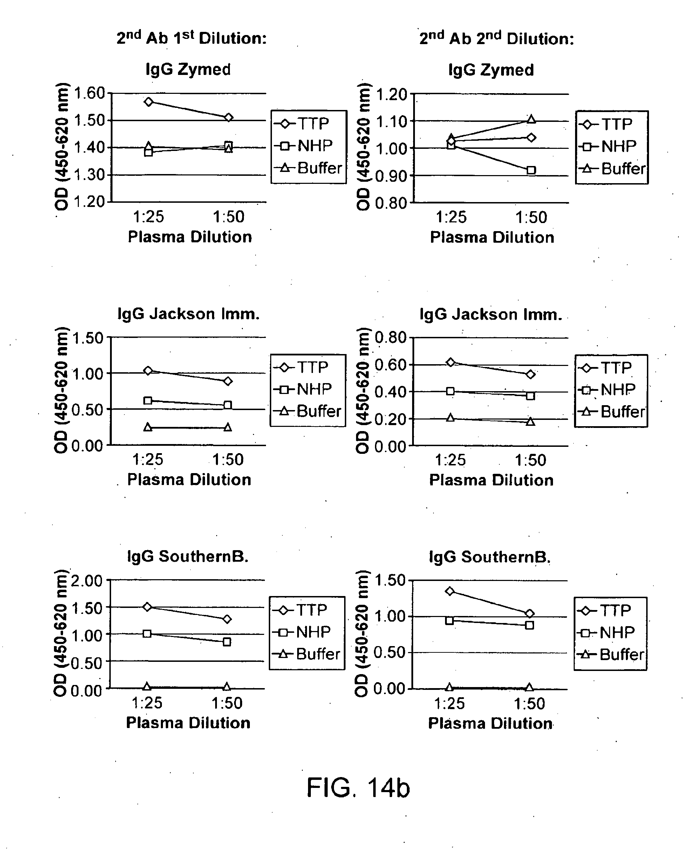

Results are shown in fig. 14 (fig. 14a: TTP plasma spiked with rADAMTS13; fig. 14b: TTP plasma). Although, TTP plasma could be distinguished from NHP plasma for all antibodies, all IgG class specific antibodies suffer from high background signals for ADAMTS13-negative NHP samples.

Example 21: Immunglobulin subtype specificity of mouse anti-human IgG specific antibody

Subtype reactivities of IgG class specific (but not subtype specific) mouse antibody was tested by coating Ig of distinct IgG subtypes onto Nunc Maxisorp ELISA plates as described in example 7. No samples were used. Immediately after coating and incubation (over night at 4°C), incubation and signal generation with secondary HRP-labeled antibody followed. The following coating antibodies were used: IgG1-IgG4 as in example 19; IgA: human IgA (Sigma I1010, lot: 057K6070, 5mg lyophilisate diluted in 5 ml PBS); IgM: human IgM (Sigma, 1.01 mg/ml); diluted to a concentration of 4 $\mu$g/ml to 0.0039 $\mu$g/ml with Coating Buffer. Bovine serum albumn (BSA) and human serum albumin (HAS) were used as negative controls. Coating antibodies were incubation over night at 4°C. Dilution Buffer contained 0.6 M NaCl. Mouse anti-human IgG-HRP (SouthernBiotech) was used as secondary antibody. Secondary antibodies were diluted 1:10 and incubated for 3h at room temperature. After washing, coloring reaction was performed by incubation with TMB Solution and Peroxide Solution for 10 min at room temperature.

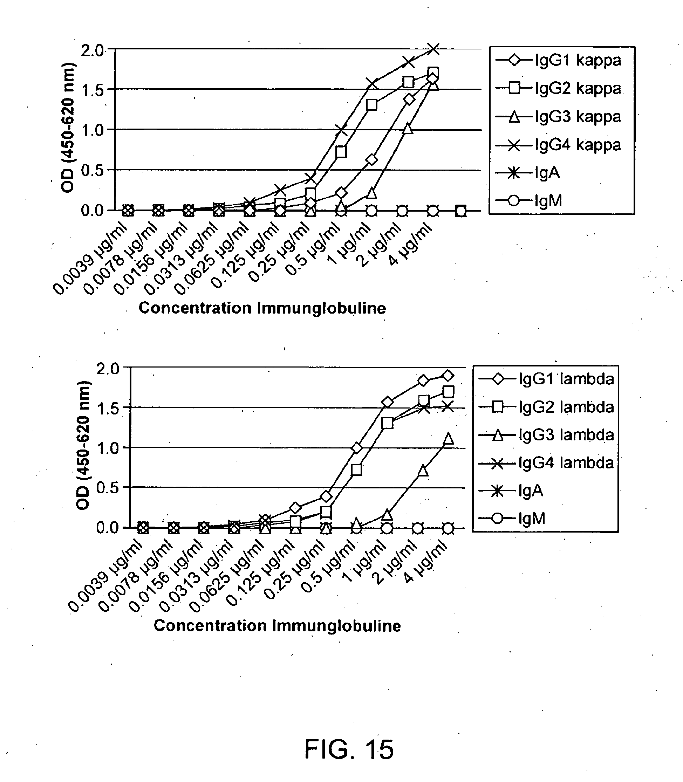

Results are shown in Fig. 15. For IgG class specific antibodies, at high concentrations (4 $\mu$g/ml) reactivities were found against all IgG1-IgG4 subtypes.

Example 22: ADAMTS13 immune complex ELISA with anti-human IgG-HRP secondary antibodies (not-subtype specific)

Polyclonal anti-ADAMTS13 antibody K1-10 was obtained and purified as described in example 3 and coated onto Nunc Maxisorp ELISA Plates as described in example 7 with the following modifications to the procedure: the initial antibody solution with a concentration of 3.135 mg/ml was diluted with 10 ml Coating Buffer to concentrations of 1 or 2 $\mu$g/ml. Incubation was for 6 hours at room temperature. Dilution Buffer was used with 0.6 M NaCl. As positive samples, TTP plasma and TTP plasma spiked with 300ng/$\mu$l rADAMTS13 produced in CHO cells according to example 1 were used and as negative sample, NHP plasma or Buffer were used. Samples were diluted 1:25 and 1:50 with Dilution Buffer and incubated over night at 4°C. As secondary detection antibody, mouse anti-human IgG-HRP (SouthernBiotech,) was used in 1:16000, 1:32000 and 1:64000 dilutions with 10 ml Dilution Buffer. Antibodies were incubated for 3h at room temperature. After washing, coloring reaction was performed by incubation with TMB Solution and Peroxide Solution for 20 min at room temperature.

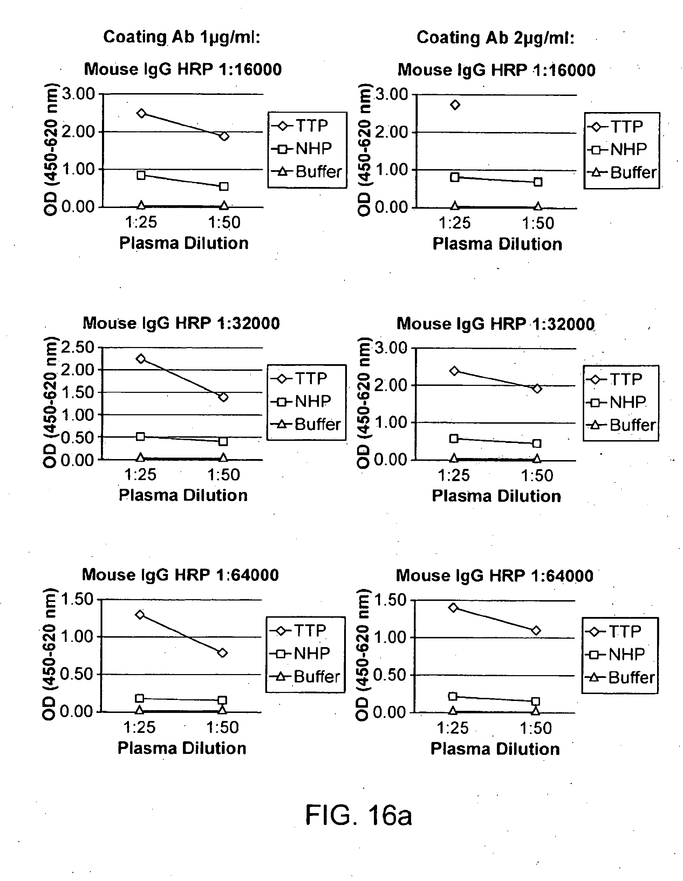

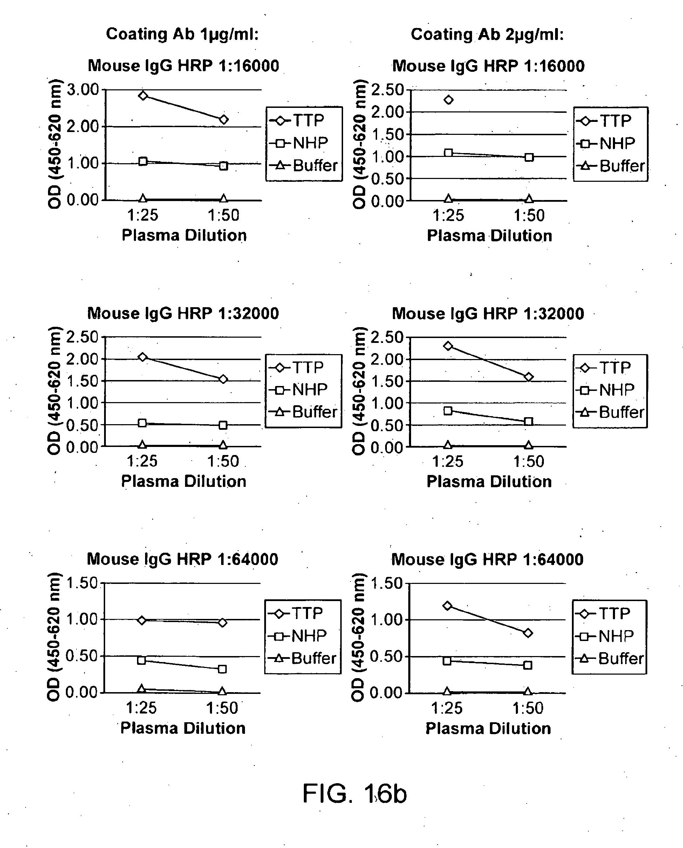

Results are shown in fig. 16 (fig. 16a: TTP plasma spiked with rADAMTS13; fig. 16b: TTP plasma). Although, TTP plasma could be distinguished from NHP plasma for all antibody dilutiens, IgG class specific antibodies suffer from background signals for ADAMTS13-negative NHP samples.

Example 23: Ionic conditions

The effect of salt concentration and ionic strength was examined in an ADAMTS13 immune complex ELISA. Polyclonal anti-ADAMTS13 antibody K1-10 was obtained and purified as described in example 3 and coated onto Nunc Maxisorp ELISA Plates as described in example 7 with the following modifications to the procedure: the initial antibody solution with a concentration of 3.135 mg/ml was diluted with 10 ml Coating Buffer to concentrations of 1 $\mu$g/ml (for IgG4 testing) or 2 $\mu$g/ml (for IgGl-3, IgA and IgM testing). Incubation was for 6 hours at room temperature.

In the Dilution Buffer, NaCl was added in concentrations of 0.25 M, 0.5 M and 1 M NaCl, together with PBS-T buffer resulting in concentrations of 0.1 M, 0.4 M, 0.6 M and 1.1 M NaCl.

As positive samples, TTP plasma (for IgG4 testing) and TTP plasma spiked with 300ng/$\mu$l rADAMTS13 produced in CHO cells according to example 1 (for IgGl-3, IgA and IgM testing) were used and as negative sample, NHP plasma or Buffer were used. Samples were diluted with Dilution Buffer comprising additional NaCl at the given concentrations (TTP: 104 $\mu$l serum + 2496$\mu$l of the Dilution Buffers, TTP+rA13: 24 $\mu$l serum + 596$\mu$l of the Dilution Buffers, NHP: 120 $\mu$l serum + 2880$\mu$l of the Dilution Buffers) and incubated over night at 4°C. As secondary detection antibody, subtype specific antibodies according to example 4 were used in dilutions of 1:1000 (anti-IgG3), 1:2000 (anti-IgGl, anti-IgG2), 1:4000 (anti-IgG4), 1:25000 (anti-IgA) or 1:80000 (anti-IgM). Secondary antibodies were either diluted with Dilution Buffer without NaCl addition or with Dilution Buffer with the indicated NaCl additions. Antibodies solutions were incubated for 3h at room temperature. After washing, coloring reaction was performed by incubation with TMB Solution and Peroxide Solution for 15 min at room temperature.

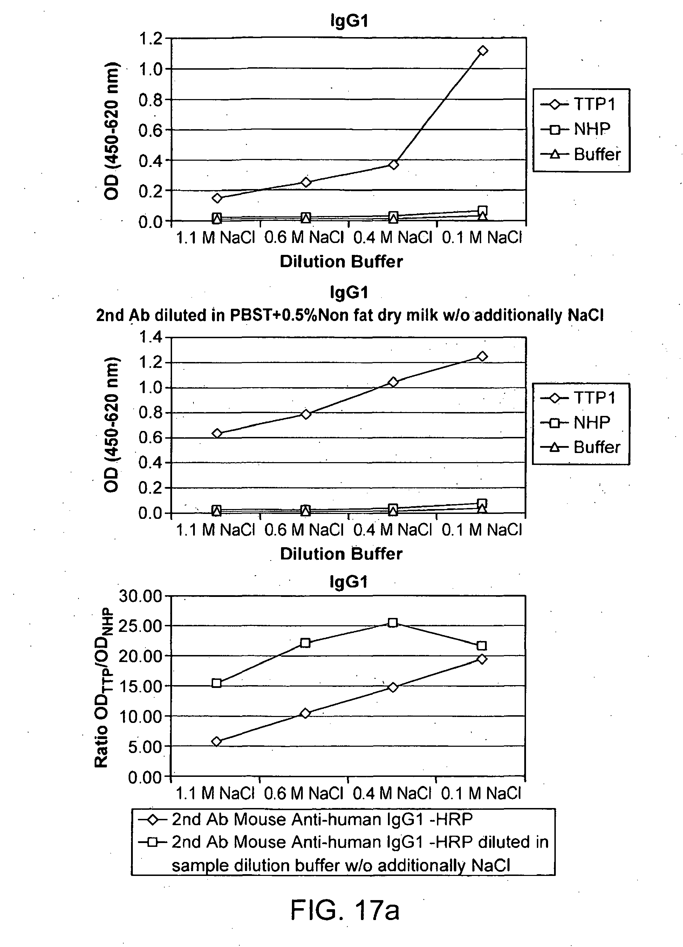

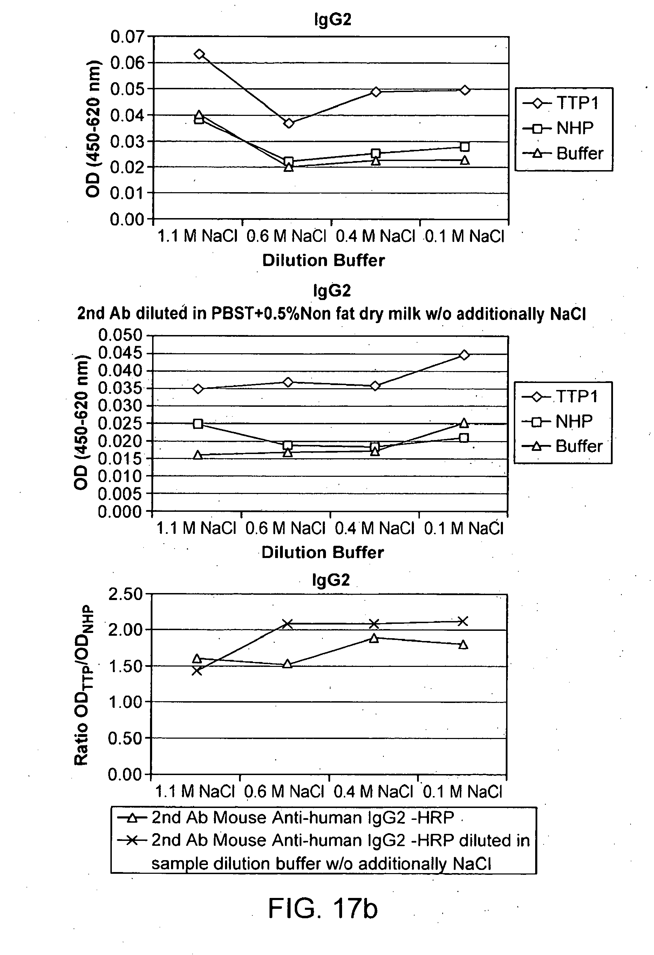

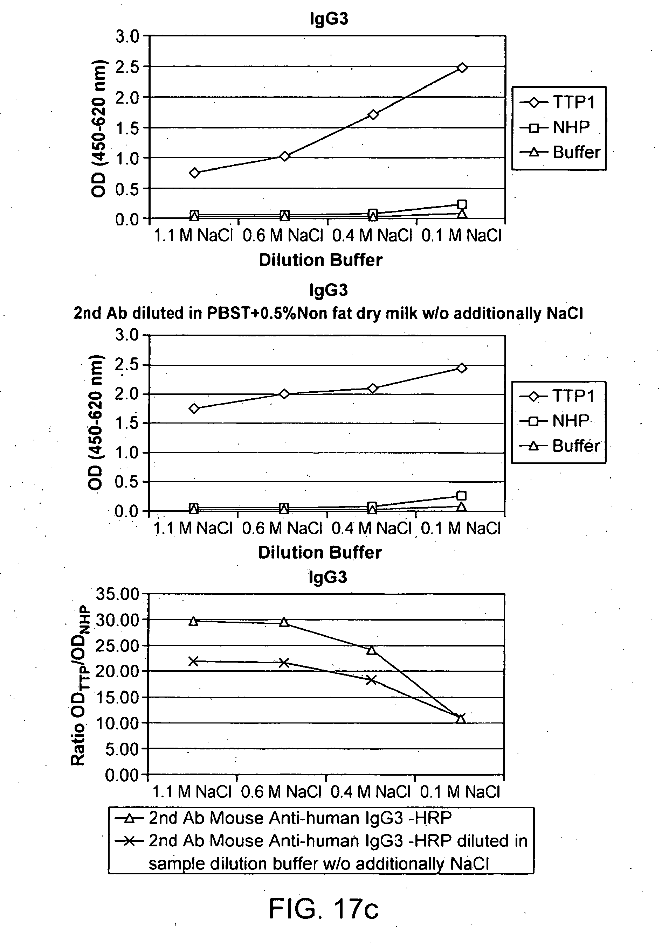

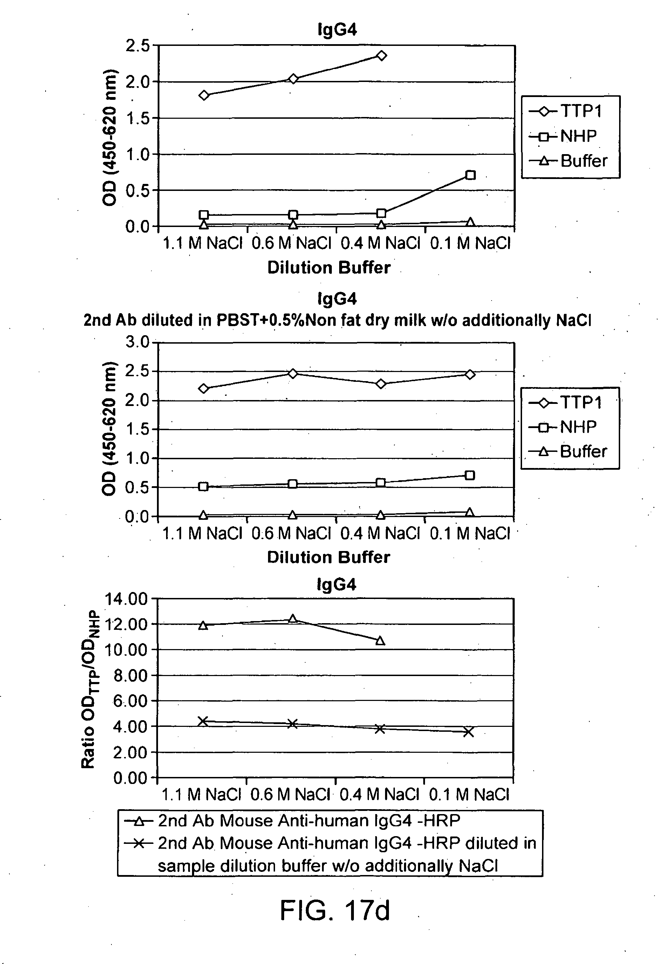

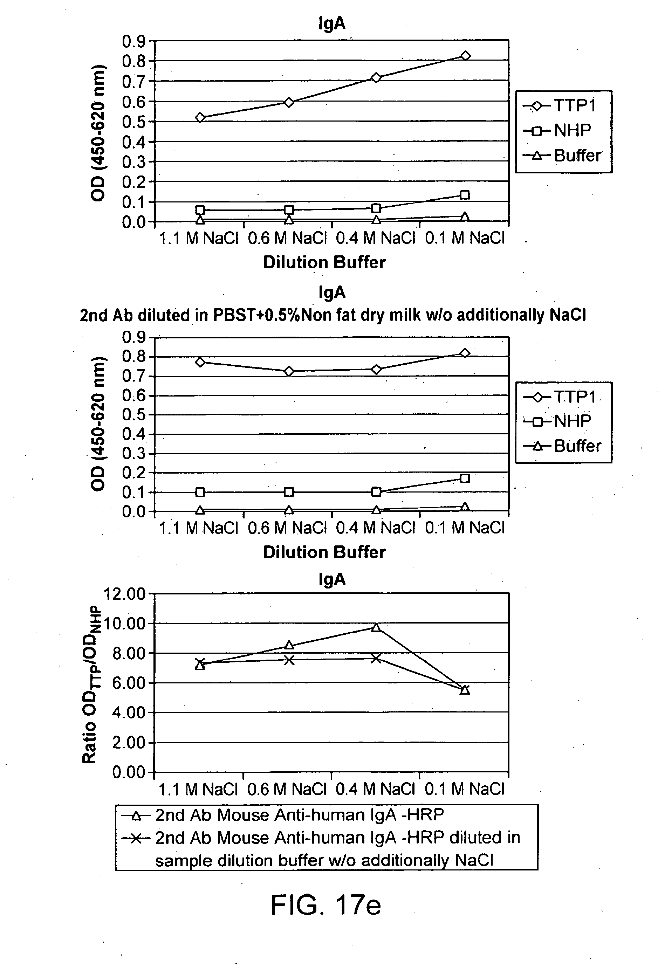

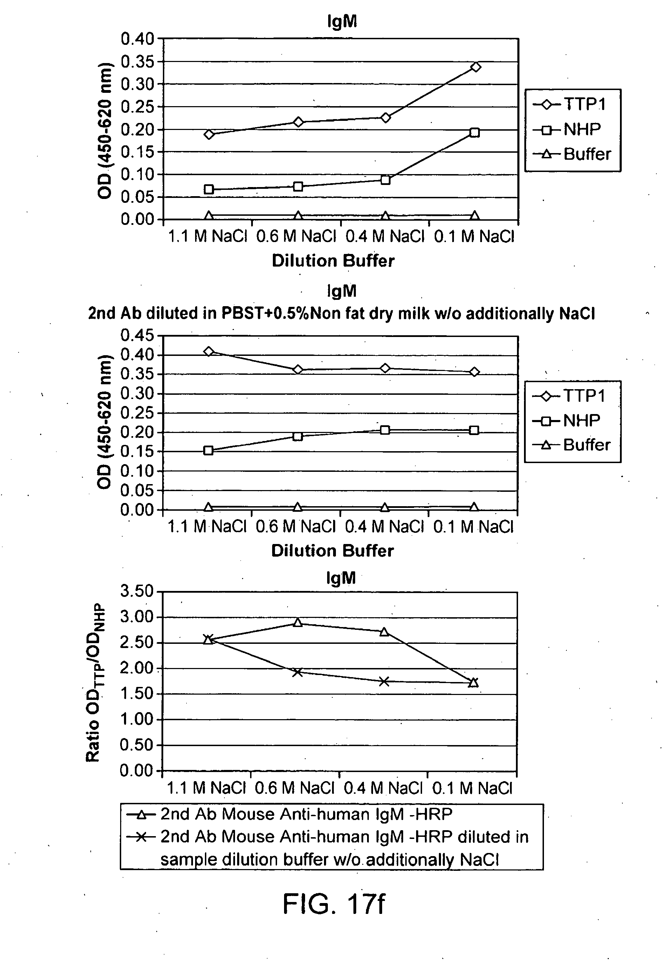

Results are shown in fig. 17 (fig. 17a: anti-IgG1, fig. 17b: anti-IgG2, fig. 17c: anti-IgG3, fig. 17d: anti-IgG4, fig. 17e: anti-IgA, fig. 17f: anti-IgM secondary antibodies). Higher salt concentrations are favourable for most secondary antibodies: Although higher signals can be observed for low salt conditions, the signal to noise ratio was generally better for high salt conditions. Very good signal (TTP)-to-noise (NHP) ratios were observed for IgG3 and IgG4. The beneficial effect of high salt is observed for IgA and IgM but is not that pronounced as seen for IgG4 and IgG3. For IgG1, without additional salt results are better, however, the ratio with high salt is still in an acceptable range.

Example 24: ADAMTS13 immune complex ELISA: testing of individual TTP samples

TTP plasma samples of individual patient according to example 5 were tested in an ADAMTS13 immune complex assay. Polyclonal anti-ADAMTS13 antibody K1-10 was obtained and purified as described in example 3 and coated onto Nunc Maxisorp ELISA Plates as described in example 7 with the following modifications to the procedure: the initial antibody solution with a concentration of 3.135 mg/ml was diluted to 2 $\mu$g/ml with 80 ml Coating Buffer or to 1 $\mu$g/ml with 20 ml Coating Buffer; for IgA and IgM testing, coating antibody was diluted to 2 $\mu$g/ml in 160 ml Coating Buffer. Incubation was for 6 hours (for IgG testing) or 5 hours (for IgA and IgM testing) at room temperature. Dilution Buffer was used with 0.6 M NaCl. Positive control TTP pooled plasma (as described in example 23, negative control NHP pooled plasma (reference plasma) and individual TTP patient plasma samples were diluted with Dilution Buffer 1:25 (72 $\mu$l plasma in 1800 $\mu$l Dilution Buffer or 36 $\mu$l plasma in 864 $\mu$l Dilution Buffer) and stepwise further diluted to 1:400 for additional testing. Plasma samples were incubated over night at 4°C. As secondary detection antibody, IgG1-4 subtype specific antibodies were used according to example 4 (mouse anti-human IgG1-HRP, Zymed, used in 1:2000 dilution; mouse anti-human IgG2-HRP, SouthernBioTech, used in 1:2000 dilution; mouse anti-human IgG3-HRP, Zymed, used in 1:1000 dilution; mouse anti-human IgG4-HRP, Zymed, used in 1:4000 dilution; goat anti-human IgA-HRP, Sigma, used in 1:25000 dilution; IgM goat anti-human IgM ($\mu$ chain specific)-HRP, Sigma, used in 1:80000 dilution). Secondary antibodies were incubated for 3h at room temperature. After washing, coloring reaction was performed by incubation with TMB Solution and Peroxide Solution for 20 min at room temperature.

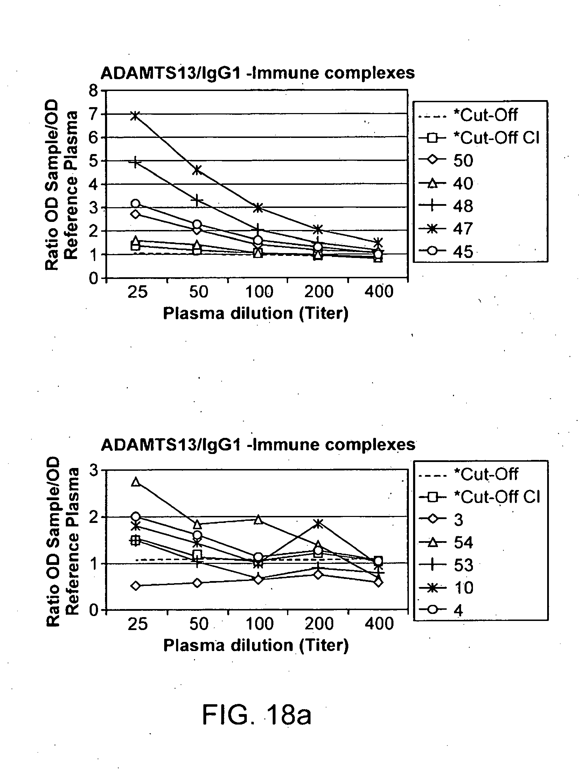

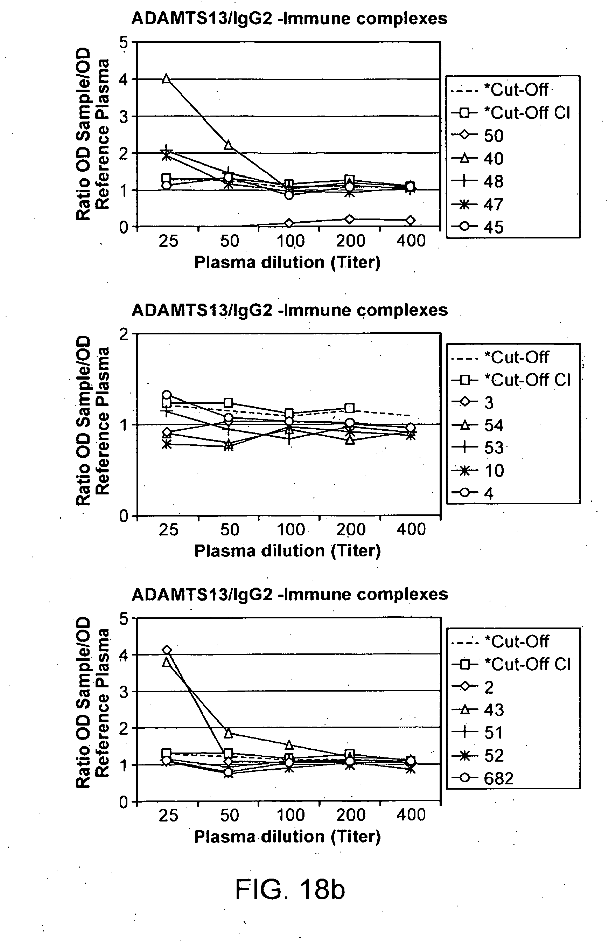

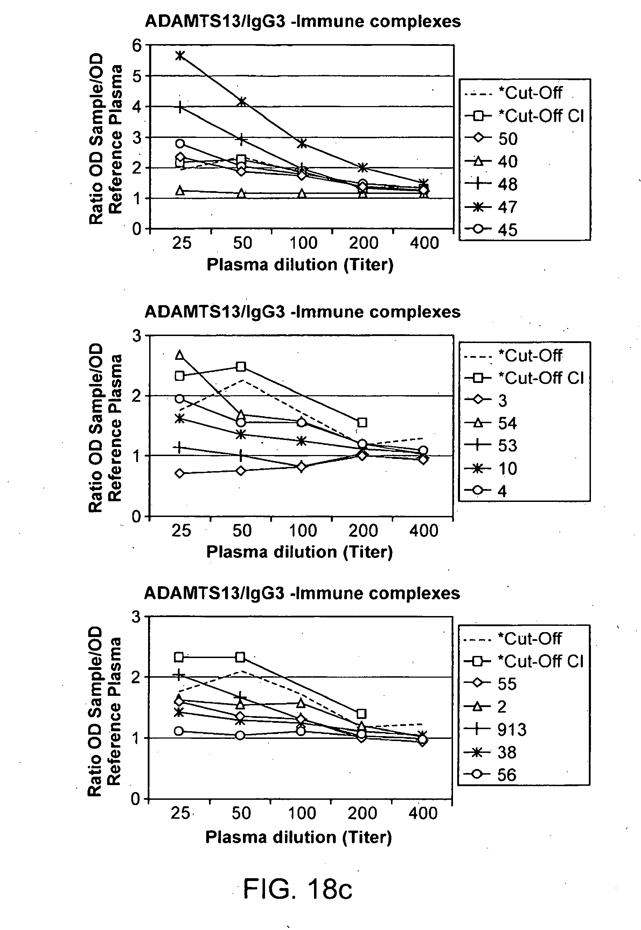

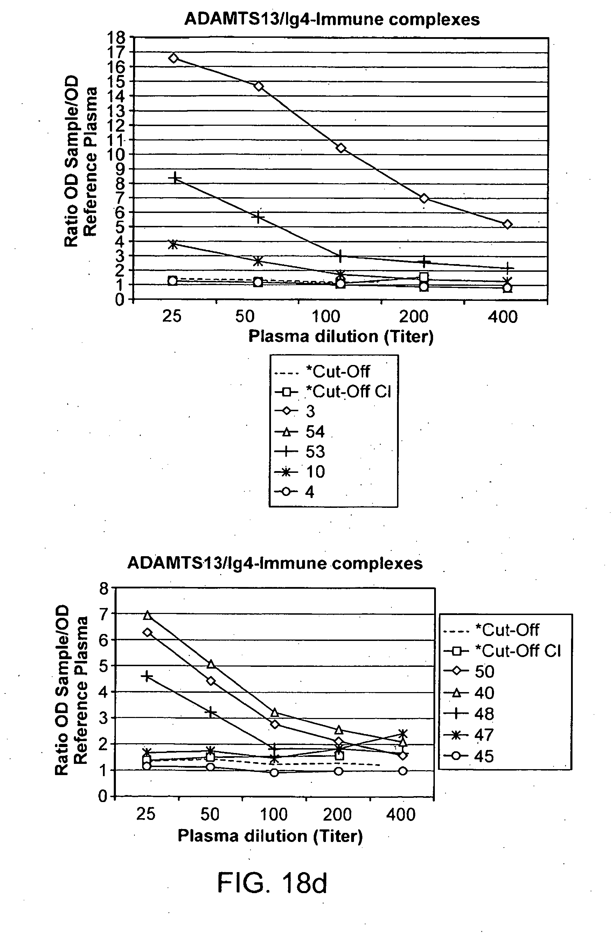

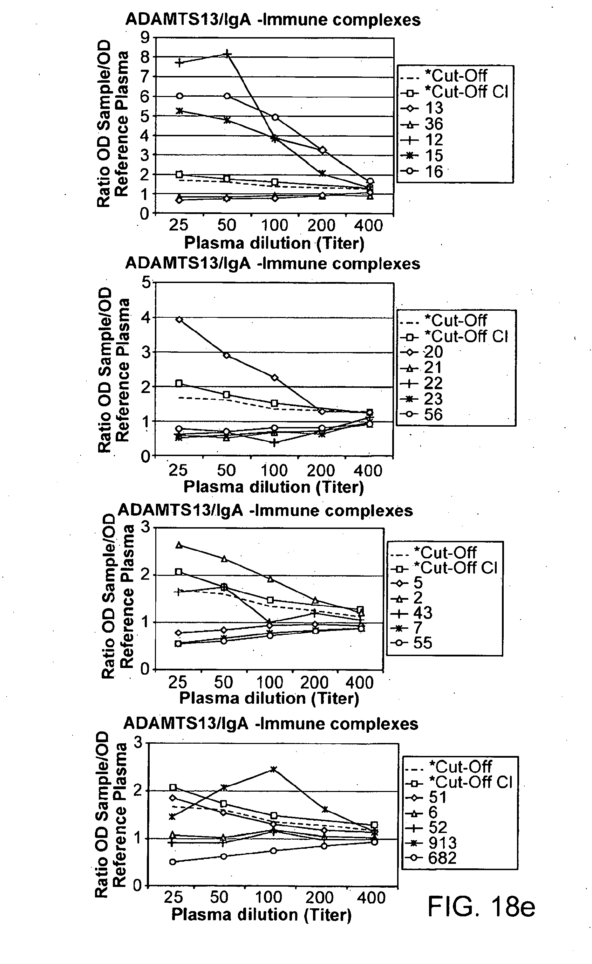

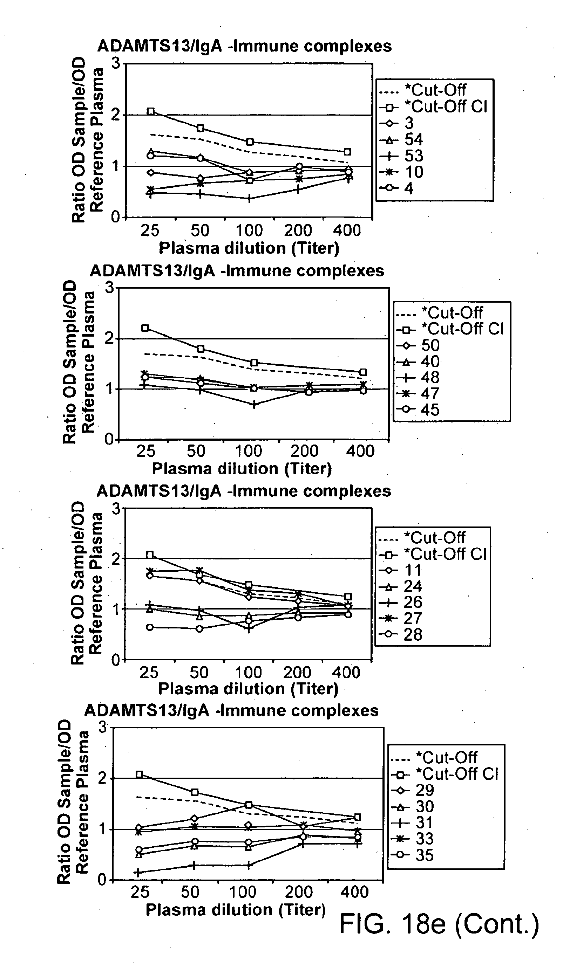

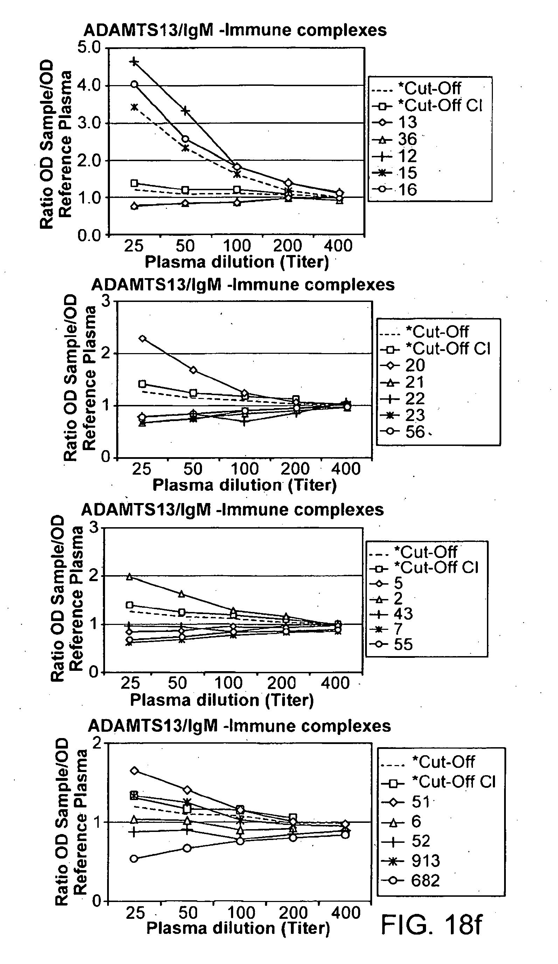

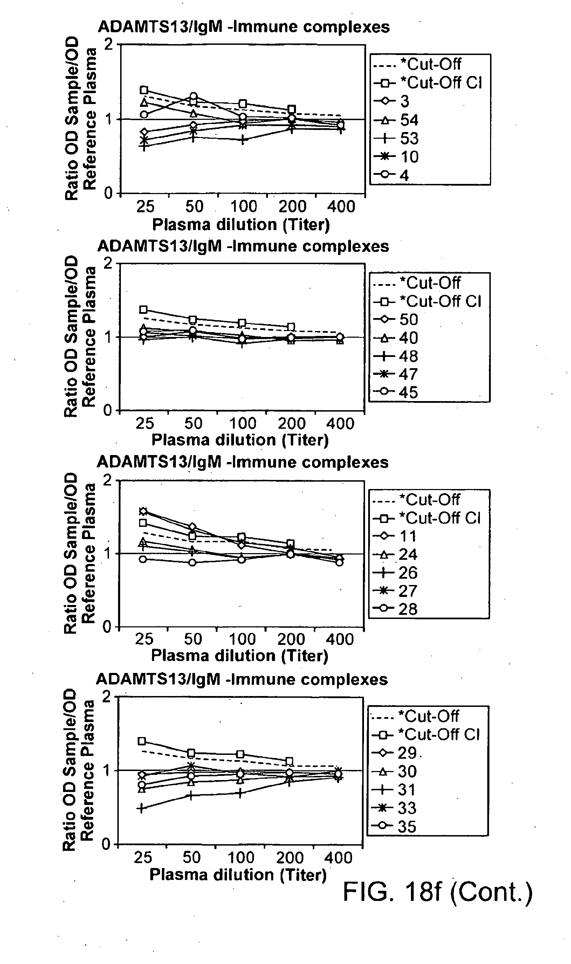

Selected patient samples are shown in figure 18 (fig. 18a: anti-IgG1, fig. 18b: anti-IgG2, fig. 18c: anti-IgG3, fig. 18d: anti-IgG4, fig. 18e: anti-IgA, fig. 18f: anti-IgM). Positive control results were usually above the depicted signal ranges: For IgG1, OD sample/OD reference ratios were for 1:25 dilution at 15.0, for 1:50 dilution at 12.1, for 1:100 dilution at 8.4, for 1:200 dilution at 4.1 and for 1:400 dilution at 2.7; for IgG2, OD sample/OD reference ratios were for 1:25 dilution at 3.40, for 1:50 dilution at 1.48, for 1:100 dilution at 0.84, for 1:200 dilution at 1.23 and for 1:400 dilution at 1.19; for IgG3, OD sample/OD reference ratios were for 1:25 dilution at 15.6, for 1:50 dilution at 15.3, for 1:100 dilution at 11.6, for 1:200 dilution at 8.0 and for 1:400 dilution at 4.4; for IgG4, OD sample/OD reference ratios were for 1:25 dilution at 17.3, for 1:50 dilution at 14.9, for 1:100 dilution at 10.1, for 1:200 dilution at 7.6 and for 1:400 dilution at 5.4; for IgA, OD sample/OD reference ratios were for 1:25 dilution at 8.4, for 1:50 dilution at 10.8, for 1:100 dilution at 10.3, for 1:200 dilution at 7.9 and for 1:400 dilution at 4.4; for IgM, OD sample/OD reference ratios were for 1:25 dilution at 5.1, for 1:50 dilution at 4.6, for 1:100 dilution at 3.6, for 1:200 dilution at 2.3 and for 1:400 dilution at 1.6. Cut-off points were statistically determined and lie in the range of 1.0 - 1.4 OD sample/OD reference ratio. Detectable ADAMTS13 IgG1 immune complexes of confirmed acquired TTP patients could be detected for all patients with the new ADAMTS13, assay. One patient's sample, patient 40, was only slightly above the cut-off value. It is likely that with less sensitive methods this patient might have been misdiagnosed. Surprisingly this patient showed higher IgG2 titres. IgG2 signals are normally very close to the cut-off value. Positive and negative patient sera testing is summarised in table 2.

Table 2: negative and positive TTP patient serum tests with highest positive dilution are given:

| Sample | IgG1 titer | IgG2 titer | IgG3 titer | IgG4 titer |
|---|---|---|---|---|
| 3 | n.d. | n.d. | n.d. | >400 |
| 54 | 200 | n.d. | 25 | n.d. |
| 53 | n.d. | n.d. | n.d. | >400 |
| 10 | 50 | n.d. | n.d | 100 |
| 4 | 200 | 25 | 25 | 25 |
| 50 | >400 | n.d. | 25 | >400 |
| 40 | 50 | 50 | n.d. | >400 |
| 48 | >400 | 100 | 200 | >400 |
| 47 | >400 | 25 | >400 | >400 |
| 45 | 200 | n.d. | 25 | n.d. |

(n.d.: not detected; all of these selected patients tested negative for anti-ADAMTS13 IgA and IgM titers, cf. figs. 18e and f)

Example 25: ionic conditions, IgG1 subtype secondary antibody

The effect of salt concentration and ionic strength was tested in an ADAMTS13 immune complex ELISA. Rabbit polyclonal anti-human ADAMTS13 Antibody K1-10 was diluted to a concentration of $2\mu g/ml$ with Coating Buffer (0.05M Carbonate - Bicarbonate Buffer pH 9,6, 25°C) and coated onto the surface of ELISA plates (Maxisorp; Nunc, Rochester, NY). After washing 4 times with 250 $\mu l$/well Wash Buffer (PBS-T:PBS with 0.01% Tween 20), free binding sites were blocked for 2 hours at room temperature with 200 $\mu l$/well Blocking Buffer (PBS-T:PBS with 0.01% Tween 20 with 0.5% Non Fat Dry Milk).

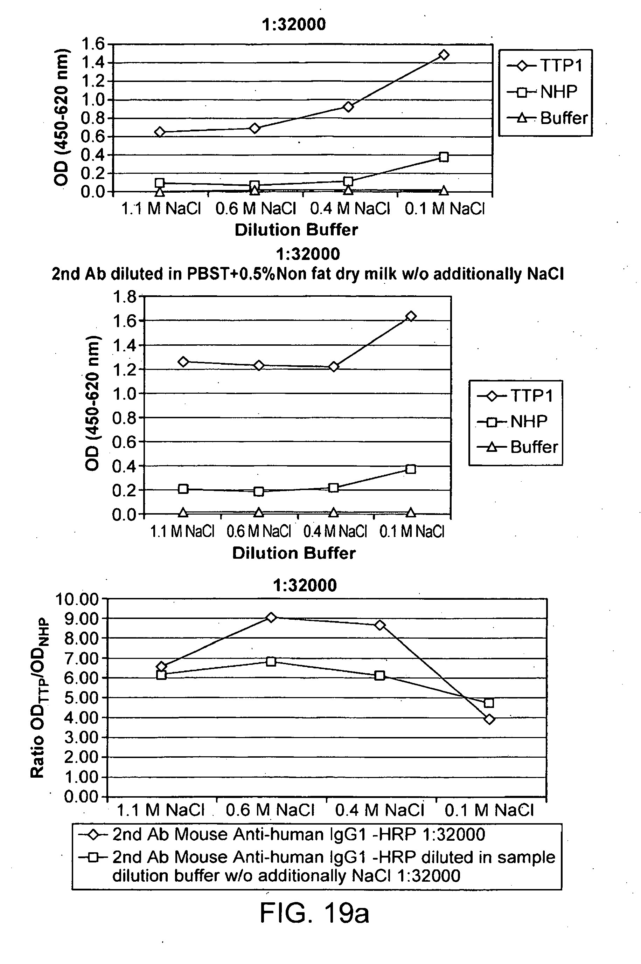

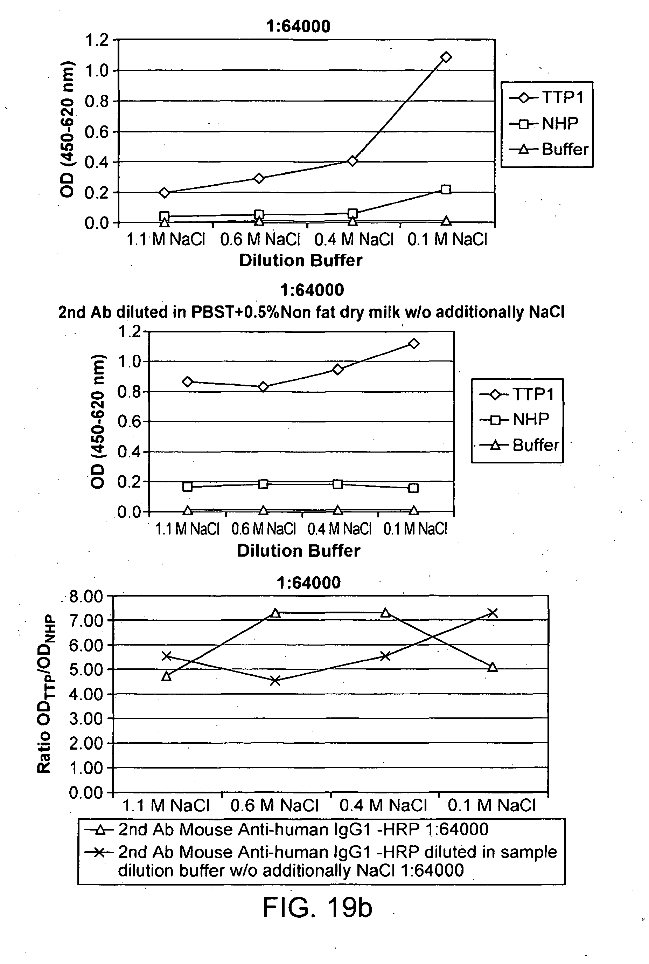

TTP plasma spiked with rADAMTS13 or NHP plasma samples were diluted in Dilution Buffer (PBS-T with 0.5 % Non Fat Dry Milk, optionally is 1.1 M, 0.6 M, 0.4 M, or 0.1 M NaCl) to 1:25. Diluted samples were added ($100\mu l$/well) and incubated overnight at 4°C. Wells were washed 4 times with 250 $\mu l$/well Wash Buffer. Anti-IgG1 antibody (mouse anti-human IgG1-HRP, Fitzgerald Cat. No.: 61R-I110aHRP) were added as a secondary antibody (diluted in Dilution Buffer 1:32000 and 1:64000) at 100 $\mu l$/well and incubated for 3h at room temperature. The wells were washed 4 times with 250 $\mu l$/well Washing buffer. Equal volumes of each of TMB Solution and Peroxide solution (Immuno Pure TMB Substrate, Pierce) were added in a total substrate volume of $100\mu l$/well. Reactions were stopped by addition of $100\mu l$ 2M $H_2SO_4$. Color reaction was measured at 450 nm versus 620 nm on a Tecan ELISA-Reader. Results are calculated as ratio between Sample OD and Control OD. The results are shown in Figure 19.

Example 26: ionic conditions, IgG4 subtype secondary antibody

The effect of salt concentration and ionic strength was tested in an ADAMTS13 immune complex ELISA. Rabbit polyclonal anti-human ADAMTS13 Antibody K1-10 was diluted to a concentration of $2\mu g/ml$ with Coating Buffer (0.05M Carbonate - Bicarbonate Buffer pH 9,6, 25°C) and coated onto the surface of ELISA plates (Maxisorp; Nunc, Rochester, NY). After washing 4 times with 250 $\mu l$/well Wash Buffer (PBS-T:PBS with 0.01% Tween 20), free binding sites were blocked for 2 hours at room temperature with 200 $\mu l$/well Blocking Buffer (PBS-T:PBS with 0.01% Tween 20 with 0.5% Non Fat Dry Milk).

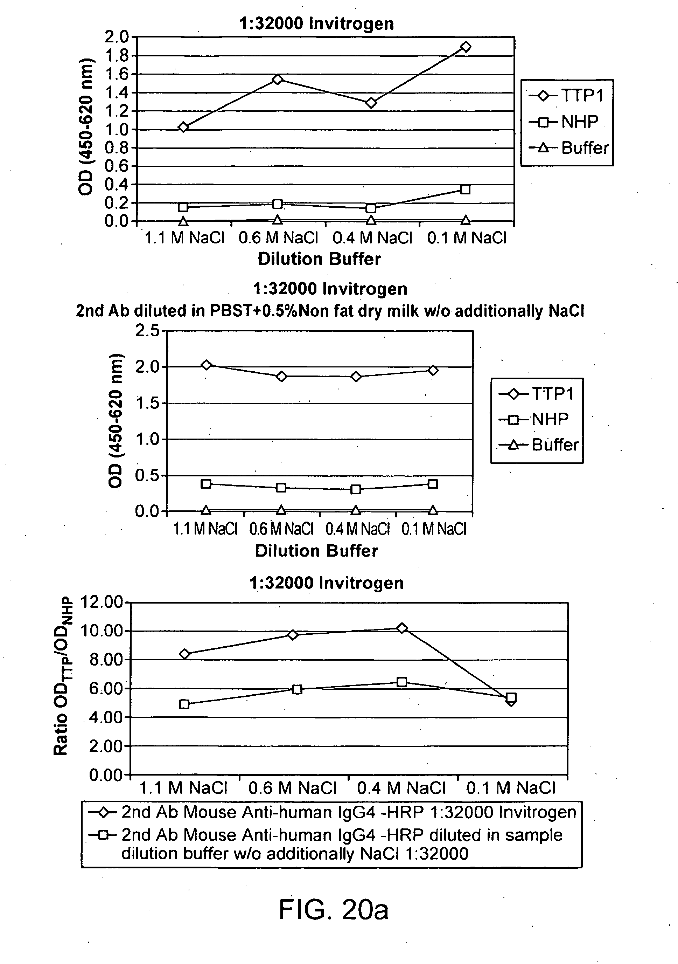

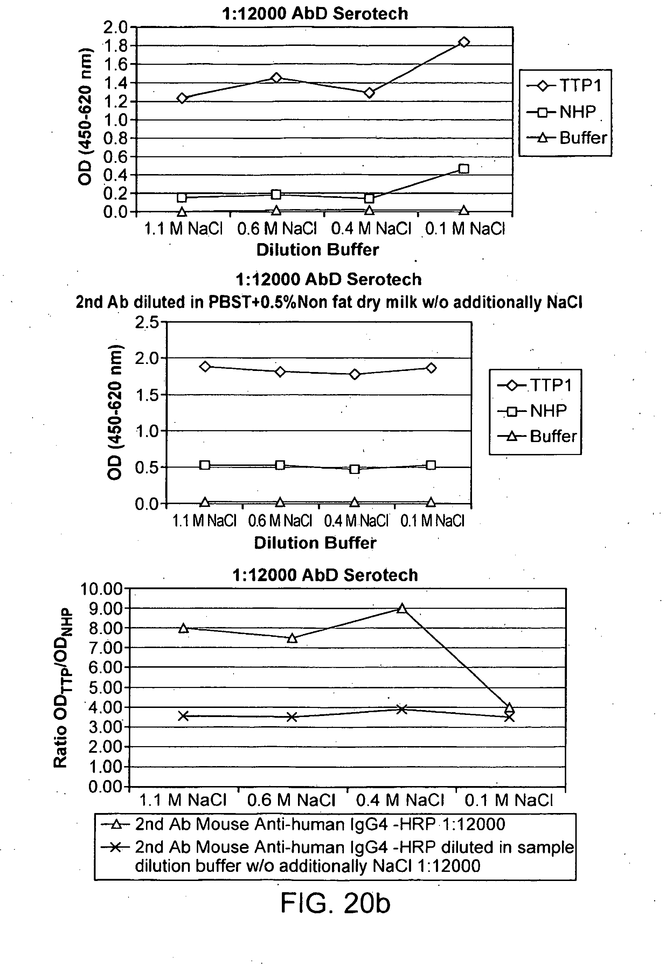

Samples were diluted in Dilution Buffer (PBS-T with 0.5 % Non Fat Dry Milk, optionally is 1.1 M, 0.6 M, 0.4 M, or 0.1 M NaCl) to 1:25. Diluted samples were added ($100\mu l$/well) and incubated overnight at 4°C. Wells were washed 4 times with 250 $\mu l$/well Wash Buffer. Anti-IgG4 antibody (mouse anti-human IgG4-HRP antibody (Invitrogen, Cat. No.: A10654 or AbD Serotec, Cat. No. MCA20980) were added as a secondary antibody (diluted in Dilution Buffer 1:32000, Invitrogen antibody and 1:2000 AbD Serotec antibody) at 100 $\mu l$/well and incubated for 3h at room temperature. The wells were washed 4 times with 250 $\mu l$/well Washing buffer. Equal volumes of each of TMB Solution and Peroxide solution (Immuno Pure TMB Substrate, Pierce) were added in a total substrate volume of $100\mu l$/well. Reaction was stopped by addition of $100\mu l$ 2M $H_2SO_4$. Color reaction was measured at 450 nm versus 620 nm on a Tecan ELISA-Reader. Results are calculated as ratio between Sample OD and Control OD. The results are shown in Figure 20.

Example 27: IgG ELISA Cross-reactivity cross-reactivity of secondary antibodies

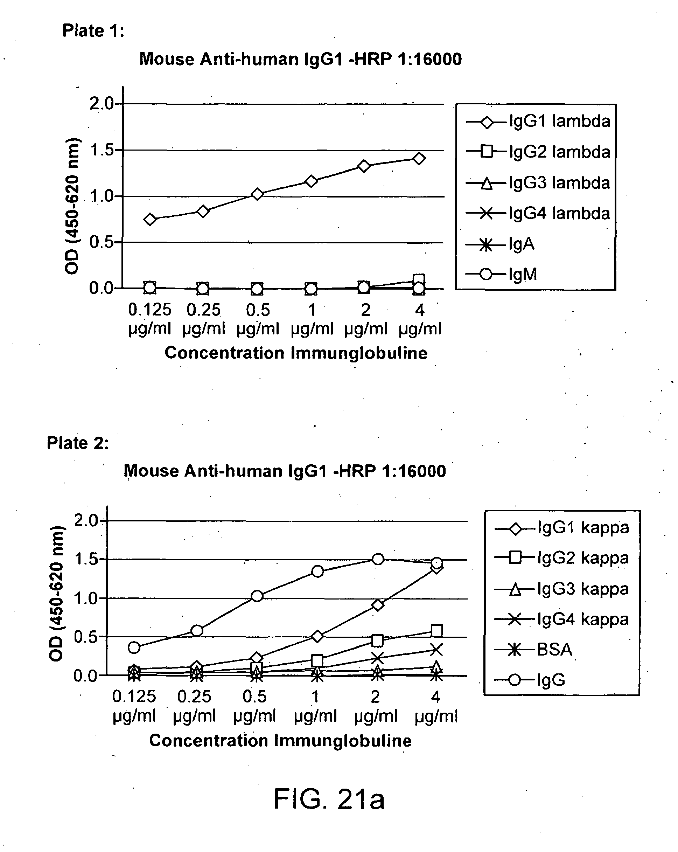

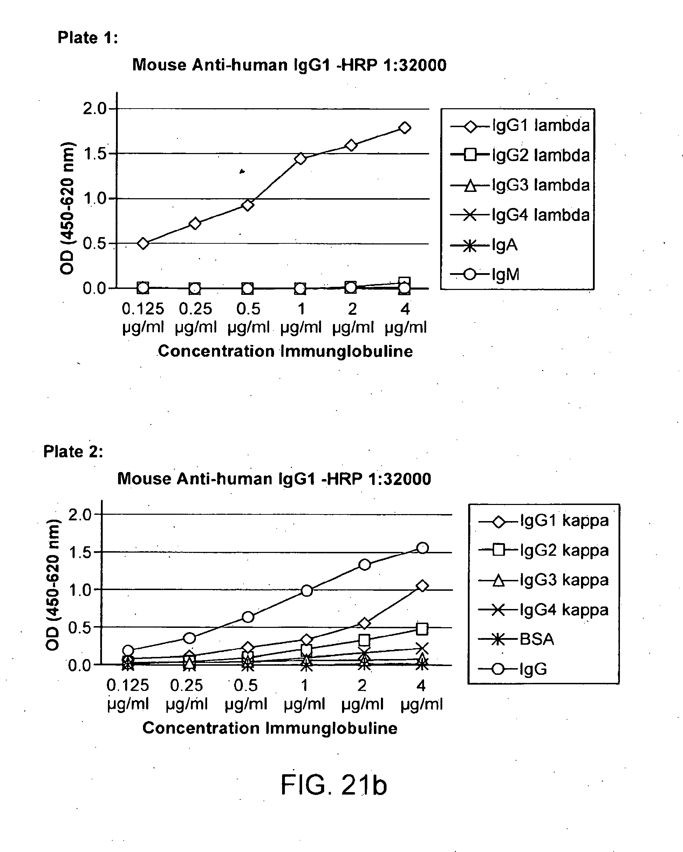

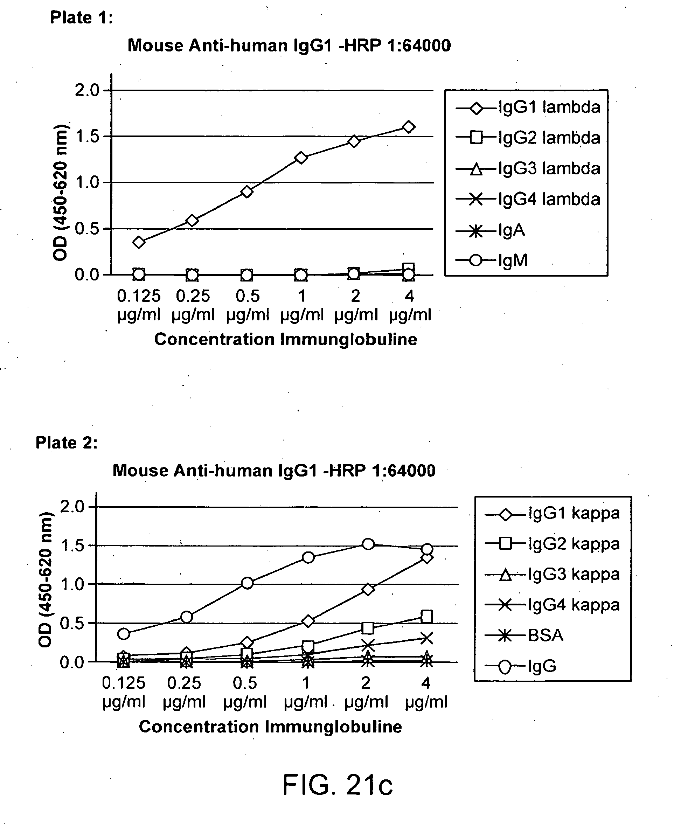

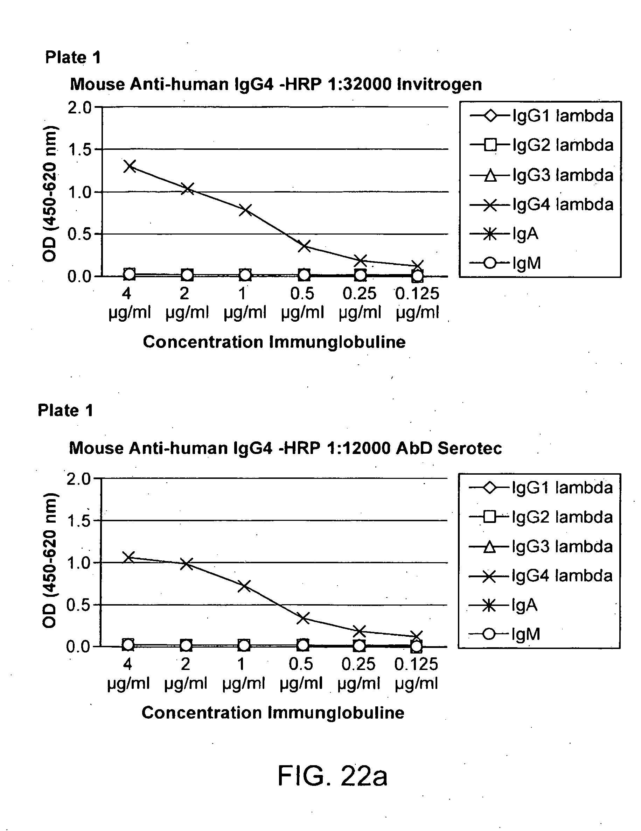

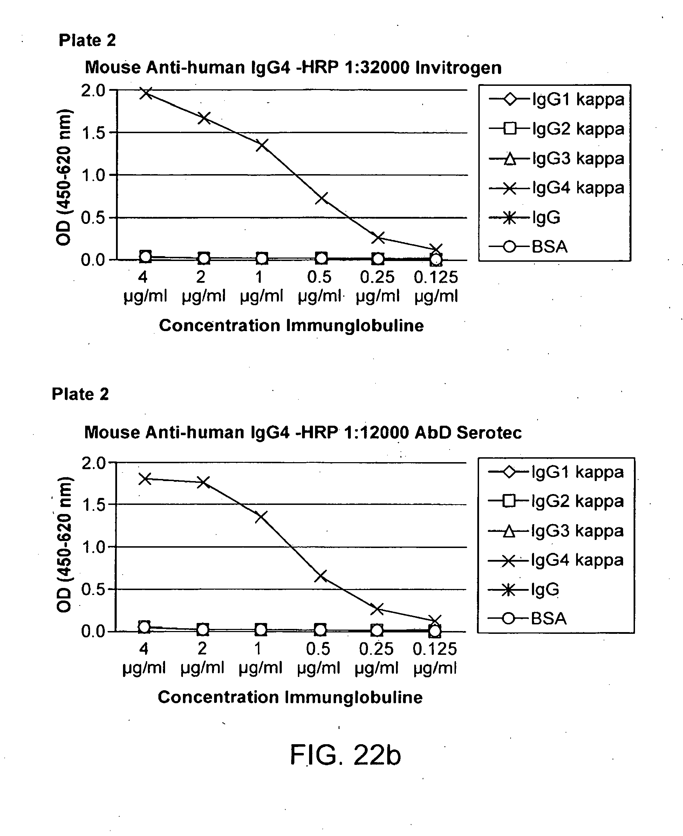

Cross-reactivities of the IgG1 antibody of Example 25 and IgG4 antibodies of Example 26 were tested by coating Ig of distinct classes and subtypes onto Nunc Maxisortp ELISA plates. No samples were used. Immediately after coating and incubation (over night at 4°C), the plates were washed and 250 $\mu$l/well of blocking buffer was added. The secondary antibody (IgG1, example 26 or an IgG4, example 27) was added and and signal generation was measured. The following coating antibodies were used: IgG1: human IgG1 kappa (Sigma, 1.2 mg/ml) and human IgG1 lambda (Sigma, 1.0 mg/ml); IgG2: human IgG2 kappa (Sigma, 1.1 mg/ml) and human IgG2 lambda (Sigma, 1.08 mg/ml); IgG3: human IgG3 kappa (Sigma, 1.1 mg/ml) and human IgG3 lambda (Sigma, 1.04 mg/ml); IgG4: human IgG4 kappa (Sigma, 1.2 mg/ml) and human IgG4 lambda (Sigma, 1.1 mg/ml); IgA: human IgA (Sigma, 1.0 mg/ml); IgM: human IgM (Sigma, 1.0 mg/ml) and IgG from human serum (Sigma, 6.4 mg/ml).
Results are shown in Figures 21 and 22. Graphs show signals of indicated concentrations of antibody.

REFERENCES

The following references relate to topics and subject matter discussed herein.

WO 1997/041206 A2
WO 02/42441 A1
WO 2004/095027 A1
Theofilopoulos et al., J Clin Invest 1976; 57:169-182
Plaimauer et al., Blood 2002; 100: 3626-3632
Scheiflinger et al., Blood 2003; 102(9): 3241-3243
Levy et al., Blood 2005; 106: 11-17
Rieger et al., Blood 2005; 106: 1262-1267
Wu et al., Journal of Thrombosis and Haemostasis 2005; 4: 129-136
Rieger et al., Thromb Haemost 2006; 95: 212-220
Sadler, Blood 2008; 112: 11-18
Ferrari et al., Journal of Thrombosis and Haemostasis 2009; 7: 1703-1710
Ito et al., Scandinavian J of Immunology 2010, vol 71(2): 109-114

**Claims**

1. A method for detecting or determining anti-ADAMTS13 antibody-ADAMTS13 immune complexes in a sample comprising contacting said sample with an anti-ADAMTS13 antibody, contacting said sample with an anti-antibody antibody and detecting anti-ADAMTS13 antibody-ADAMTS13 immune complexes bound by said anti-ADAMTS13 antibody and said anti-antibody antibody.

2. The method according to claim 1, further comprising detecting free anti-ADAMTS13 antibodies in the sample.

3. The method of claim 2, wherein the method of detecting or determining free anti-ADAMTS13 antibodies comprises binding said free anti-ADAMTS13 antibodies to ADAMTS13 or a fragment thereof and detecting said anti-ADAMTS13 antibodies bound to ADAMTS13 or said fragment with anti-antibody antibody specific for said anti-ADAMTS13 antibodies.

4. The method of any one of claims 1 to 3, further comprising detecting or determining free ADAMTS13 or determining ADAMTS13 activity in the sample.

5. The method of claim 4, wherein detecting or determining free ADAMTS13 comprises contacting said sample with an anti-ADAMTS13 antibody and detecting bound ADAMTS13 by said anti-ADAMTS13 antibody.

6. The method of claim 4, wherein determining ADAMTS13 activity comprises contacting the sample with a substrate of ADAMTS13 under conditions where ADAMTS13 is active to cleave the substrate and determining cleavage products of said substrate.

7. The method of any one of claims 1 to 6, wherein said anti-antibody antibodies are antibody subtype or antibody class specific.

8. The method of claim 7, wherein said antibody class is selected from IgG, IgM, IgA, IgD, IgE.

9. The method of claim 7 wherein said antibody subtype is selected from IgG1, IgG2, IgG3, IgG4.

10. The method of any one of claims 1 to 9, wherein said anti-antibody antibody recognizes the Fc part of an antibody.

11. The method of any one of claims 1 to 10, wherein said anti-ADAMTS13 antibody is a polyclonal antibody, a monoclonal antibody, or a mixture of two or more monoclonal antibodies.

12. The method of any one of claims 1 to 10, wherein said anti-ADAMTS13 antibody is a polyclonal antibody, further being immune or affinity purified by contacting with ADAMTS13.

13. The method of any one of claims 1 to 12, wherein said anti-ADAMTS13 antibody or said ADAMTS13 or fragment thereof is labelled and/or said anti-antibody antibody is labelled.

14. The method of any one of claims 1 to 13, wherein said anti-ADAMTS13 antibody or said ADAMTS13 or fragment thereof is immobilized and/or said anti-antibody antibody is immobilized.

15. The method of any one of claims 1 to 14, wherein said sample is a human sample.

16. The method of any one of claims 1 to 15, wherein said anti-antibody antibody recognizes human antibodies.

17. The method of any one of claims 1 to 16, wherein in said binding reaction or in said detecting step the ionic strength is at least 0.4 N.

18. The method of any one of claims 1 to 17, wherein in said binding reaction or detecting step the salt concentration is at least 0.4 M NaCl.

19. A method of diagnosing a disease associated with ADAMTS13 dysfunction in a patient comprising detecting ADAMTS13 immune complexes in a sample obtained from a patient according to a method of any one of claims 1 to 18.

20. Use of a method of any one of claims 1-19 to monitor an ADAMTS13 substitution therapy.

21. The method of claim 19 or use of claim 20, wherein the disease is TTP (thrombotic thrombocytopenic purpura), especially acquired TTP.


**Patentansprüche**

1. Verfahren zum Nachweisen oder zur Bestimmung von anti-ADAMTS13-Antikörper-ADAMTS13-Immunkomplexen in einer Probe, umfassend das Kontaktieren der Probe mit einem anti-ADAMTS13-Antikörper, das Kontaktieren der Probe mit einem anti-Antikörper-Antikörper und Nachweisen von anti-ADAMTS13-Antikörper-ADAMTS13-Immunkomplexen, die von dem anti-ADAMTS13-Antikörper und dem anti-Antikörper-Antikörper gebunden sind.

2. Verfahren gemäß Anspruch 1, ferner umfassend das Nachweisen freier anti-ADAMTS13-Antikörper in der Probe.

3. Verfahren gemäß Anspruch 2, wobei das Verfahren zum Nachweisen oder zur Bestimmung freier anti-ADAMTS13-Antikörper das Binden der freien anti-ADAMTS13-Antikörper an ADAMTS13 oder ein Fragment davon und Nachweisen der an ADAMTS13 oder das Fragment gebundenen anti-ADAMTS13-Antikörper mit für die anti-ADAMTS13-Antikörper spezifischem anti-Antikörper-Antikörper umfasst.

4. Verfahren gemäß einem der Ansprüche 1 bis 3, ferner umfassend das Nachweisen oder die Bestimmung von freiem ADAMTS13 oder die Bestimmung der ADAMTS13-Aktivität in der Probe.

5. Verfahren gemäß Anspruch 4, wobei das Nachweisen oder die Bestimmung von freiem ADAMTS13 das Kontaktieren der Probe mit einem anti-ADAMTS13-Antikörper und Nachweisen von gebundenem ADAMTS13 durch den anti-ADAMTS13-Antikörper umfasst.

**6.** Verfahren gemäß Anspruch 4, wobei die Bestimmung der ADAMTS13-Aktivität das Kontaktieren der Probe mit einem Substrat von ADAMTS13 unter Bedingungen, bei denen ADAMTS13 aktiv zum Spalten des Substrats ist, und die Bestimmung von Spaltprodukten des Substrats umfasst.

**7.** Verfahren gemäß einem der Ansprüche 1 bis 6, wobei die anti-Antikörper-Antikörper Antikörper-Subtyp- oder Antikörper-Klassen-spezifisch sind.

**8.** Verfahren gemäß Anspruch 7, wobei die Antikörper-Klasse ausgewählt ist aus IgG, IgM, IgA, IgD, IgE.

**9.** Verfahren gemäß Anspruch 7, wobei der Antikörper-Subtyp ausgewählt ist aus IgG1, IgG2, IgG3, IgG4.

**10.** Verfahren gemäß einem der Ansprüche 1 bis 9, wobei der anti-Antikörper-Antikörper den Fc-Teil eines Antikörpers erkennt.

**11.** Verfahren gemäß einem der Ansprüche 1 bis 10, wobei der anti-ADAMTS13-Antikörper ein polyklonaler Antikörper, ein monoklonaler Antikörper, oder eine Mischung von zwei oder mehreren monoklonalen Antikörpern ist.

**12.** Verfahren gemäß einem der Ansprüche 1 bis 10, wobei der anti-ADAMTS13-Antikörper ein polyklonaler Antikörper ist, der ferner durch das Kontaktieren mit ADAMTS13 immun- oder affinitätsgereinigt ist.

**13.** Verfahren gemäß einem der Ansprüche 1 bis 12, wobei der anti-ADAMTS13-Antikörper oder das ADAMTS13 oder Fragment davon markiert ist und/oder der anti-Antikörper-Antikörper markiert ist.

**14.** Verfahren gemäß einem der Ansprüche 1 bis 13, wobei der anti-ADAMTS13-Antikörper oder das ADAMTS13 oder Fragment davon immobilisiert ist und/oder der anti-Antikörper-Antikörper immobilisiert ist.

**15.** Verfahren gemäß einem der Ansprüche 1 bis 14, wobei die Probe eine menschliche Probe ist.

**16.** Verfahren gemäß einem der Ansprüche 1 bis 15, wobei der anti-Antikörper-Antikörper menschliche Antikörper erkennt.

**17.** Verfahren gemäß einem der Ansprüche 1 bis 16, wobei bei der Bindereaktion oder bei dem Nachweisschritt die Ionenstärke zumindest 0,4 N beträgt.

**18.** Verfahren gemäß einem der Ansprüche 1 bis 17, wobei bei der Bindereaktion oder dem Nachweisschritt die Salzkonzentration zumindest 0,4 M NaCl beträgt.

**19.** Verfahren zur Diagnose einer Krankheit, die mit ADAMTS13-Dysfunktion assoziiert ist, bei einem Patienten, umfassend das Nachweisen von ADAMTS13-Immunkomplexen in einer von einem Patienten erhaltenen Probe gemäß einem Verfahren gemäß einem der Ansprüche 1 bis 18.

**20.** Verwendung von einem Verfahren gemäß einem der Ansprüche 1 bis 19, um eine ADAMTS13-Substitutionstherapie zu überwachen.

**21.** Verfahren gemäß Anspruch 19 oder Verwendung gemäß Anspruch 20, wobei die Krankheit TTP (thrombotischthrombozytopenische Purpura), insbesondere erworbene TTP, ist.

**Revendications**

**1.** Procédé pour détecter ou déterminer des complexes immuns d'anticorps anti-ADAMTS13 et de ADAMTS13 dans un échantillon comprenant le fait de mettre en contact ledit échantillon avec un anticorps anti-ADAMTS13, de mettre en contact ledit échantillon avec un anticorps anti-anticorps et de détecter des complexes immuns d'anticorps anti-ADAMTS13 et de ADAMTS13 liés par ledit anticorps anti-ADAMTS13 et ledit anticorps anti-anticorps.

**2.** Procédé selon la revendication 1, comprenant en outre le fait de détecter des anticorps anti-ADAMTS13 libres dans l'échantillon.

**3.** Procédé de la revendication 2, dans lequel le procédé de détection ou de détermination d'anticorps anti-ADAMTS13 libres comprend le fait de lier lesdits anticorps anti-ADAMTS13 libres au ADAMTS13 ou un fragment de celui-ci et de détecter lesdits anticorps anti-ADAMTS13 liés au ADAMTS13 ou audit fragment avec un anticorps anti-anticorps spécifique pour lesdits anticorps anti-ADAMTS13.

**4.** Procédé de l'une quelconque des revendications 1 à 3, comprenant en outre le fait de détecter ou de déterminer le ADAMTS13 libre ou de déterminer l'activité de ADAMTS13 dans l'échantillon.

**5.** Procédé de la revendication 4, dans lequel la détection ou la détermination de ADAMTS13 libre comprend le fait de mettre en contact ledit échantillon avec un anticorps anti-ADAMTS13 et de détecter le ADAMTS13 lié par ledit anticorps anti-ADAMTS13.

**6.** Procédé de la revendication 4, dans lequel la détermination de l'activité de ADAMTS13 comprend le fait de mettre en contact l'échantillon avec un substrat de ADAMTS13 dans des conditions où ADAMTS13 est actif pour cliver le substrat et de déterminer des produits de clivage dudit substrat.

**7.** Procédé de l'une quelconque des revendications 1 à 6, dans lequel lesdits anticorps anti-anticorps sont spécifiques pour un sous-type d'anticorps ou spécifiques pour une classe d'anticorps.

**8.** Procédé de la revendication 7, dans lequel ladite classe d'anticorps est choisie parmi IgG, IgM, IgA, IgD, IgE.

**9.** Procédé de la revendication 7, dans lequel ledit sous-type d'anticorps est choisi parmi IgG1, IgG2, IgG3, IgG4.

**10.** Procédé de l'une quelconque des revendications 1 à 9, dans lequel ledit anticorps anti-anticorps reconnaît la partie Fc d'un anticorps.

**11.** Procédé de l'une quelconque des revendications 1 à 10, dans lequel ledit anticorps anti-ADAMTS13 est un anticorps polyclonal, un anticorps monoclonal, ou un mélange de deux anticorps monoclonaux ou plus.

**12.** Procédé de l'une quelconque des revendications 1 à 10, dans lequel ledit anticorps anti-ADAMTS13 est un anticorps polyclonal étant en outre immun purifié ou purifié par affinité par mise en contact avec le ADAMTS13.

**13.** Procédé de l'une quelconque des revendications 1 à 12, dans lequel ledit anticorps anti-ADAMTS13 ou ledit ADAMTS13 ou un fragment de celui-ci est marqué et/ou ledit anticorps anti-anticorps est marqué.

**14.** Procédé de l'une quelconque des revendications 1 à 13, dans lequel ledit anticorps anti-ADAMTS13 ou ledit ADAMTS13 ou un fragment de celui-ci est immobilisé et/ou ledit anticorps anti-anticorps est immobilisé.

**15.** Procédé de l'une quelconque des revendications 1 à 14, dans lequel ledit échantillon est un échantillon humain.

**16.** Procédé de l'une quelconque des revendications de 1 à 15, dans lequel ledit anticorps anti-anticorps reconnaît des anticorps humains.

**17.** Procédé de l'une quelconque des revendications 1 à 16, dans lequel, dans ladite réaction de liaison ou dans ladite étape de détection, la force ionique est d'au moins 0,4 N.

**18.** Procédé de l'une quelconque des revendications 1 à 17, dans lequel, dans ladite réaction de liaison ou étape de détection, la concentration en sel est d'au moins 0,4 M de NaCl.

**19.** Procédé de diagnostic d'une maladie associée à un dysfonctionnement de ADAMTS13 chez un patient comprenant le fait de détecter de complexes immuns de ADAMTS13 dans un échantillon obtenu à partir d'un patient selon un procédé de l'une quelconque des revendications 1 à 18.

**20.** Utilisation d'un procédé de l'une quelconque des revendications 1 à 19 pour surveiller une thérapie de substitution de ADAMTS13.

**21.** Procédé de la revendication 19 ou utilisation de la revendication 20, où la maladie est TTP (purpura thrombocytopénique thrombotique), en particulier TTP acquis.

FIG. 1

FIG. 2a

FIG. 2b

## Plate coated with Rbt Anti-human ADAMTS Ab (abcam)

Legend:
- ◇ IgG4
- + ADAMTS13
- □ hAlbumine (5-0,15%)
- △ IgG4 (10µg/ml) + Albumin diluted
- ✳ IgG4 (10µg/ml) + ADAMTS13 diluted
- ✱ ADAMTS13 (10µg/ml) + IgG4 diluted
- ○ ADAMTS13 (10µg/ml) + Albumin diluted

Y-axis: OD (450 nm)

X-axis: 20 µg/ml, 10 µg/ml, 5 µg/ml, 2.5 µg/ml, 1.25 µg/ml, 0.625 µg/ml

Concentration of diluted rADAMTS13/IgG4/hAlbumine

FIG. 3a

## Plate coated with Rbt Anti-human ADAMTS Ab K1-4

Legend:
- ◇ IgG4
- + ADAMTS13
- □ hAlbumine (5-0,15%)
- △ IgG4 (10µg/ml) + Albumin diluted
- ✳ IgG4 (10µg/ml) + ADAMTS13 diluted
- ✱ ADAMTS13 (10µg/ml) + IgG4 diluted
- ○ ADAMTS13 (10µg/ml) + Albumin diluted

Y-axis: OD (450 nm)

X-axis: 20 µg/ml, 10 µg/ml, 5 µg/ml, 2.5 µg/ml, 1.25 µg/ml, 0.625 µg/ml

Concentration of diluted rADAMTS13/IgG4/hAlbumine

FIG. 3b

## Plate coated with Rbt Anti-human ADAMTS Ab K6

Legend:
- ◇ IgG4
- + ADAMTS13
- □ hAlbumine (5-0,15%)
- △ IgG4 (10µg/ml) + Albumin diluted
- ✳ IgG4 (10µg/ml) + ADAMTS13 diluted
- ✱ ADAMTS13 (10µg/ml) + IgG4 diluted
- ○ ADAMTS13 (10µg/ml) + Albumin diluted

Y-axis: OD (450 nm)

X-axis: 20 µg/ml, 10 µg/ml, 5 µg/ml, 2.5 µg/ml, 1.25 µg/ml, 0.625 µg/ml

Concentration of diluted rADAMTS13/IgG4/hAlbumine

FIG. 3c

**Plate coated with MAb clone 5.1**

Legend:
- ◇ IgG4
- + ADAMTS13
- □ hAlbumine (5-0,15%)
- △ IgG4 (10µg/ml) + Albumin diluted
- ✳ IgG4 (10µg/ml) + ADAMTS13 diluted
- ✲ ADAMTS13 (10µg/ml) + IgG4 diluted
- ○ ADAMTS13 (10µg/ml) + Albumin diluted

FIG. 3d

**Plate coated with MAb clone 12.1**

Legend:
- ◇ IgG4
- + ADAMTS13
- □ hAlbumine (5-0,15%)
- △ IgG4 (10µg/ml) + Albumin diluted
- ✳ IgG4 (10µg/ml) + ADAMTS13 diluted
- ✲ ADAMTS13 (10µg/ml) + IgG4 diluted
- ○ ADAMTS13 (10µg/ml) + Albumin diluted

FIG. 3e

**IgG1**

Legend:
- ◇ IgG1 lambda
- □ IgG2 lambda
- △ IgG3 lambda
- ✳ IgG4 lambda
- ✲ IgA
- ○ IgM
- + Buffer

Legend:
- ◇ IgG1 kappa
- □ IgG2 kappa
- △ IgG3 kappa
- ✳ IgG4 kappa
- ✲ IgA
- ○ IgM
- + Buffer

FIG. 4a

FIG. 4b

FIG. 4c

FIG. 4d

FIG. 4e

FIG. 4f

FIG. 5a

FIG. 5b

FIG. 5c

**IgG4**

**FIG. 5d**

FIG. 5e

FIG. 5f

**pAb K1-4**

FIG. 6a

**mAb 5C11**

FIG. 6b

**Plate coated with K1-4 (AP)**

Concentration of diluted rADAMTS13/IgG4/hAlbumine

-◇- IgG4
-+- ADAMTS13
-□- hAlbumin (10-0.3%)
-△- IgG4 (10µg/ml) + Albumin diluted
-✕- IgG4 (10µg/ml) + ADAMTS13 diluted
-✳- ADAMTS13 (10µg/ml) + IgG4 diluted
-○- ADAMTS13 (10µg/ml) + Albumin diluted

FIG. 6c

FIG. 7a

FIG. 7b

36

# IgG1

FIG. 8a

# IgG2

## 2nd Ab 1:1000

## 2nd Ab 1:2000

## 2nd Ab 1:4000

## S/N Ratio

FIG. 8b

FIG. 8c

FIG. 8d

FIG. 8e

**IgM**

FIG. 8f

## ADAMTS-13/IgG1 Immune-complexes

FIG. 9a

## ADAMTS-13/IgG2 Immune-complexes

FIG. 9b

## ADAMTS-13/IgG3 Immune-complexes

FIG. 9c

FIG. 9d

FIG. 9e

FIG. 9f

Plate coated with rabbit pAb K1-4 aganist ADAMTS-13, HEK derived (2µg/ml, affinity purified)

Plate coated with rabbit pAb K1-4 aganist ADAMTS-13, HEK derived (4µg/ml, affinity purified)

FIG. 10a

FIG. 10b

**K1-10, 2µg/ml**

Legend: TTP, NHP, Buffer

**K1-10, 4µg/ml**

Legend: TTP, NHP, Buffer

**K1-10, 6µg/ml**

Legend: TTP, NHP, Buffer

FIG. 10c

Plate coated with polyclonal Ab aganist human
ADAMTS-13 K1-10 (CHO derived; Prot-G Eluat)

Legend:
- IgG4
- ADAMTS13
- hAlbumine (10-0.3%)
- IgG4 (10µg/ml) + ADAMTS13 diluted
- IgG4 (10µg/ml) + Albumin diluted
- ADAMTS13 (10µg/ml) + IgG4 diluted
- ADAMTS13 (10µg/ml) + Albumin diluted

Concentration of diluted
rADAMTS13/IgG4/hAlbumine

FIG. 11a

Plate coated with polyclonal Ab aganist human
ADAMTS-13 K1-10 (CHO derived; affinity purified)

- ◇ IgG4
- + ADAMTS13
- □ Albumin
- △ IgG4 (10μg/ml) + ADAMTS13 diluted
- ✕ IgG4 (10μg/ml) + Albumin diluted
- ✳ ADAMTS13 (10μg/ml) + IgG4 diluted
- ○ ADAMTS13 (10μg/ml) + Albumin diluted

Concentration of diluted
rADAMTS13/IgG4/hAlbumine

FIG. 11b

Results: 2nd Antibody: Mouse anti-human IgA -HRP (Pierce)
1:32000

Cross-reactions of Mouse anti human IgA -HRP

- ◇ IgG1 lambda
- □ IgG2 lambda
- △ IgG3 lambda
- ✕ IgG4 lambda
- ✳ IgA
- ○ IgM

concentration of Immunglobulin

Cross-reactions of Mouse anti human IgA -HRP

- ◇ IgG1 kappa
- □ IgG2 kappa
- △ IgG3 kappa
- ✕ IgG4 kappa
- ✳ IgA
- ○ IgM

concentration of Immunglobulin

FIG. 12

Results: 2nd Antibody: Goat anti-human IgA -HRP (Sigma)
1:25000

Cross-reactions of Goat anti human IgA -HRP

concentration of Immunglobulin

Cross-reactions of Goat anti human IgA -HRP

concentration of Immunglobulin

FIG. 12 (Cont.)

FIG. 13

FIG. 14a

51

FIG. 14b

FIG. 15

Coating Ab 1µg/ml:

Mouse IgG HRP 1:16000

Coating Ab 2µg/ml:

Mouse IgG HRP 1:16000

Mouse IgG HRP 1:32000

Mouse IgG HRP 1:32000

Mouse IgG HRP 1:64000

Mouse IgG HRP 1:64000

FIG. 16a

FIG. 16b

FIG. 17a

FIG. 17b

FIG. 17c

FIG. 17d

FIG. 17e

FIG. 17f

FIG. 18a

FIG. 18b

FIG. 18c

ADAMTS13/Ig4-Immune complexes

ADAMTS13/Ig4-Immune complexes

FIG. 18d

FIG. 18e

FIG. 18e (Cont.)

FIG. 18f

FIG. 18f (Cont.)

FIG. 19a

FIG. 19b

FIG. 20a

FIG. 20b

Plate 1:

Mouse Anti-human IgG1 -HRP 1:16000

Plate 2:

Mouse Anti-human IgG1 -HRP 1:16000

FIG. 21a

Plate 1:

Mouse Anti-human IgG1 -HRP 1:32000

Plate 2:

Mouse Anti-human IgG1 -HRP 1:32000

FIG. 21b

**Plate 1:**

## Mouse Anti-human IgG1 -HRP 1:64000

**Plate 2:**

## Mouse Anti-human IgG1 -HRP 1:64000

FIG. 21c

FIG. 22a

**Plate 2**

**Mouse Anti-human IgG4 -HRP 1:32000 Invitrogen**

**Plate 2**

**Mouse Anti-human IgG4 -HRP 1:12000 AbD Serotec**

**FIG. 22b**

EP 2 710 377 B1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 1997041206 A
- WO 0242441 A
- WO 2004095027 A, Rieger
- WO 2002042441 A
- WO 97041206 A
- WO 0242442 A
- WO 1997041206 A2
- WO 0242441 A1
- WO 2004095027 A1

### Non-patent literature cited in the description

- **THEOFILOPOULOS et al.** *J Clin Invest,* 1976, vol. 57, 169-182
- **PLAIMAUER et al.** *Blood,* 2002, vol. 100, 3626-3632
- **SCHEIFLINGER et al.** *Blood,* 2003, vol. 102 (9), 3241-3243
- **LEVY et al.** *Blood,* 2005, vol. 106, 11-17
- **RIEGER et al.** *Blood,* 2005, vol. 106, 1262-1267
- **WU et al.** *Journal of Thrombosis and Haemostasis,* 2005, vol. 4, 129-136
- **RIEGER et al.** *Thromb Haemost,* 2006, vol. 95, 212-220
- **SADLER.** *Blood,* 2008, vol. 112, 11-18
- **FERRARI et al.** *Journal of Thrombosis and Haemostasis,* 2009, vol. 7, 1703-1710
- **ITO et al.** *Scandinavian J of Immunology,* 2010, vol. 71 (2), 109-114